(19) Europäisches Patentamt
European Patent Office
Office européen des brevets

(11) **EP 2 527 343 A1**

(12) **EUROPEAN PATENT APPLICATION**
published in accordance with Art. 153(4) EPC

(43) Date of publication:
**28.11.2012 Bulletin 2012/48**

(21) Application number: **11734701.3**

(22) Date of filing: **20.01.2011**

(51) Int Cl.:
***C07D 413/04*** *(2006.01)*
***A61K 31/4375*** *(2006.01)*
***A61K 31/4709*** *(2006.01)*
***A61P 31/18*** *(2006.01)*
***C07D 471/04*** *(2006.01)*

(86) International application number:
**PCT/JP2011/050927**

(87) International publication number:
**WO 2011/090095 (28.07.2011 Gazette 2011/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.01.2010 JP 2010012573**

(71) Applicant: **Toyama Chemical Co., Ltd.**
**Shinjuku-ku**
**Tokyo 160-0023 (JP)**

(72) Inventors:
- **OONISHI, Yuji**
  **Toyama-shi**
  **Toyama 930-8508 (JP)**
- **AWASAGUCHI, Kenichiro**
  **Toyama-shi**
  **Toyama 930-8508 (JP)**
- **NOMURA, Nobuhiko**
  **Toyama-shi**
  **Toyama 930-8508 (JP)**
- **TOHDO, Keisuke**
  **Toyama-shi**
  **Toyama 930-8508 (JP)**
- **KAWAI, Hyouei**
  **Toyama-shi**
  **Toyama 930-8508 (JP)**
- **WAKATSUKI, Tomomi**
  **Toyama-shi**
  **Toyama 930-8508 (JP)**

(74) Representative: **Blodig, Wolfgang**
**Wächtershäuser & Hartz**
**Patentanwaltspartnerschaft**
**Ottostrasse 4**
**80333 München (DE)**

# (54) HETEROCYCLIC COMPOUND HAVING AZOLE GROUP

(57) A heterocyclic compound represented by general formula (I)

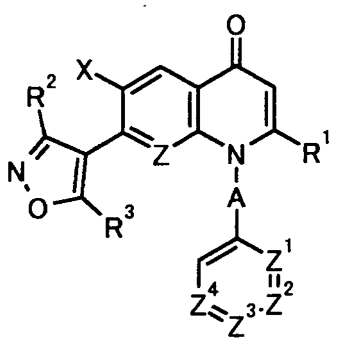

[wherein $R^1$ represents an optionally protected amino group, or an optionally substituted $C_{1-6}$ alkyl group; $R^2$ and $R^3$ independently represent a $C_{1-2}$ alkyl group; X represents a hydrogen atom, or a halogen atom; $Z^1$ represents C-$R^4$ (wherein $R^4$ represents a hydrogen atom, a halogen atom, an optionally protected amino group, or a $C_{1-6}$ alkylsulfonyl group), or N; $Z^2$ represents C-$R^5$ (wherein, $R^5$ represents a hydrogen atom, a hydrogen atom, a halogen atom, an optionally protected amino group, or a $C_{1-6}$ alkylsulfonyl group), or N; $Z^3$ represents C-$R^6$ (wherein $R^6$ represents a hydrogen atom, a hydrogen atom, a halogen atom, an optionally protected amino group, or a $C_{1-6}$ alkylsulfonyl group ), or N; $Z^4$ represents C-$R^7$ (wherein, $R^7$ represents a hydrogen atom, a hydrogen atom, a halogen atom, an optionally protected amino group, or a $C_{1-6}$ alkylsulfonyl group), or N; Z represents CH or N; and A represents a methylene group, or a bond] or a salt thereof, which has an excellent anti-HIV activity and is thereof useful as an anti-HIV agent.

EP 2 527 343 A1

## Description

Background of the invention

(1) Field of the invention

The present invention relates to a heterocyclic compound or a salt thereof useful as an anti-HIV agent.

(2) Description of related art

The human immunodeficiency virus (HIV), which is a retrovirus, infects immunocytes to cause acquired immunodeficiency syndrome (AIDS). The number of HIV infected patients has heretofore reached several tens of millions of people worldwide. In particular, recent infection spreading in Asian and African regions is regarded as a serious issue.

HIV has in its coat RNA genes encoding enzymes specific to viruses (such as protease, reverse transcriptase and integrase), and invades a target cell via CD4 and chemokine receptors which are present on an immune cell surface layer. After uncoating, HIV releases complexes such as RNA and integrase into the cytoplasm, and performs reverse transcription of its genetic information into a double strand provirus DNA using its own reverse transcriptase. Further, by means of integrase specific to HIV, HIV integrates the provirus DNA into the DNA of the target cell. The provirus DNA thus integrated into the target cell is transcribed to an RNA chain, and efficiently produces viral proteins by viral regulatory gene products such as Tat and Rev. The viral proteins are combined with a virus RNA which is separately formed, and bud from the membrane surface of the host cell. The virus which migrates outside the cell wall repeatedly infects immune cells (such as CD4-positive T cell and macrophage) and grows, leading to immune deficiency disease in the host.

Anti-HIV agents targeting enzymes specific to viruses have been heretofore developed. For example, nucleoside analogue reverse transcriptase inhibitors such as Zidovudine, Didanosine, Lamivudine, Abacavir and Tenofovir; non-nucleoside analogue reverse transcriptase inhibitors such as Efavirentz and Nevirapine; protease inhibitors such as Lopinavir, fosamprenavir, Atazanavir and Darunavir; integrase inhibitors such as Raltegravir; and invasion inhibitors such as Maraviroc are commercially available.

In AIDS therapy, combination therapy combining these pharmaceuticals is employed. For example, a combination of a non-nucleoside analogue reverse transcriptase inhibitor and two nucleoside analogue reverse transcriptase inhibitors, and a combination of a protease inhibitor and two nucleoside analogue reverse transcriptase inhibitors are recommended (Antibiotics & Chemotherapy, 2009, Vol. 25, pages 26 to 33).

However, HIV easily acquires resistance. Once the virus has acquired resistance, the virus is less sensitive to agents of the same line (Antibiotics & Chemotherapy, 2004, Vol. 20, pages 58 to 68). Further, the number of agents to be employed in combination therapy is limited, and the effects of such combination therapy are not necessarily satisfactory.

As side effects of the currently used agents, for example, lactic acidosis due to a nucleoside analogue reverse transcriptase inhibitor, and complications such as lipidosis and diabetes due to a protease inhibitor, have been reported (Antibiotics & Chemotherapy, 2009, Vol. 25, pages 40 to 53; Antibiotics & Chemotherapy, 2009, Vol. 25, pages 54 to 61).

On the other hand, naphthyridone and quinolone compounds which have an antivirus activity are known (JP 3713291, JP 3754467).

Brief summary of the invention

A compound having a new action mechanism, excellent in pharmacokinetics, having less side effects, and having an excellent anti-HIV activity is strongly desired.

In order to solve the above problem, the present inventors have dedicatedly studied and found that a heterocyclic compound represented by a general formula [A]:

[Formula 1]

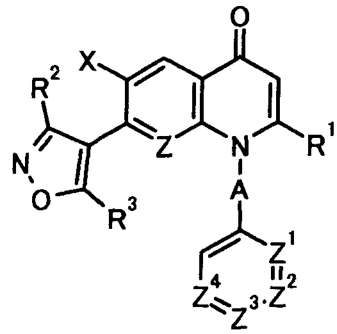

[1A]

wherein $R^1$ represents an optionally protected amino group, or an optionally substituted $C_{1-6}$ alkyl group; $R^2$ and $R^3$ are the same or different and represent a $C_{1-2}$ alkyl group; X represents a hydrogen atom or a halogen atom; $Z^1$ represents C-$R^4$ (wherein $R^4$ represents a hydrogen atom, a halogen atom, an optionally protected amino group or a $C_{1-6}$ alkylsulfonyl group), or N; $Z^2$ represents C-$R^5$ (wherein $R^5$ represents a hydrogen atom, a halogen atom, an optionally protected amino group or a $C_{1-6}$ alkylsulfonyl group), or N; $Z^3$ represents C-$R^6$ (wherein $R^6$ represents a hydrogen atom, a halogen atom, an optionally protected amino group or a $C_{1-6}$ alkylsulfonyl group), or N; $Z^4$ represents C-$R^7$ (wherein $R^7$ represents a hydrogen atom, a halogen atom, an optionally protected amino group or a $C_{1-6}$ alkylsulfonyl group), or N; Z represents CH or N; A represents a methylene group or a bond; or a salt thereof has an excellent anti-HIV activity and is useful as an anti-HIV agent.

Further, the present inventors have found that a heterocyclic compound represented by a general formula [1B]:

[Formula 2]

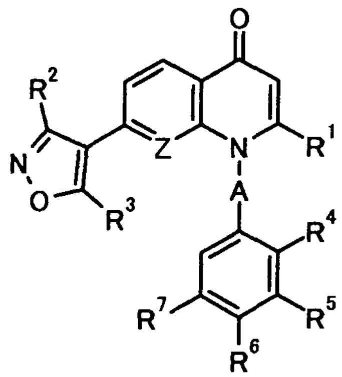

[1B]

wherein $R^1$ represents an optionally protected amino group, or an optionally substituted $C_{1-6}$ alkyl group; $R^2$ and $R^3$ are the same or different and represent a $C_{1-2}$ alkyl group; $R^4$, $R^5$, $R^6$ and $R^7$ are the same or different and represent a hydrogen atom, a halogen atom, an optionally protected amino group or a $C_{1-6}$ alkylsulfonyl group; Z represents CH or N; A represents a methylene group or a bond; or a salt thereof has a more excellent anti-HIV activity and is useful as an anti-HIV agent, and then has completed the present invention.

As the heterocyclic compound or a salt thereof of the present invention has an excellent anti-HIV activity, it is useful as an anti-HIV agent.

In another aspect, the heterocyclic compound or a salt thereof of the present invention is excellent in pharmacokinetics, has less side effects and an excellent anti-HIV activity.

Detailed description of the invention

In the following, the present invention will be described in detail.

In the present invention, unless otherwise stated, each term has the following meaning.

A halogen atom means a fluorine atom, a chlorine atom, a bromine atom or an iodine atom.

A $C_{1-2}$ alkyl group means a methyl group or an ethyl group.

A $C_{1-6}$ alkyl group means a straight or branched $C_{1-6}$ alkyl group such as a methyl group, an ethyl group, a propyl group, an isopropyl group, a butyl group, a sec-butyl group, an isobutyl group, a tert-butyl group, a pentyl group, an isopentyl group and a hexyl group.

An ar-$C_{1-6}$ alkyl group means an ar-$C_{1-6}$ alkyl group such as benzyl, diphenylmethyl, trityl, phenethyl and naphthylmethyl.

A $C_{1-6}$ alkoxy group means a straight or branched $C_{1-6}$ alkyloxy group such as methoxy, ethoxy, propoxy, isopropoxy, butoxy, isobutoxy, sec-butoxy, tert-butoxy, pentyloxy and hexyloxy.

A$C_{1-6}$ alkoxy $C_{1-6}$ alkyl group means a $C_{1-6}$ alkyloxy $C_{1-6}$ alkyl group such as methoxymethyl and 1-ethoxyethyl.

A $C_{2-12}$ alkanoyl group means a straight or branched $C_{2-12}$ alkanoyl group such as acetyl, propionyl, valeryl, isovaleryl and pivaloyl.

An aroyl group means a benzoyl group or a naphthoyl group.

A heterocyclic carbonyl group means a nicotinoyl group, a thenoyl group, a pyrrolidinocarbonyl group or a furoyl group.

An (oc-substituted)aminoacetyl group means an ($\alpha$-substituted)aminoacetyl group of which N-terminal may be protected derived from an amino acid (amino acids such as glycine, alanine, valine, leucine, isoleucine, serine, threonine, cysteine, methionine, asparatic acid, glutamic acid, asparagine, glutamine, arginine, lysine, histidine, hydroxylysine, phenylalanine, tyrosine, tryptophane, proline and hydroxyproline can be mentioned).

An acyl group means a formyl group, a succinyl group, a glutaryl group, a maleoyl group, a phthaloyl group, a $C_{2-12}$ alkanoyl group, an aroyl group, a heterocyclic carbonyl group or an ($\alpha$-substituted)aminoacetyl group.

A $C_{1-6}$ alkoxycarbonyl group means a straight or branched $C_{1-6}$ alkyloxycarbonyl group such as methoxycarbonyl, ethoxycarbonyl, isopropoxycarbonyl, tert-butoxycarbonyl and 1, 1-dimethylpropoxycarbonyl.

An ar-$C_{1-6}$ alkoxycarbonyl group means an ar-$C_{1-6}$ alkyloxycarbonyl group such as benzyloxycarbonyl and phenethyloxycarbonyl.

An aryloxycarbonyl group means a phenyloxycarbonyl group or a naphthyloxycarbonyl group.

A $C_{1-6}$ alkylsulfonyl group means a $C_{1-6}$ alkylsulfonyl group such as methylsulfonyl, ethylsulfonyl and propylsulfonyl.

An arylsulfonyl group means a benzenesulfonyl group, a p-toluenesulfonyl group or a naphthalenesulfonyl group.

A $C_{1-6}$ alkylsulfonyloxy group means a $C_{1-6}$ alkylsulfonyloxy group such as methylsulfonyloxy and ethylsulfonyloxy.

An arylsulfonyloxy group means a benzenesulfonyloxy group or a p-toluenesulfonyloxy group.

A silyl group means a trimethylsilyl group, a triethylsilyl group or a tributylsilyl group.

A boron-containing heterocyclic group means a monocyclic boron-containing heterocyclic group containing a boron atom as a hetero atom forming the ring such as 1, 3, 2-dioxaboretan-2-yl, 1, 3, 2-dioxaborolan-2-yl and 1, 3, 6, 2-dioxazaborocan-2-yl.

The amino protecting group include all the groups which can be used as a usual protecting group for an amino group, and examples of which include the groups described in W. Greene et al., Protective Groups in Organic Synthesis, Fourth edition, pages 696 to 926, 2007, John Wiley & Sons, INC. Specifically, an ar-$C_{1-6}$ alkyl group, a $C_{1-6}$ alkoxy $C_{1-6}$ alkyl group, an acyl group, a $C_{1-6}$ alkoxycarbonyl group, an ar-$C_{1-6}$ alkoxycarbonyl group, an aryloxycarbonyl group, a $C_{1-6}$ alkylsulfonyl group, an arylsulfonyl group and a silyl group etc. can be mentioned. The above groups may be substituted by one or more groups selected from a halogen atom, a nitro group, a cyano group, a $C_{1-6}$ alkyl group and a $C_{1-6}$ alkoxy group.

As leaving groups, a halogen atom, a $C_{1-6}$ alkylsulfonyloxy group and an arylsulfonyloxy group can be mentioned.

Halogenated hydrocarbons mean methylene chloride, chloroform or dichloroethane.

Ethers mean diethyl ether, diisopropyl ether, dioxane, tetrahydrofuran, anisole, ethylene glycol dimethyl ether, diethylene glycol dimethyl ether or diethylene glycol diethyl ether.

Alcohols mean methanol, ethanol, propanol, 2-propanol, butanol or 2-methyl-2-propanol.

Esters mean methyl acetate, ethyl acetate, propyl acetate or butyl acetate.

Amides mean N, N-dimethylformamide, N, N-dimethylacetamide or 1-methyl-2-pyrrolidone.

Aromatic hydrocarbons mean benzene, toluene or xylene.

As salts of the compound of the general formula [1A] or the compound of the general formula [1B], commonly known salts of basic groups such as an amino group can be mentioned.

Examples of salts of a basic group include salts with mineral acids such as hydrochloric acid, hydrobromic acid, nitric acid and sulfuric acid; salts with organic carboxylic acids such as formic acid, acetic acid, citric acid, oxalic acid, fumaric acid, maleic acid, succinic acid, malic acid, tartaric acid, asparatic acid, trichloroacetic acid and trifluoroacetic acid; and salts with sulfonic acids such as methanesulfonic acid, benzenesulfonic acid, p-toluenesulfonic acid, mesitylenesulfonic acid and naphthalenesulfonic acid.

Further, as preferable salts of the compound of the general formula [1A] or the compound of the general formula [1B] among the above salts, pharmacologically acceptable salts can be mentioned.

The $C_{1-6}$ alkyl group of $R^1$ may be substituted by one or more groups selected from a halogen atom, an amino group and a hydroxyl group.

In the compound of the general formula [1A] or the compound of the general formula [1B] of the present invention, following compounds can be mentioned as preferable compounds.

A compound in which $Z^1$ is C-$R^4$ (wherein $R^4$ has the same meaning as described above) is preferable.

A compound in which $Z^2$ is C-$R^5$ (wherein $R^5$ has the same meaning as described above), or N is preferable, and a compound in which $Z^2$ is C-$R^5$ (wherein $R^5$ has the same meaning as described above) is more preferable.

A compound in which $Z^3$ is C-$R^6$ (wherein $R^6$ has the same meaning as described above) is preferable.

A compound in which $Z^4$ is C-$R^7$ (wherein $R^7$ has the same meaning as described above) is preferable.

A compound in which X is a hydrogen atom is preferable.

A compound in which $R^1$ is a methyl group optionally substituted by a hydroxyl group or an optionally protected amino group is preferable, and a compound in which $R^1$ is a methyl group is more preferable.

A compound in which $R^2$ is a methyl group is preferable.

A compound in which $R^3$ is a methyl group is preferable.

A compound in which $R^4$ is a hydrogen atom, a halogen atom or an optionally protected amino group is preferable, and a compound in which $R^4$ is a halogen atom or an optionally protected amino group is more preferable, and a compound in which $R^4$ is a fluorine atom or an optionally protected amino group is further preferable.

A compound in which $R^5$ is a hydrogen atom, a halogen atom or an optionally protected amino group is preferable, and a compound in which $R^5$ is a hydrogen atom or a halogen atom is more preferable, and a compound in which $R^5$ is a hydrogen atom or a fluorine atom is further preferable.

A compound in which $R^6$ is a hydrogen atom, a halogen atom or an optionally protected amino group is preferable, and a compound in which $R^6$ is a hydrogen atom or a halogen atom is more preferable, and a compound in which $R^6$ is a hydrogen atom or a fluorine atom is further preferable.

A compound in which $R^7$ is a hydrogen atom, a halogen atom or an optionally protected amino group is preferable, and a compound in which $R^7$ is a hydrogen atom or an optionally protected amino group is more preferable.

A compound in which A is a bond (the bond means a single bond. In the compound of the general formula [1A] or the compound of the general formula [1B] of the present invention, when A is a bond, N-A-C means N-C.) is preferable.

In the compound of the general formula [1A] or the compound of the general formula [1B] of the present invention, following compounds can be mentioned as preferable compounds:

1-(5-amino-2, 4-difluorophenyl)-7-(3, 5-dimethylisoxazol-4-yl)-2-methyl-1, 4-dihydro-4-oxoquinoline, 1-(5-amino-2, 4-difluorophenyl)-7-(3, 5-dimethylisoxazol-4-yl)-2-methyl-1, 4-dihydro-4-oxo-1, 8-naphthyridine, 1-(5-amino-2, 3, 4-trifluorophenyl)-7-(3, 5-dimethylisoxazol-4-yl)-2-methyl-1, 4-dihydro-4-oxo-1, 8-naphthyridine and 1-(2-amino-3, 5-difluorobenzyl)-7-(3, 5-dimethylisoxazol-4-yl)-2-methyl-1, 4-dihydro-4-oxoquinoline.

In the compound of the general formula [1A] or the compound of the general formula [1B] or salts thereof, when isomers (for example, optical isomers, geometrical isomers and tautomers) are present, these isomers also can be used. When solvates, hydrates and various shaped crystals are present, these solvates, hydrates and various shaped crystals also can be used.

Next, the method for producing the compound of the present invention will be described.

The compound of the present invention is produced by combining the methods known per se, but for example, can be produced according to the following production methods.

[Production Method 1]

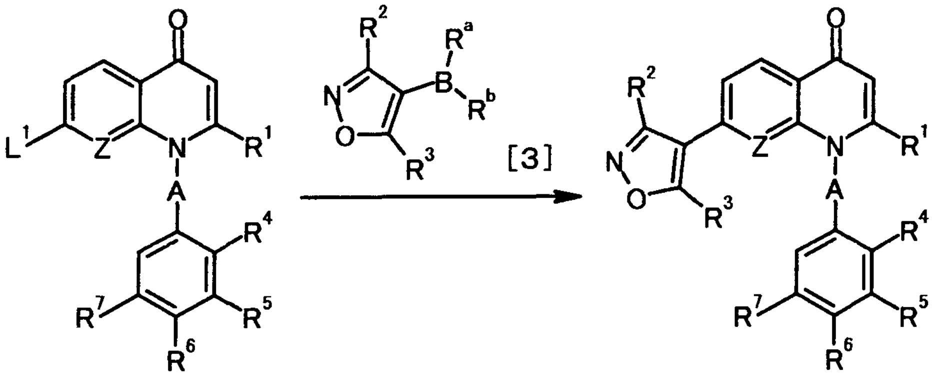


[2] [1B]

wherein $L^1$ represents a leaving group; $R^a$ and $R^b$ are the same or different and represent a hydroxyl group, an optionally substituted $C_{1-6}$ alkoxy group, or an optionally substituted boron-containing heterocyclic group together with a boron atom to which the group binds; $R^1$, $R^2$, $R^3$, $R^4$, $R^5$, $R^6$, $R^7$, Z and A have the same meanings as described above.

Examples of the compound of the general formula [3] include 3, 5-dimethylisoxazole-4-boronic acid and 3, 5-dimethyl-4-(4, 4, 5, 5-tetramethyl-1, 3, 2-dioxaborolan-2-yl)isoxazole.

For example, the compound of the general formula [3] can be produced according to the method described in Tetrahedron, Vol. 58, pages 3323 to 3328 (2002) from the corresponding halogeno form.

The compound of the general formula [1B] can be produced by reacting the compound of the general formula [2] with the compound of the general formula [3] in the presence or absence of a base, in the presence of a palladium catalyst, and in the presence or absence of a ligand.

The solvent used in this reaction is not particularly limited as long as it does not affect the reaction, but for example, halogenated hydrocarbons, ethers, alcohols, aromatic hydrocarbons, acetonitrile and water can be mentioned, and these solvents may be used in combination.

As preferable solvents, a mixed solvent of alcohols, aromatic hydrocarbons and water, and ethers; and a mixed solvent of ethanol, toluene and water, and dioxane are preferable.

The used amount of the solvent is not particularly limited, but preferably is 1 to 100 times in volume (v/w), more preferably 10 to 30 times in volume (v/w) to the compound of the general formula [2].

Examples of the palladium catalysts used in this reaction include metallic palladium such as palladium-carbon and palladium black; inorganic palladium salts such as palladium chloride; organic palladium salts such as palladium acetate; organic palladium complexes such as tetrakis(triphenylphosphine)palladium(0), bis(triphenylphosphine)palladium(II) chloride, 1, 1'-bis(diphenylphosphino)ferrocene palladium(II) chloride and tris(dibenzylideneacetone)dipalladium (0); and polymer immobilized organic palladium complexes such as polymer-supported bis(acetate)triphenylphosphine palladium(II) and polymer-supported di(acetate)dicyclohexylphenylphosphine palladium(II), and these may be used in combination.

The used amount of the palladium catalyst may be 0.00001 to 1 times in mole, preferably 0.01 to 0.2 times in mole to the compound of the general formula [2].

Examples of the ligand used in this reaction if desired include trialkylphosphines such as trimethylphosphine and tri-tert-butylphosphine; tricycloalkylphosphines such as tricyclohexylphosphine; triarylphosphines such as triphenylphosphine and tritolylphosphine; trialkylphosphites such as trimethylphosphite, triethylphosphite and tributylphosphite; tricycloalkylphosphites such as tricyclohexylphosphite; triarylphosphites such as triphenylphosphite; imidazolinium salts such as 1, 3-bis(2, 4, 6-trimethylphenyl)imidazolinium chloride; diketones such as acetylacetone and octafluoroacetylacetone; amines such as trimethylamine, triethylamine, tripropylamine and tributylamine; 1, 1'-bis(diphenylphosphino)ferrocene; 2, 2'-bis(diphenylphosphino)-1, 1'-binaphthyl; 2-dicyclohexylphosphino-2', 6'-dimethoxybiphenyl; 2-dicyclohexylphosphino-2', 4', 6'-triisopropylbiphenyl; 2-(di-tert-butylphosphino)-2', 4', 6'-triisopropylbiphenyl; and 2-(di-tert-butylphosphino)biphenyl, and these may be used in combination.

The used amount of the ligand may be 0.00001 to 1 times in mole, preferably 0.02 to 0.5 times in mole to the compound of the general formula [2].

Examples of the base used in this reaction if desired include inorganic bases such as sodium bicarbonate, potassium carbonate, cesium carbonate and tripotassium phosphate, and organic bases such as triethylamine and diisopropylethylamine.

The used amount of the base may be 1 to 50 times in mole, preferably, 2 to 10 times in mole to the compound of the general formula [2].

The used amount of the compound of the general formula [3] may be 1 to 50 times in mole, preferably 1 to 2

times in mole to the compound of the general formula [2].

This reaction may be usually carried out under an inert gas (for example, nitrogen and/or argon) atmosphere at 0 to 160°C, preferably 20 to 120°C for 1 minute to 96 hours.

[Production Method 2]

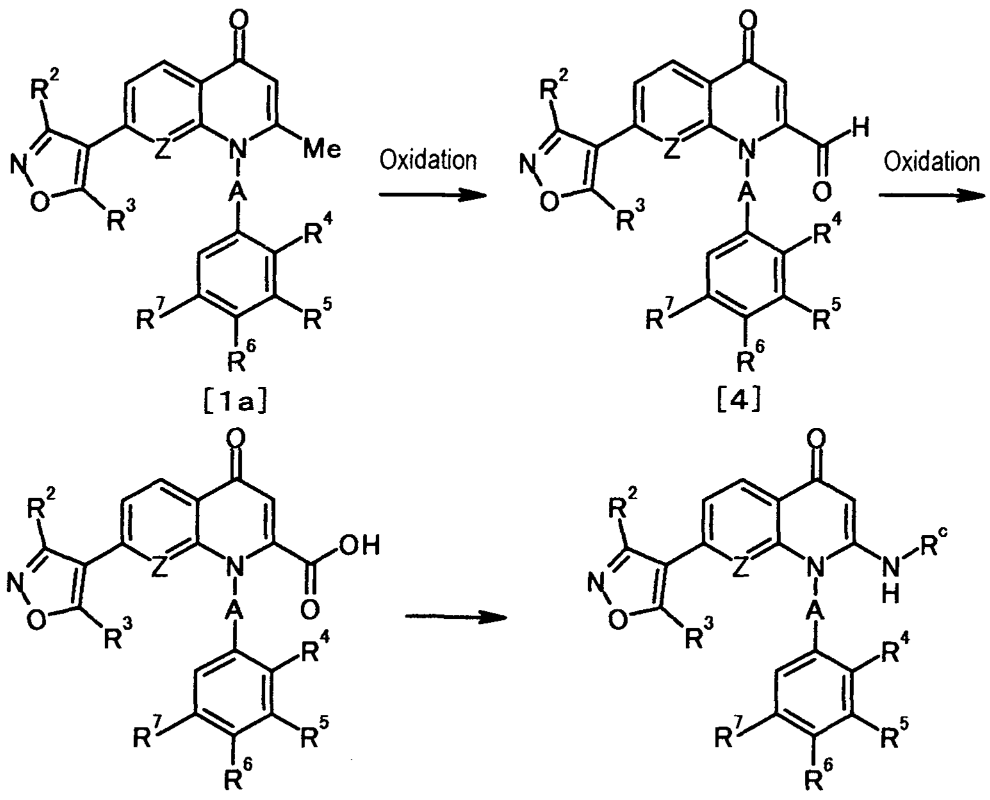


[1a] [4]

[5] [1b]

wherein $R^c$ represents an amino protecting group; $R^2$, $R^3$, $R^4$, $R^5$, $R^6$, $R^7$, Z and A have the same meanings as described above.

(2-1)

The compound of the general formula [4] can be produced by reacting the compound of the general formula [1a] with an oxidizing agent.

The solvent used in this reaction is not particularly limited as long as it does not affect the reaction, but for example, halogenated hydrocarbons, ethers and alcohols can be mentioned, and these solvents may be used in combination.

As preferable solvents, halogenated hydrocarbons and ethers can be mentioned, and dioxane is preferable.

As the oxidizing agent used in this reaction, for example, selenium dioxide can be mentioned.

The used amount of the oxidizing agent may be 1 to 50 times in mole, preferably 1 to 5 times in mole to the compound of the general formula [1a].

This reaction may be usually carried out at 0 to 150°C, preferably 60 to 100°C for 30 minutes to 24 hours.

(2-2)

The compound of the general formula [5] can be produced by reacting the compound of the general formula [4] with an oxidizing agent in the presence or absence of an acid, in the presence or absence of a base, in the presence or absence of a scavenger, and in the presence or absence of a buffer.

The solvent used in this reaction is not particularly limited as long as it does not affect the reaction, but for example, halogenated hydrocarbons, ethers, alcohols and water can be mentioned, and these solvents may be used

in combination.

As preferable solvents, a mixed solvent of ethers, alcohols and water can be mentioned, and a mixed solvent of tetrahydrofuran, 2-methyl-2-propanol and water is preferable.

The used amount of the solvent is not particularly limited, but preferably 1 to 100 times in volume (v/w), more preferably 2 to 50 times in volume (v/w) to the compound of the general formula [4].

Examples of the oxidizing agent used in this reaction include chromates such as chromium oxide (VI) and sodium dichromate; permanganates such as potassium permanganate, barium permanganate, calcium permanganate and magnesium permanganate; silver (I) oxide; and sodium chlorite.

The used amount of the oxidizing agent may be 1 to 20 times in mole, preferably 2 to 5 times in mole to the compound of the general formula [4].

Examples of the acid used in this reaction if desired include mineral acids such as sulfuric acid, hydrochloric acid and nitric acid.

The used amount of the acid may be 1 to 1000 times in mole to the compound of the general formula [4].

Examples of the base used in this reaction if desired include inorganic bases such as sodium hydroxide and potassium hydroxide.

The used amount of the base may be 1 to 1000 times in mole to the compound of the general formula [4].

Examples of the scavenger used in this reaction if desired include olefins such as 2-methyl-2-butene.

The used amount of the scavenger may be 1 to 100 times in mole, preferably 10 to 20 times in mole to the compound of the general formula [4].

When sodium chlorite is used as the oxidizing agent, it is preferable to use the scavenger.

Examples of the buffer used in this reaction if desired include inorganic phosphates such as sodium dihydrogen phosphate and potassium dihydrogen phosphate.

The used amount of the buffer may be 1 to 100 times in mole, preferably 10 to 20 times in mole to the compound of the general formula [4].

When sodium chlorite is used as the oxidizing agent, it is preferable to use the buffer.

This reaction may be usually carried out at 0 to 150°C, preferably 10 to 100°C.

(2-3)

The compound of the general formula [1b] can be produced by subjecting the compound of the general formula [5] to a one-pot reaction in the presence of a base, in the presence of an alcohol, with an azidation agent.

This reaction can be carried out by the method described in or based on Journal of the American Chemical Society, Vol. 94, pages 6203 to 6205 (1972).

The solvent used in this reaction is not particularly limited as long as it does not affect the reaction, but for example, alcohols, aromatic hydrocarbons and water can be mentioned, and these solvents may be used in combination.

As preferable solvents, alcohols can be mentioned, and 2-methyl-2-propanol is preferable.

The used amount of the solvent is not particularly limited, but preferably 1 to 100 times in volume (v/w), more preferably 10 to 50 times in volume (v/w) to the compound of the general formula [5].

Examples of the azidation agent used in this reaction include diphenylphosphoryl azide.

The used amount of the azidation agent may be 1 to 20 times in mole, preferably 1 to 4 times in mole to the compound of the general formula [5].

The used amount of the alcohol may be 1 to 100 times in mole, preferably 10 to 50 times in mole to the compound of the general formula [5].

This reaction may be usually carried out at 0 to 150°C, preferably 60 to 100°C.

(2-4)

The compound of the general formula [1b] can be produced by subjecting the compound of the general formula [5] to Schmidt reaction, Hofmann rearrangement or Lossen rearrangement.

These reactions can be carried out by the method described in or based on Strategic Applications of Named Reactions in Organic Synthesis, pages 210 to 211, pages 266 to 267, pages 396 to 397, Elsevier, INC.

Deprotection of the amino group of the compound of the general formula [1b] can be carried out by, for example, the method described in W. Greene et al., Protective Groups in Organic Synthesis, Fourth edition, pages 696 to 926, John Wiley & Sons, INC.

[Production Method 3]

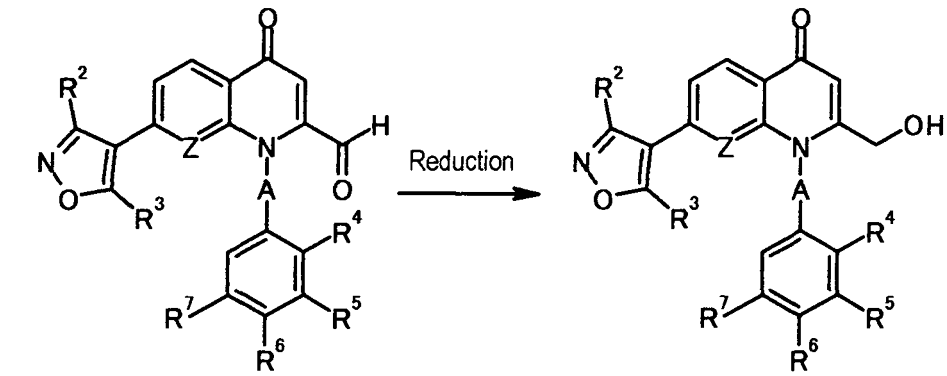


[4] [1c]

wherein $R^2$, $R^3$, $R^4$, $R^5$, $R^6$, $R^7$, Z and A have the same meanings as described above.

The compound of the general formula [1c] can be produced by reacting the compound of the general formula [4] with a reducing agent.

The solvent used in this reaction is not particularly limited as long as it does not affect the reaction, but for example, ethers and alcohols can be mentioned, and these solvents may be used in combination.

As preferable solvents, methanol and tetrahydrofuran can be mentioned.

The used amount of the solvent is not particularly limited, but preferably 1 to 1000 times in volume (v/w), more preferably 10 to 100 times in volume (v/w) to the compound of the general formula [4].

Examples of the reducing agent used in this reaction include metal hydrides such as lithium aluminum hydride, sodium bis(2-methoxyethoxy)aluminum hydride, diisobutylaluminum hydride, sodium borohydride, sodium cyanoborohydride and sodium triacetoxyborohydride.

The used amount of the reducing agent may be 1 to 100 times in mole, preferably 1 to 20 times in mole to the compound of the general formula [4].

This reaction may be usually carried out under an inert gas (for example, nitrogen and/or argon) atmosphere at -78 to 100°C, preferably -78 to 30°C for 1 minute to 24 hours.

[Production Method 4]

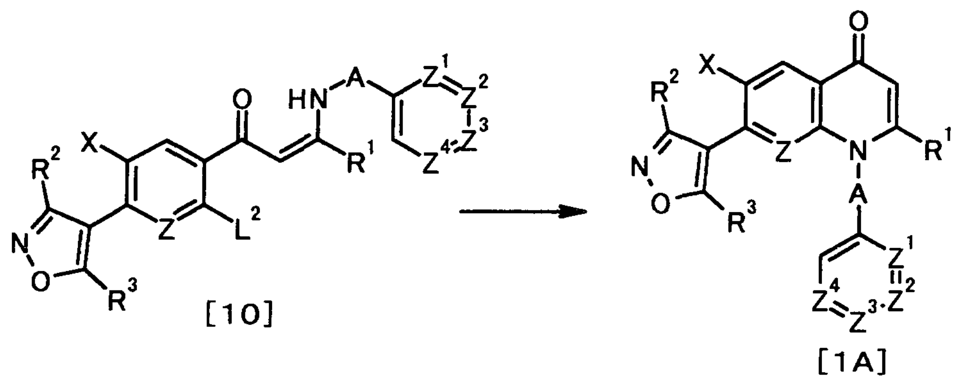

[10] [1A]

wherein $L^2$ represents a leaving group; $R^1$, $R^2$, $R^3$, X, Z, $Z^1$, $Z^2$, $Z^3$, $Z^4$ and A have the same meanings as described above.

The compound of the general formula [1A] can be produced by subjecting the compound of the general formula [10] to the ring closure reaction in the presence or absence of a base based on, for example, the method described in Collection of Czechoslovak Chemical Communications, Vol. 69, pages 822 to 832 (2004).

The solvent used in this reaction is not particularly limited as long as it does not affect the reaction, but for example, ethers, amides and dimethylsulfoxide can be mentioned, and these solvents may be used in combination.

As preferable solvents, acetonitrile, amides and dimethylsulfoxide can be mentioned, and acetonitrile, N, N-dimethylformamide and dimethylsulfoxide are preferable.

The used amount of the solvent is not particularly limited, but preferably 1 to 1000 times in volume (v/w), more preferably 1 to 100 times in volume (v/w) to the compound of the general formula [10].

Examples of the base used in this reaction if desired include sodium bicarbonate, potassium carbonate, potassium tert-butoxide and sodium hydride.

The used amount of the base may be 1 to 5 times in mole to the compound of the general formula [10].

This reaction may be usually carried out at 0 to 140°C, preferably 40 to 120°C for 30 minutes to 24 hours.

Next, the methods for producing the compounds of the general formula [2] and the general formula [10] which are the raw materials of the compound of the present invention will be described.

[Production Method A]

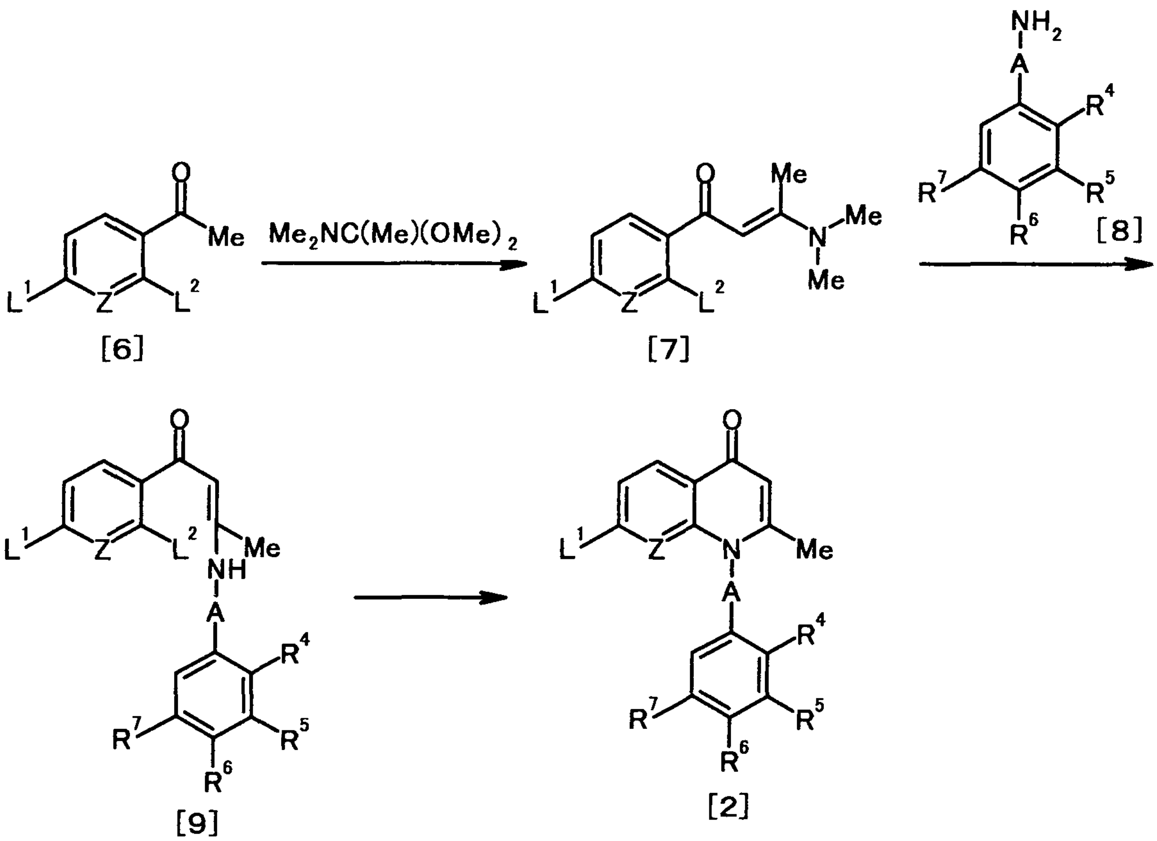


wherein $R^4$, $R^5$, $R^6$, $R^7$, Z, A, $L^1$ and $L^2$ have the same meanings as described above.

<u>(A-A1)</u>

The compound of the general formula [7] can be produced by reacting the compound of the general formula [6] with N, N-dimethylacetamide dimethyl acetal in the presence or absence of an acid anhydride based on, for example, the method described in Collection of Czechoslovak Chemical Communications, Vol. 69, pages 822 to 832 (2004).

The solvent used in this reaction is not particularly limited as long as it does not affect the reaction, but for example, halogenated hydrocarbons, ethers, amides and aromatic hydrocarbons can be mentioned, and these solvents may be used in combination.

As preferable solvents, amides can be mentioned, and N, N-dimethylformamide and N, N-dimethylacetamide are preferable.

The used amount of the solvent is not particularly limited, but preferably 1 to 100 times in volume (v/w), more preferably 1 to 10 times in volume (v/w) to the compound of the general formula [6].

N, N-dimethylacetamide dimethyl acetal may be used as a solvent.

The used amount ofN, N-dimethylacetamide dimethyl acetal is 1 to 100 times in volume (v/w), more preferably

1 to 10 times in volume (v/w) to the compound of the general formula [6].

Examples of the acid anhydride used in this reaction if desired include acetic anhydride.

The used amount of the acid anhydride is 1 to 100 times in volume (v/w), more preferably 1 to 10 times in volume (v/w) to the compound of the general formula [6].

When Z is a nitrogen atom, it is preferable to use the acid anhydride.

This reaction may be usually carried out at 0 to 160°C, preferably 40 to 110°C for 30 minutes to 24 hours.

(A-2)

The compound of the general formula [9] can be produced by reacting the compound of the general formula [7] with the compound of the general formula [8] based on, for example, the method described in Collection of Czechoslovak Chemical Communications, Vol. 69, pages 822 to 832 (2004).

The solvent used in this reaction is not particularly limited as long as it does not affect the reaction, but for example, halogenated hydrocarbons, ethers, alcohols, esters, aromatic hydrocarbons, and organic acids such as formic acid and acetic acid can be mentioned, and these solvents may be used in combination.

As preferable solvents, esters and organic acids can be mentioned, and ethyl acetate and acetic acid are preferable.

The used amount of the solvent is not particularly limited, but preferably 1 to 1000 times in volume (v/w), more preferably 1 to 50 times in volume (v/w) to the compound of the general formula [7].

This reaction may be usually carried out at 0 to 110°C, preferably 20 to 80°C for 30 minutes to 24 hours.

(A-3)

The compound of the general formula [2] can be produced by subjecting the compound of the general formula [9] to the ring closure reaction in the presence or absence of a base based on, for example, the method described in Collection of Czechoslovak Chemical Communications, Vol. 69, pages 822 to 832 (2004).

The solvent used in this reaction is not particularly limited as long as it does not affect the reaction, but for example, ethers, amides and dimethylsulfoxide can be mentioned, and these solvents may be used in combination.

As preferable solvents, acetonitrile, amides and dimethylsulfoxide can be mentioned, and acetonitrile, N, N-dimethylformamide and dimethylsulfoxide are preferable.

The used amount of the solvent is not particularly limited, but preferably 1 to 1000 times in volume (v/w), more preferably 10 to 100 times in volume (v/w) to the compound of the general formula [9].

Examples of the base used in this reaction if desired include sodium bicarbonate, potassium carbonate, potassium tert-butoxide and sodium hydride.

The used amount of the base may be 1 to 5 times in mole to the compound of the general formula [9].

This reaction may be usually carried out at 0 to 140°C, preferably 40 to 120°C for 30 minutes to 24 hours.

[Production Method B]

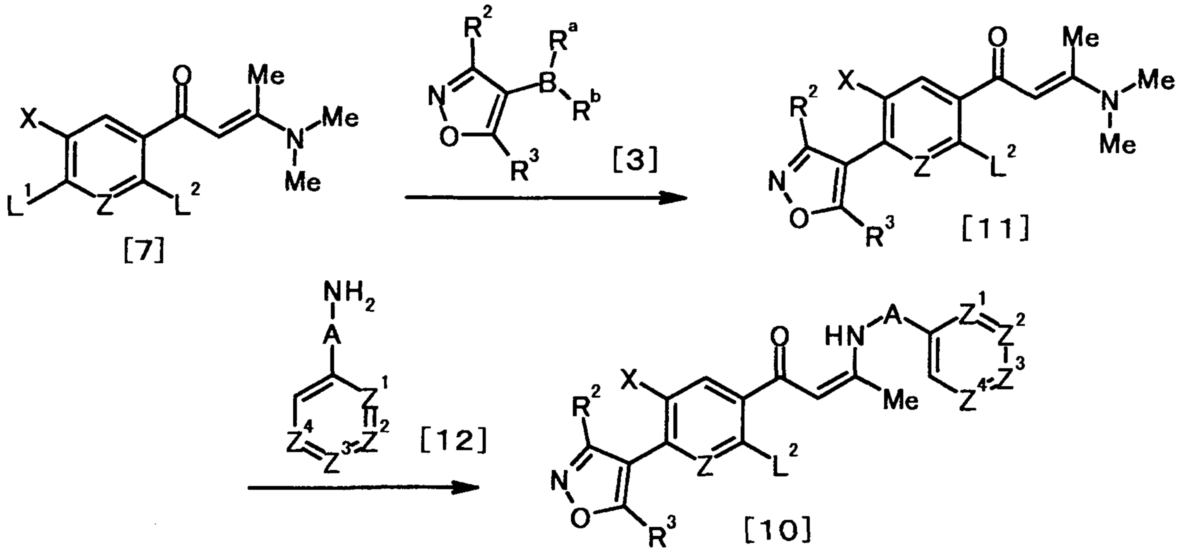


wherein $R^2$, $R^3$, $R^a$, $R^b$, X, Z, $Z^1$, $Z^2$, $Z^3$, $Z^4$, A, $L^1$ and $L^2$ have the same meanings as described above.

(B-1)

The compound of the general formula [11] can be produced by reacting the compound of the general formula [7] with the compound of the general formula [3] in the presence or absence of a base, in the presence of a palladium catalyst, and in the presence or absence of a ligand, based on Production Method 1.

(B-2)

Examples of the compound of the general formula [12] include benzylamine and 3-(aminomethyl)pyridine.

The compound of the general formula [10] can be produced by reacting the compound of the general formula [11] with the compound of the general formula [12].

The solvent used in this reaction is not particularly limited as long as it does not affect the reaction, but for example, alcohols and esters can be mentioned, and these solvents may be used in combination.

As preferable solvents, ethanol and ethyl acetate can be mentioned.

The used amount of the solvent is not particularly limited, but preferably 1 to 1000 times in volume (v/w), more preferably 10 to 100 times in volume (v/w) to the compound of the general formula [11].

This reaction may be usually carried out at 0 to 140°C, preferably 40 to 120°C for 30 minutes to 24 hours.

In the compound used in the above production method, when isomers (for example, optical isomers, geometrical isomers and tautomers) are present, these isomers also can be used. When solvates, hydrates and various shaped crystals are present, these solvates, hydrates and various shaped crystals also can be used.

In the compound used in the above production method, the compound which has an amino group has been previously protected by a usual protecting group, and after the reaction, the protecting group can be deprotected by a method known per se.

Protection and deprotection of an amino group can be carried out by, for example, the method described in W. Greene et al., Protective Groups in Organic Synthesis, Fourth edition, pages 696 to 926, John Wiley & Sons, INC.

The compound obtained by the above production method can be derived to other compounds by subjecting the compound to a reaction known per se such as condensation, addition, oxidation, reduction, rearrangement, substitution, halogenation, dehydration or hydrolysis, or by appropriately combining those reactions.

When the compound of the present invention is used as a pharmaceutical, usually, formulation additives such as an excipient, a carrier and a diluent to be used in formulation may be appropriately mixed. They can be orally or parenterally administered in the usual manner in the form of tablets, capsules, powders, syrups, granules, pills, suspensions, emulsions, solutions, powder preparations, suppositories, eye drops, nasal drops, ear drops, patches, ointments or injections. Moreover, the administration method, the dose, and the frequency of administration may be selected

appropriately according to the age, the body weight, and the symptom of a patient. It may usually be administered orally or parenterally (e.g., injection, drip infusion and administration to the rectal site) to an adult in one to several divided doses at doses of 0.01 to 1000 mg/kg per day.

Next, utility of a representative compound of the present invention will be described in the following test examples.

Test Example 1-1: Anti-HIV activity

This example was carried out by reference to Journal of Clinical Microbiology, 2007, Vol. 45, pages 477 to 487.

An anti-HIV activity was evaluated using MaRBLE cells obtained by transfection of HPB-M(a) derived from human T-lymphocytes with a luciferase gene of which expressions were regulated by HTV-1 LTR and a CCR5 gene etc..

The MaRBLE cells were suspended in RPMI1640 containing 10% FCS (fetal calf serum) and penicillin/streptomycin. After infected with HIV-1 (JRCSF), the cells were seeded on a 96-well plate ($1\times10^5$ cells/well). As a control group, the same number of uninfected cells were seeded.

After incubating the plate under the condition of 37°C and 5% $CO_2$ for 2 hours, a fresh culture medium or a culture medium containing an appropriately diluted test compound was added to each well.

After further culturing at 37°C under 5% $CO_2$ for 7 days, an intracellular luciferase activity was measured using Dual-Glo Luciferase assay system (Promega).

The virus growth rate was calculated by the following equation.

$$\text{Virus growth rate (\%)} = (A/B) \times 100$$

A = (Firefly luciferase activity in the well with the compound) - (Firefly luciferase activity of uninfected cells)
B = (Firefly luciferase activity in the well without the compound) - (Firefly luciferase activity of uninfected cells)

The IC50 of the compound was calculated by applying virus growth rates of various concentrations to 4 parameter Logistic Model of curve-fitting software XLfit 4.

The results are shown in Table 1.

[Table 1]

| Example No. | Anti-HIV Activity $IC_{50}$($\mu$mol/L) | Example No. | Anti-HIV Activity $IC_{50}$($\mu$mol/L) |
|---|---|---|---|
| 2 | 0.0014 | 20 | 0.0026 |
| 4 | 0.00038 | 22 | 0.0006 |
| 5-2 | 0.00027 | 24 | 0.00054 |
| 7 | 0.00020 | 25 | 0.00064 |
| 10 | 0.0028 | 26 | 0.00023 |
| 11 | 0.00062 | 27 | 0.0003 |
| 16 | 0.0072 | 28 | 0.0003 |
| 17 | 0.00026 | 29 | 0.0012 |
| 18 | 0.0035 | 31 | 0.0018 |
| 19 | 0.0017 | 34 | 0.00048 |

Test Example 1-2: Anti-HIV activity

This example was carried out by reference to Journal of Clinical Microbiology, 2007, Vol. 45, pages 477 to 487.

An anti-HIV activity was evaluated using MaRBLE cells obtained by transfection of HPB-M(a) derived from human T-lymphocytes with a luciferase gene of which expressions were regulated by HTV-1 LTR and a CCR5 gene etc..

A plate containing a drug solution was made by dispensing a fresh culture medium or an appropriately diluted test compound to a 96-well plate.

MaRBLE cells were suspended in RPMI1640 containing 10% FCS (fetal calf serum) and penicillin/streptomycin. After infected with HIV-1 (JRCSF), the cells were seeded on the above-described plate containing a drug solution ($1\times10^5$ cells/well). As a control group, the same number of uninfected cells were seeded.

After culturing at 37°C under 5% $CO_2$ for 7 days, an intracellular luciferase activity was measured using Dual-Glo Luciferase assay system (Promega).

The virus growth rate was calculated by the following equation.

$$\text{Virus growth rate (\%)} = (A/B) \times 100$$

A = (Firefly luciferase activity in the well with the compound) - (Firefly luciferase activity of uninfected cells)
B = (Firefly luciferase activity in the well without the compound) - (Firefly luciferase activity of uninfected cells)

The IC50 of the compound was calculated by plotting the concentration with the logarithm and by plotting the virus growth rate with a real number, using the FORECAST function (linear regression technique) of Microsoft Office Excel 2003.
The results are shown in Table 2.

[Table 2]

| Example No. | Anti-HIV Activity $IC_{50}$($\mu$mol/L) |
|---|---|
| 12 | 0.0019 |
| 13 | 0.0029 |
| 14 | 0.00055 |
| 33 | 0.00087 |
| 35 | 0.0026 |

Test Example 2: Pharmacokinetic study in mice (oral administration)

The compounds of Examples 2, 4, 5, 12 and 16 were used as test compounds.
A test compound suspended in 0.5% methylcellulose (2.5 mg/mL) was orally administered at 10 mL/kg to male ICR mice (6 week-old, three animals in a group). At 12 hours after the administration, the blood of the mice was collected, and the concentrations of the test compound in the serum were measured by HPLC.
As a result, the serum concentration of the compound of Example 4 was 0.5 $\mu$g/mL or more, showing good pharmacokinetics. The serum concentrations of the compounds of Examples 2, 5, 12 and 16 were also 0.5 $\mu$g/mL or more, showing good pharmacokinetics.

Test Example 3: Single-dose oral toxicity study in mice

The compounds of Examples 2, 4 and 12 were used as test compounds. 10 mL/kg of a test compound suspended in 0.5% methylcellulose was orally administered to male ICR mice (6 week-old, one animal in a group).
On the next day after the administration, the conditions of the mice were observed.
As a result, the compound of Example 4 showed good safety at a dose of 750 mg/kg. The compound of Example 2 showed good safety at a dose of 2000 mg/kg. The compound of Example 12 showed good safety at a dose of 1000 mg/kg.

Test Example 4: In vivo micronucleus study in mice

The compounds of Examples 2, 4 and 12 were used as test compounds.
A test compound suspended in 0.5% methylcellulose was orally administered to male ICR mice (8 week-old, three animals in a group) twice at a 24 hour interval. To a control group, 0.5% methylcellulose was orally administered in a similar manner.
At 24 hours after the final administration, the mice in the highest dose group in which all the animals were alive were killed by exsanguination under ether anesthesia, and one thigh bone was collected from each animal. After cutting the collected thigh bone at the proximal end, bone marrow cells were washed out with fetal calf serum to a centrifuge tube using a disposable syringe, and after centrifugation (1000 rpm, 5 minutes, HITACHI 05PR-22), a part of the supernatant was discarded to obtain a suspension of bone marrow cells. This suspension was smeared on a slide glass, and then dried followed by methanol fixation to prepare a sample.
The sample was observed by acridine orange staining.
Under a fluorescent microscope, the presence or absence of the micronucleus was observed for 1000 or more polychromatic erythrocytes (PCE) per one animal, and the appearance frequency of micronucleated polychromatic erythrocytes (MNPCE) (frequency of MNPCE) was calculated according to the following equation. The sample of which frequency of MNPCE exceeds 0.5% was judged positive.

$$\text{Frequency of MNPCE (\%)} = (\text{MNPCE number}/\text{Observed PCE number}) \times 100$$

The compounds of Examples 2, 4 and 12 were negative.

Examples

Next, the present invention will be described by reference of Reference Examples and Examples, but the present invention is not restricted to them.

The mixing ratios of the eluents are volume ratios. Unless otherwise stated, the carrier in a silica gel column chromatography is silica gel 60N (spherical, neutral, 63 to 210 μm) manufactured by Kanto Chemical Co., Inc. Flash column chromatography is a medium pressure liquid chromatograph, YFLC-Wprep2XYN manufactured by Yamazen Corporation. Unless otherwise stated, the silica gel column is Hi-Flash column, W001, W002, W003 or W004 manufactured by Yamazen Corporation.

In each Example, each abbreviation means as follows.

Ac: acetyl
Boc: tert-butoxycarbonyl
DBU: 1, 8-diazabicyclo[5.4.0]undec-7-ene
DMADA: N, N-dimethylacetamide dimethyl acetal
DMF: N, N-dimethylformamide
DMSO: dimethylsulfoxide
DPPA: diphenylphosphoryl azide
Me: methyl
S-Phos: 2-dicyclohexylphosphino-2', 6'-dimethoxybiphenyl
TFA: trifluoroacetic acid
THF: tetrahydrofuran
DMSO-$d_6$: deutero dimethylsulfoxide
s: singlet
br: broad
brs: broad singlet
d: doublet
dd: double doublet
ddd: double double doublet
t: triplet
q: quartet
ABq: AB quartet
m: multiplet

Reference Example 1

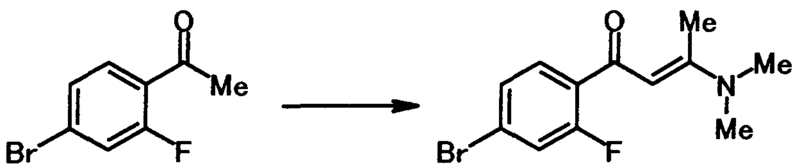

A solution of 12.3 g of 4-bromo-2-fluoroacetophenone in 25.0 g of DMADA was heated under reflux for 3 hours and 15 minutes. The solvent was distilled off under reduced pressure, and the resulting residue was purified by silica gel column chromatography [eluent; hexane : ethyl acetate = 6:1 → 1:1], and suspended in diisopropyl ether, and the solid was filtered to obtain 7.96 g of (E)-1-(4-bromo-2-fluorophenyl)-3-dimethylamino-2-buten-1-one as a yellow white solid.
$^1$H-NMR ($CDCl_3$) δ:2.66 (s, 3H), 3.07 (s, 6H), 5.51 (d, J = 2.0 Hz, 1H), 7.23 (dd, J = 10.0, 1.8 Hz, 1H), 7.31 (ddd, J = 8.2, 1.8, 0.5 Hz, 1H), 7.62 (dd, J 8.2, 8.2 $H_2$, 1H).

Reference Example 2

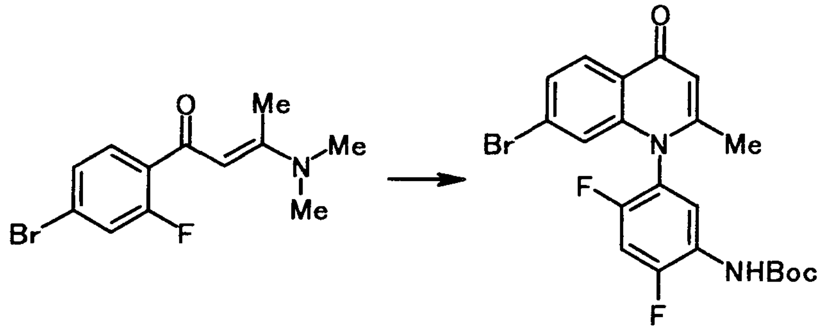

A solution of 3.00 g of (E)-1-(4-bromo-2-fluorophenyl)-3-dimethylamino-2-buten-1-one obtained in a similar manner as the method described in Reference Example 1 and 3.20 g of tert-butyl 5-amino-2, 4-difluorophenylcarbamate in 10.5 mL of acetic acid was heated under stirring at 40 to 50°C for 4 hours. To the reaction mixture were added a saturated aqueous sodium bicarbonate solution and ethyl acetate. The organic layer was separated, and after washing with a saturated aqueous sodium chloride solution, the layer was dried over anhydrous magnesium sulfate, and the solvent was distilled off under reduced pressure. To the solution of the resulting residue in 40 mL of DMF was added 4.35 g of potassium carbonate, and the mixture was heated under stirring at 60 to 70°C for 1 hour. After cooling the reaction mixture, ethyl acetate was added, and after washing with water and a saturated aqueous sodium chloride solution successively, the layer was dried over anhydrous magnesium sulfate, and the solvent was distilled off under reduced pressure. The resulting residue was suspended in a mixed solvent of ethyl acetate and diisopropyl ether, and the solid was filtered to obtain 3.86 g of 7-bromo-1-(5-tert-butoxycarbonylamino-2, 4-difluorophenyl)-2-methyl-1, 4-dihydro-4-oxoquinoline as a pale brown solid.
$^1$H-NMR (DMSO-$d_6$) $\delta$:1.44 (s, 9H), 2.05 (s, 3H), 6.29 (s, 1H), 6.90 (d, J = 1.6 Hz, 1H), 7.56 (dd, J = 8.5, 1.6 Hz, 1H), 7.76 (dd, J = 10.0, 10.0 Hz, 1H), 7.93 (dd, J = 8.1, 8.1 Hz, 1H), 8.10 (d, J = 8.5 Hz, 1H), 9.36 (brs, 1H).

Reference Example 3

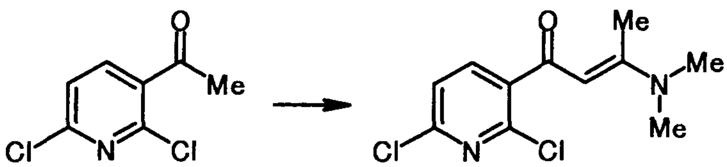

A solution of 8.00 g of 3-acetyl-2, 6-dichloropyridine in 12 mL of DMADA was heated under reflux for 2 hours. The solvent was distilled off under reduced pressure, and the resulting residue was purified by silica gel column chromatography [eluent; hexane : ethyl acetate = 3:1 $\rightarrow$ 1:3], and suspended in a mixed solvent of ethyl acetate and diisopropyl ether, and the solid was filtered to obtain 3.91 g of (E)-1-(2, 6-dichloropyridin-3-yl)-3-dimethylamino-2-buten-1-one as a yellow solid.
$^1$H-NMR ($CDCl_3$) $\delta$:2.67 (s, 3H), 3.08 (brs, 6H), 5.25 (s, 1H), 7.27 (d, J = 8.1 Hz, 1H), 7.74 (d, J =8.1 Hz, 1H).

Reference Example 4

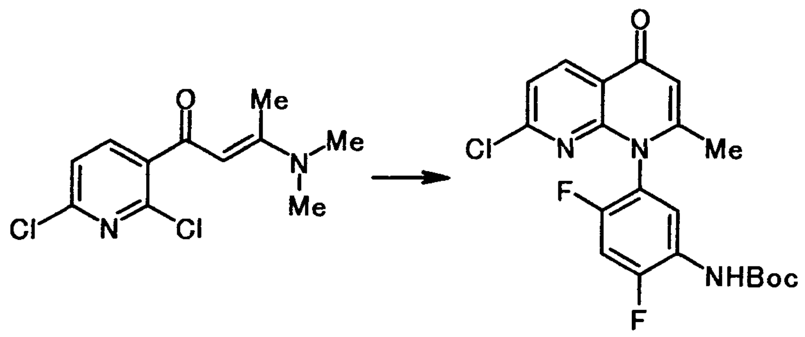

A solution of 3.00 g of (E)-1-(2, 6-dichloropyridin-3-yl)-3-dimethylamino-2-buten-1-one obtained in a similar manner as the method described in Reference Example 3 and 2.97 g of tert-butyl 5-amino-2, 4-difluorophenylcarbamate in 15 mL of acetic acid was heated under stirring at 70 to 80°C for 4 hours and 20 minutes. To the reaction mixture were added an aqueous sodium hydroxide solution and ethyl acetate. The organic layer was separated, and after washing with a saturated aqueous sodium chloride solution, the layer was dried over anhydrous magnesium sulfate, and the solvent was distilled off under reduced pressure. The resulting residue was suspended in a mixed solvent of ethyl acetate and diisopropyl ether, and the solid was filtered to obtain 3.34 g of a white solid. To the solution of 3.34 g of the resulting white solid in 18 mL ofDMF was added 2.01 g of potassium carbonate, and the mixture was heated under stirring at 70 to 80°C for 1 hour and 30 minutes. To the reaction mixture were added water and ethyl acetate. The organic layer was separated, and after washing with water and a saturated aqueous sodium chloride solution successively, the layer was dried over anhydrous magnesium sulfate, and the solvent was distilled off under reduced pressure. The resulting residue was suspended in a mixed solvent of ethyl acetate and diisopropyl ether, and the solid was filtered to obtain 2.62 g of 1-(5-tert-butoxycarbonylamino-2, 4-difluorophenyl)-7-chloro-2-methyl-1, 4-dihydro-4-oxo-1, 8-naphthyridine as a white solid.
$^1$H-NMR (DMSO-$d_6$) δ:1.44 (s, 9H), 2.11 (s, 3H), 6.37 (s, 1H), 7.51 (d, J = 8.3 Hz, 1H), 7.66 (dd, J = 10.1, 10.1 Hz, 1H), 7.86 (dd, J = 8.2, 8.2 Hz, 1H), 8.51 (d, J = 8.3 Hz, 1H), 9.26 (brs, 1H).

Reference Example 5

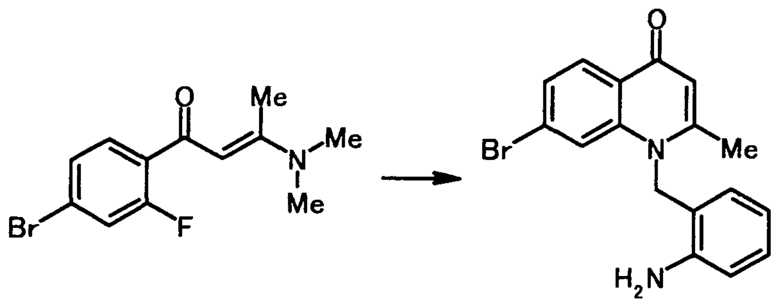

A solution of 2.83 g of (E)-1-(4-bromo-2-fluorophenyl)-3-dimethylamino-2-buten-1-one obtained in a similar manner as the method described in Reference Example 1 and 1.45 g of 2-aminobenzylamine in 50 mL of ethyl acetate was heated under reflux for 6 hours. The solvent was distilled off under reduced pressure, and to the solution of the resulting residue in 100 mL of DMSO was added 2.05 g of potassium carbonate, and the mixture was heated under stirring at 70 to 80°C for 3 hours and 5 minutes. To the reaction mixture was added ethyl acetate, and after washing with water and a saturated aqueous sodium chloride solution successively, the layer was dried over anhydrous magnesium sulfate, and the solvent was distilled off under reduced pressure. The resulting residue was purified by silica gel column chromatography [eluent; chloroform:acetone = 3:1], and suspended in ethyl acetate, and the solid was filtered to obtain 2.18 g of 1-(2-aminobenzyl)-7-bromo-2-methyl-1, 4-dihydro-4-oxoquinoline as a pale brown solid.
$^1$H-NMR (DMSO-$d_6$) δ:2.37 (s, 3H), 5.23 (s, 2H), 5.26 (s, 2H), 6.15 (dd, J = 7.4, 0.9 Hz, 1H), 6.22 (s, 1H), 6.43-6.48 (m, 1H), 6.79 (dd, J = 9.2, 1.0 Hz, 1H), 6.97-7.03 (m, 1H), 7.51 (dd, J = 8.5, 1.5 Hz, 1H), 7.56 (d, J = 1.5 Hz, 1H), 8.09 (d, J = 8.5 Hz, 1H).

Reference Example 6

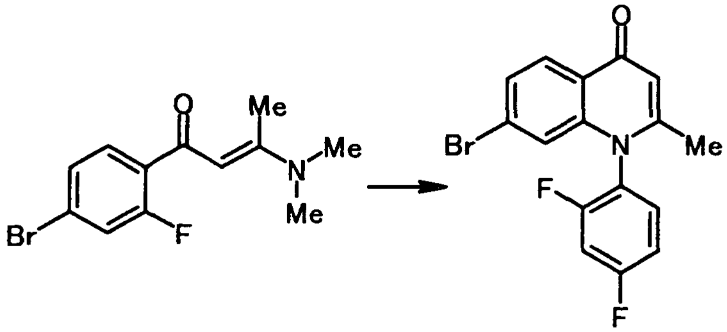

A solution of 2.30 g of (E)-1-(4-bromo-2-fluorophenyl)-3-dimethylamino-2-buten-1-one obtained in a similar manner as the method described in Reference Example 1 and 1.55 g of 2, 4-difluoroaniline in 5 mL of acetic acid was heated under stirring at 50 to 60°C for 25 minutes. To the reaction mixture were added a saturated aqueous sodium bicarbonate solution and ethyl acetate. The organic layer was separated, and after washing with a saturated aqueous sodium chloride solution, the layer was dried over anhydrous magnesium sulfate, and the solvent was distilled off under reduced pressure. To the solution of the resulting residue in 16 mL ofDMF was added 2.22 g of potassium carbonate, and the mixture was heated under stirring at 90°C for 24 minutes. To the reaction mixture were added water and ethyl acetate. The organic layer was separated, and after washing with water and a saturated aqueous sodium chloride solution successively, the layer was dried over anhydrous magnesium sulfate, and the solvent was distilled off under reduced pressure. The resulting residue was suspended in a mixed solvent of ethyl acetate and diisopropyl ether, and the solid was filtered to obtain 2.77 g of 7-bromo-1-(2, 4-difluorophenyl)-2-methyl-1, 4-dihydro-4-oxoquinoline as a pale brown solid. $^1$H-NMR (DMSO-$d_6$) 5:2.03 (s, 3H), 6.31 (d, J = 0.5 Hz, 1H), 6.86 (d, J = 1.5 Hz, 1H), 7.42-7.49 (m, 1H), 7.57 (dd, J = 8.5, 1.5 Hz, 1H), 7.74 (ddd, J = 11.0, 8.2, 2.1 Hz, 1H), 7.83 (ddd, J = 8.9, 8.9, 6.0 Hz, 1H), 8.10 (d, J = 8.5 Hz, 1H).

Reference Example 7

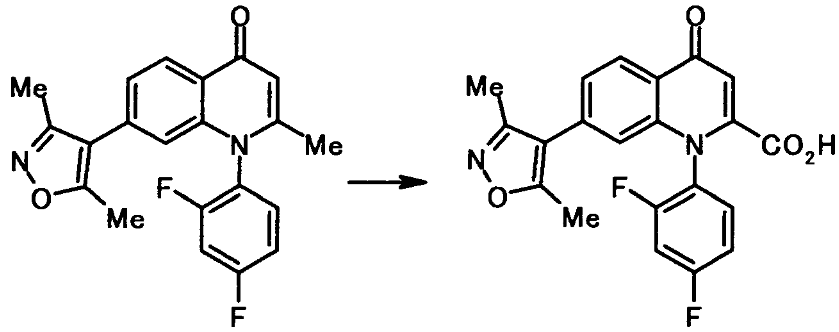

To a solution of 6.08 g of 1-(2, 4-difluorophenyl)-7-(3, 5-dimethylisoxazol-4-yl)-2-methyl-1, 4-dihydro-4-oxoquinoline obtained in a similar manner as the method described in Example 8 in 180 mL of dioxane was added 2.03 g of selenium dioxide, and the mixture was heated under reflux for 8 hours. To the reaction mixture were added a saturated aqueous sodium bicarbonate solution and ethyl acetate, and insoluble matters were filtered off. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform: methanol = 100:0 → 5:1]. To the solution of the resulting solid and 21 mL of 2-methyl-2-butene in 61 mL of 2-methyl-2-propanol and 61 mL of THF was added a previously prepared aqueous solution [a solution obtained by adding 2.34 g of sodium chlorite to the solution of 24.8 g of sodium dihydrogen phosphate dihydrate in 53 mL of water, followed by stirring at room temperature], and the mixture was stirred at room temperature for 2 hours. To the reaction mixture were added ethyl acetate and an aqueous solution of sodium hydroxide. The aqueous layer was separated, and after adjusting to pH 3 with hydrochloric acid, ethyl acetate was added. The organic layer was

separated, and after washing with a saturated aqueous sodium chloride solution, the layer was dried over anhydrous magnesium sulfate, and the solvent was distilled off under reduced pressure. The resulting residue was suspended in diisopropyl ether, and the solid was filtered to obtain 2.66 g of 1-(2, 4-difluorophenyl)-7-(3, 5-dimethylisoxazol-4-yl)-1, 4-dihydro-4-oxoquinoline-2-carboxylic acid as a yellow white solid.

$^1$H-NMR (DMSO-$d_6$) $\delta$:2.13 (s, 3H), 2.33 (s, 3H), 6.61 (s, 1H), 6.79 (brs, 1H), 7.30-7.37 (m, 1H), 7.52 (dd, J = 8.3, 1.5 Hz, 1H), 7.64 (ddd, J = 10.5, 8.9, 2.9 Hz, 1H), 7.76 (ddd, 8.9, 8.9, 5.9 Hz, 1H), 8.28 (d, J = 8.3 Hz, 1H).

Reference Example 8

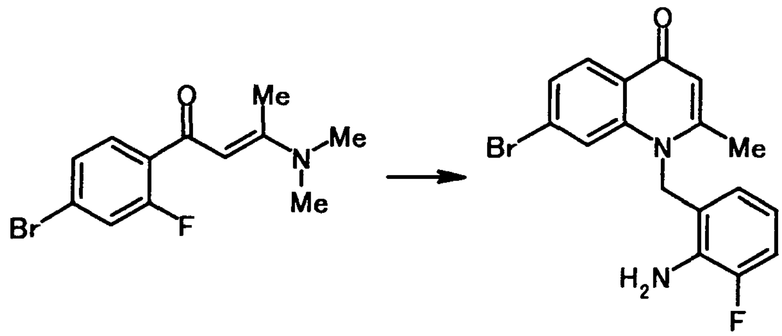

To a suspension of 1.57 g of (E)-1-(4-bromo-2-fluorophenyl)-3-dimethylamino-2-buten-1-one obtained in a similar manner as the method described in Reference Example 1 and 1.16 g of 2-amino-3-fluorobenzylamine hydrochloride in 5.5 mL of ethyl acetate were added 1 mL of triethylamine and 5 mL of ethanol, and the mixture was heated under stirring at 60 to 70°C for 1 hour. The solvent was distilled off under reduced pressure, and the resulting residue was purified by silica gel column chromatography [eluent; hexane : ethyl acetate = 3:2] to obtain 1.79 g of a pale brown solid.

To the solution of 1.79 g of the resulting solid in 15 mL of DMSO was added 1.79 g of potassium carbonate, and the mixture was heated under stirring at 100 to 110°C for 1 hour. To the reaction mixture was added ethyl acetate, and after washing with water and a saturated aqueous sodium chloride solution successively, the layer was dried over anhydrous magnesium sulfate, and the solvent was distilled off under reduced pressure. The resulting residue was suspended in a mixed solvent of ethyl acetate and diisopropyl ether, and the solid was filtered to obtain 1.00 g of 1-(2-amino-3-fluorobenzyl)-7-bromo-2-methyl-1, 4-dihydro-4-oxoquinoline as a brown solid.

Reference Example 9

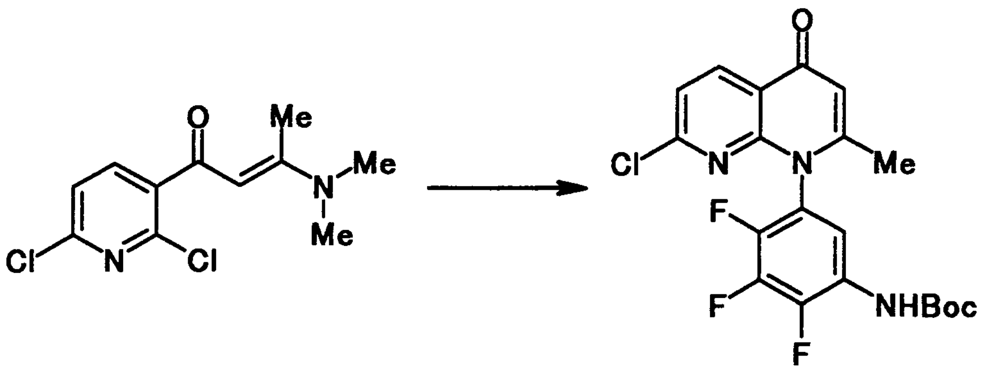

A solution of 13.8 g of (E)-1-(2, 6-dichloropyridin-3-yl)-3-dimethylamino-2-buten-1-one obtained in a similar manner as the method described in Reference Example 3 and 14.0 g of tert-butyl (5-amino-2, 3, 4-trifluorophenyl) carbamate in 70 mL of acetic acid was heated under stirring at 45 to 50°C for 3 hours and 30 minutes. After cooling the reaction mixture, ethyl acetate and a saturated aqueous sodium bicarbonate solution were added. The organic layer was separated, and after washing with a saturated aqueous sodium chloride solution, the layer was dried over anhydrous

magnesium sulfate, and the solvent was distilled off under reduced pressure. The resulting residue was purified by silica gel column chromatography [eluent; hexane : ethyl acetate = 2:1] to obtain 14.4 g of a yellow solid.

To the solution of 14.2 g of the resulting yellow solid in 140 mL of DMF was added 4.94 g of potassium carbonate, and the mixture was heated under stirring at 50°C for 3 hours. After cooling the reaction mixture, ethyl acetate and water were added. The organic layer was separated, and after washing with a saturated aqueous sodium bicarbonate solution and a saturated aqueous sodium chloride solution successively, the layer was dried over anhydrous sodium sulfate, and the solvent was distilled off under reduced pressure. The resulting residue was suspended in a mixed solvent of diisopropyl ether and hexane, and the solid was filtered to obtain 11.0 g of 1-(5-tert-butoxycarbonylamino-2, 3, 4-trifluorophenyl)-7-chloro-2-methyl-1, 4-dihydro-4-oxo-1, 8-naphthyridine as a yellow brown solid.

$^1$H-NMR (DMSO-$d_6$) δ:1.45 (s, 9H), 2.15 (s, 3H), 6.39 (s, 1H), 7.54 (d, J = 8.3 Hz, 1H), 7.73-7.81 (m, 1H), 8.52 (d, J = 8.3 Hz, 1H), 9.57 (brs, 1H).

Reference Example 10

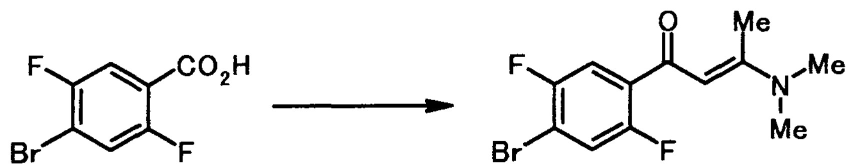

To a solution of 3.00 g of 4-bromo-2, 5-difluorobenzoic acid in 30 mL of ethyl acetate were added 0.1 mL ofDMF and 1.66 g of thionyl chloride, and the mixture was heated under reflux for 45 minutes. 0.30 g of thionyl chloride was added, followed by heating under reflux for further 30 minutes. After distilling off the solvent under reduced pressure, 20 mL of toluene was added, and the mixture was added dropwise to a previously prepared solution of 1.61 g ofN, O-dimethylhydroxylamine hydrochloride and 1.77 g of sodium hydroxide in 17 mL of water at 8 to 12°C. To the reaction mixture were added ethyl acetate and water. The organic layer was separated, and the aqueous layer was extracted with ethyl acetate. The organic layer and the extract were combined, and after washing with a saturated aqueous sodium chloride solution, the mixture was dried over anhydrous sodium sulfate, and the solvent was distilled off under reduced pressure. To the solution of the resulting residue in 25 mL of THF, 6.3 mL of a solution of 3.0 mol/L methylmagnesium bromide in diethyl ether was added dropwise under water cooling for 20 minutes. The reaction mixture was poured into ice water, and ethyl acetate and 6 mol/L hydrochloric acid were added. The organic layer was separated, and the aqueous layer was extracted with ethyl acetate. The organic layer and the extract were combined, and after washing with water and a saturated aqueous sodium chloride solution successively, the layer was dried over anhydrous magnesium sulfate, and the solvent was distilled off under reduced pressure. The solution of the resulting residue in 9 mL of DMADA was heated under reflux for 1 hour. To the reaction mixture were added ethyl acetate and water. The organic layer was separated, and the aqueous layer was extracted with ethyl acetate. The organic layer and the extract were combined, and after washing with water and a saturated aqueous sodium chloride solution successively, the layer was dried over anhydrous sodium sulfate, and the solvent was distilled off under reduced pressure. The resulting solid was suspended in a mixed solvent of diisopropyl ether, ethyl acetate and hexane, and the solid was filtered to obtain 1.68 g of (E)-1-(4-bromo-2, 5-difluorophenyl)-3-dimethylamino-2-buten-1-one as a purple solid.

$^1$H-NMR ($CDCl_3$) δ:2.66 (s, 3H), 3.08 (brs, 6H), 5.50 (s, 1H), 7.24-7.31 (m, 1H), 7.52 (dd, J = 8.9, 6.2 Hz, 1H).

Reference Example 11

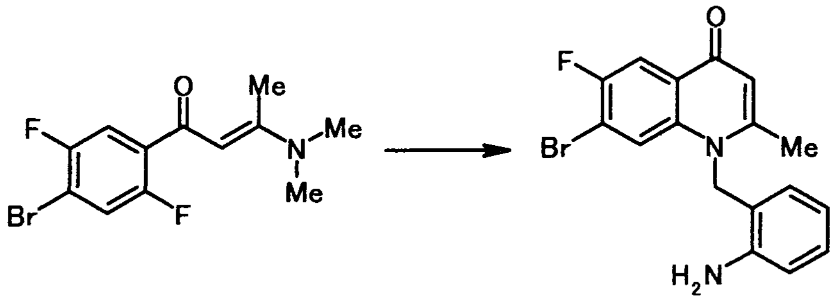

A solution of 1.50 g of (E)-1-(4-bromo-2, 5-difluorophenyl)-3-dimethylamino-2-buten-1-one obtained in a similar manner as the method described in Reference Example 10 and 0.78 g of 2-(aminomethyl)aniline in 15 mL of ethyl acetate was heated under stirring at 50 to 60°C for 4 hours. To the reaction mixture were added ethyl acetate and water. The organic layer was separated, and after washing with a saturated aqueous sodium chloride solution, the layer was dried over anhydrous sodium sulfate, and the solvent was distilled off under reduced pressure. The resulting solid was suspended in diisopropyl ether, and the solid was filtered to obtain 1.14 g of a pale yellow solid.

To the solution of 1.00 g of the resulting pale yellow solid in 10 mL of DMF was added 440 mg of potassium tert-butoxide, and the mixture was heated under stirring at 50 to 60°C for 1 hour. To the reaction mixture were added water and ethyl acetate. The organic layer was separated, and after washing with water and a saturated aqueous sodium chloride solution successively, the layer was dried over anhydrous magnesium sulfate, and the solvent was distilled off under reduced pressure. The resulting solid was suspended in a mixed solvent of diisopropyl ether and ethyl acetate, and the solid was filtered to obtain 0.66 g of 1-(2-aminobenzyl)-7-bromo-6-fluoro-2-methyl-1, 4-dihydro-4-oxoquinoline as a pale brown solid. $^1$H-NMR ($CDCl_3$) $\delta$:2.42 (s, 3H), 3.73 (brs, 2H), 5.13 (s, 2H), 6.29 (s, 1H), 6.43 (d, J = 7.6 Hz, 1H), 6.68-6.77 (m, 1H), 6.82-6.88 (m, 1H), 7.13-7.21 (m, 1H), 7.45 (d, J = 5.4 Hz, 1H), 8.15 (d, J = 8. 6 Hz, 1H).

Reference Example 12

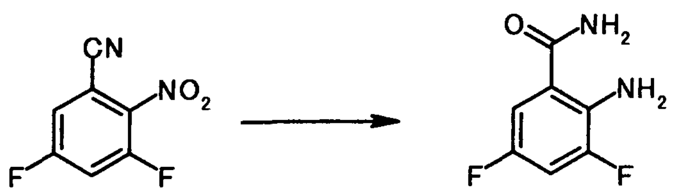

A suspension of 1.50 g of 3, 5-difluoro-2-nitrobenzonitrile, 2.28 g of iron powder and 261 mg of ammonium chloride in 15 mL of ethanol and 15 mL of water was heated under reflux for 2 hours and 20 minutes. After cooling the reaction mixture, insoluble matters were filtered off, and the filter residue was washed with ethanol. The filtrate and the wash liquid were combined, and the solvent was distilled off under reduced pressure. To the resulting residue were added ethyl acetate and water. The organic layer was separated, and after washing with a saturated aqueous sodium chloride solution, the layer was dried over anhydrous sodium sulfate, and the solvent was distilled off under reduced pressure. The resulting solid was suspended in diisopropyl ether, and the solid was filtered to obtain 818 mg of 2-amino-3, 5-difluorobenzamide as a yellow solid.
$^1$H-NMR ($CDCl_3$) $\delta$:5.34-5.96 (m, 3H), 6.38-6.42 (br, 1H), 6.89-6.98 (m, 2H).

Reference Example 13

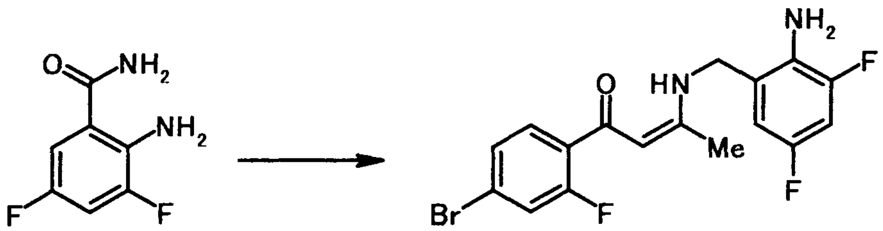

To a solution of 818 mg of 2-amino-3, 5-difluorobenzamide in 30 mL of THF, under nitrogen atmosphere at 1 to 5°C, 13.2 mL of a solution of a borane-tetrahydrofuran complex in tetrahydrofuran (1.08 mol/L) was added dropwise over 7 minutes, and the mixture was heated under reflux for 2 hours and 33 minutes. Under ice cooling, to the reaction mixture was added 30 mL of 1 mol/L hydrochloric acid, and the mixture was heated under reflux for 45 minutes. After cooling the reaction mixture, the mixture was adjusted to pH 8 to 9 with a 1 mol/L aqueous sodium hydroxide solution, and ethyl acetate was added. The organic layer was separated, and the aqueous layer was extracted with ethyl acetate. The organic layer and the extract were combined, and after washing with a saturated aqueous sodium chloride solution, the mixture was dried over anhydrous sodium sulfate, and the solvent was distilled off under reduced pressure. To the resulting residue were added 10 mL of ethyl acetate and 1.00 g of (E)-1-(4-bromo-2-fluorophenyl)-3-dimethylamino-2-buten-1-one obtained in a similar manner as the method described in Reference Example 1, and the mixture was heated under stirring at 49 to 75°C for 2 hours and 20 minutes. To the reaction mixture were added ethyl acetate and a saturated aqueous sodium bicarbonate solution. The organic layer was separated, and after washing with a saturated aqueous sodium chloride solution, the layer was dried over anhydrous sodium sulfate, and the solvent was distilled off under reduced pressure. The resulting solid was suspended in diisopropyl ether, and the solid was filtered to obtain 547 mg of (Z)-3-(2-amino-3, 5-difluorobenzylamino)-1-(4-bromo-2-fluorophenyl)-2-buten-1-one as a pale yellow solid.
$^1$H-NMR (DMSO-$d_6$) δ:2.08 (s, 3H), 4.50 (d, J = 5.7 Hz, 2H), 5.09 (brs, 2H), 5.55 (d, J = 2.0 Hz, 1H), 6.79 (d, J = 9.3 Hz, 1H), 7.02-7.14 (m, 1H), 7.47 (dd, J = 8.2, 1.7 Hz, 1H), 7.58 (dd, J = 10.5, 1.7 Hz, 1H), 7.63 (dd, J = 8.2, 8.2 Hz, 1H), 11.35 (t, J = 5.7 Hz, 1H).

<u>Reference Example 14</u>

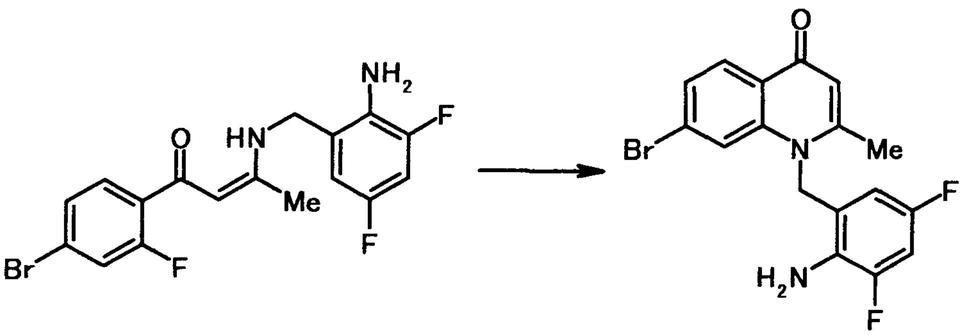

A suspension of 547 mg of (Z)-3-(2-amino-3, 5-difluorobenzylamino)-1-(4-bromo-2-fluorophenyl)-2-buten-1-one obtained in a similar manner as the method described in Reference Example 13 and 189 mg of potassium carbonate in 5 mL of DMSO was heated under stirring at 48 to 65°C for 4 hours and 15 minutes. To the reaction mixture were added ethyl acetate and water. The organic layer was separated, and the aqueous layer was extracted with ethyl acetate. The organic layer and the extract were combined, and after washing with a saturated aqueous sodium chloride solution, the mixture was dried over anhydrous sodium sulfate, and the solvent was distilled off under reduced pressure. The resulting residue was suspended in a mixed solvent of diisopropyl ether and ethyl acetate, and the solid was filtered to obtain 253 mg of 1-(2-amino-3, 5-difluorobenzyl)-7-bromo-2-methyl-1, 4-dihydro-4-oxoquinoline as a white solid.
$^1$H-NMR (DMSO-$d_6$) δ:2.37 (s, 3H), 5.16 (brs, 2H), 5.36 (brs, 2H), 5.80-5.90 (m, 1H), 6.23 (s, 1H), 7.03-7.12 (m, 1H), 7.50-7.57 (m, 1H), 7.58 (brs, 1H), 8.10 (d, J = 8.5 Hz, 1H).

<u>Reference Example 15</u>

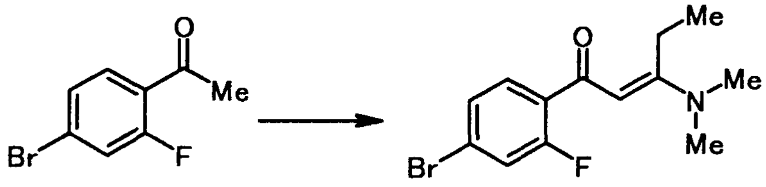

To a solution of 1, 1-dimethoxy-N, N-dimethylpropan-1-amine in 8 mL of methanol (20%(w/w)) was added 1.00 g of 1-(4-bromo-2-fluorophenyl)ethanone, and the mixture was heated under reflux for 40 minutes. The solvent was distilled off under reduced pressure, and hexane was added to the resulting residue. The mixture was cooled with dry ice, and the solid was filtered to obtain 1.09 g of (E)-1-(4-bromo-2-fluorophenyl)-3-dimethylamino-2-penten-1-one as an orange solid.

$^1$H-NMR ($CDCl_3$) δ:1.22 (t, J = 7.4 Hz, 3H), 3.00-3.25 (m, 8H), 5.46 (s, 1H), 7.23 (dd, J = 10.0, 1.6 Hz, 1H), 7.30 (dd, J = 8.2, 1.6 Hz, 1H), 7.64 (dd, J = 8.2, 8.2 Hz, 1H).

Reference Example 16

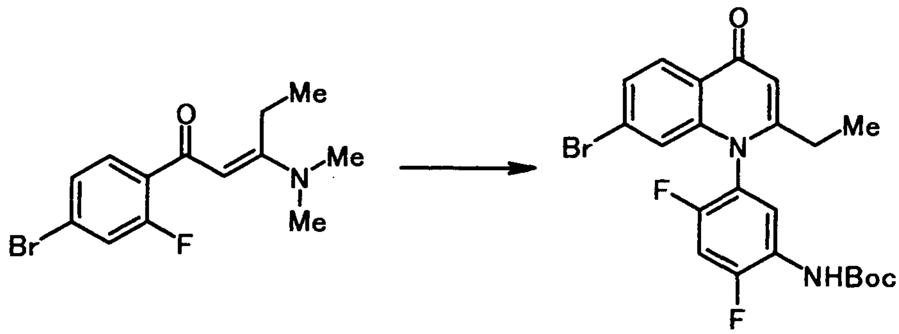

A solution of 0.50 g of (E)-1-(4-bromo-2-fluorophenyl)-3-dimethylamino-2-penten-1-one obtained in a similar manner as the method described in Reference Example 15 and 0.45 g of tert-butyl (5-amino-2, 4-difluorophenyl)carbamate in 2 mL of acetic acid was heated under stirring at 40 to 50°C for 1 hour and 30 minutes. 0.16 g of tert-butyl (5-amino-2, 4-difluorophenyl)carbamate was added, and the mixture was heated under stirring for 1 hour and 30 minutes. The reaction mixture was poured into a saturated aqueous sodium bicarbonate solution, and ethyl acetate was added. The organic layer was separated, and after washing with a saturated aqueous sodium chloride solution, the layer was dried over anhydrous magnesium sulfate, and the solvent was distilled off under reduced pressure. To the resulting residue were added 3.2 mL of DMSO and 0.69 g of potassium carbonate, and the mixture was heated under stirring at 70 to 80°C for 40 minutes. After cooling the reaction mixture, ethyl acetate and water were added. The organic layer was separated, and after washing with water and a saturated aqueous sodium chloride solution successively, the layer was dried over anhydrous magnesium sulfate, and the solvent was distilled off under reduced pressure. The resulting residue was purified by silica gel column chromatography [eluent; hexane : ethyl acetate = 1:1] to obtain 0.61 g of 7 -bromo-1-(5-tert-butoxycarbonylamino-2, 4-difluorophenyl)-2-ethyl-1, 4-dihydro-4-oxoquinoline as a yellow white solid.

$^1$H-NMR (DMSO-$d_6$) δ:1.08 (t, J = 7.3 Hz, 3H), 1.44 (s, 9H), 2.22-2.36 (m, 2H), 6.23 (s, 1H), 6.86 (brs, 1H), 7.57 (dd, J = 8.6, 1.7 Hz, 1H), 7.77 (dd, J = 10.1, 10.1 Hz, 1H), 7.95 (dd, J = 7.9, 7.9 Hz, 1H), 8.10 (d, J = 8.6 Hz, 1H), 9.32-9.41 (br, 1H).

Reference Example 17

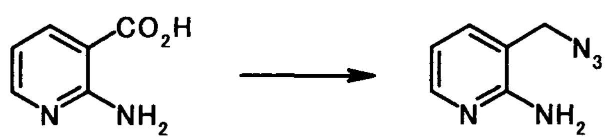

To a suspension of 2.47 g of lithium aluminum hydride in 100 mL of THF, under nitrogen atmosphere at room

temperature, a suspension of 3.00 g of 2-aminonicotinic acid in 50 mL of THF was added dropwise over 20 minutes. After the reaction mixture was heated under reflux for 6 hours, water and ethyl acetate were added. The organic layer was separated, and after washing with water and a saturated aqueous sodium chloride solution successively, the layer was dried over anhydrous magnesium sulfate, and the solvent was distilled off under reduced pressure. To the resulting residue was added 45 mL of toluene, and after cooling to 5°C or lower, 5.11 g of DBU was added. After 5 minutes, 9.24 g of DPPA was added, and the mixture was stirred at room temperature for 3 hours. To the reaction mixture were added ethyl acetate and water. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 4:1] to obtain 2.23 g of 2-amino-3-(azidomethyl)pyridine as a yellow solid.
$^1$H-NMR ($CDCl_3$) δ:4.25 (s, 2H), 4.59-4.95 (br, 2H), 6.69 (dd, J = 7.2, 4.9 Hz, 1H), 7.37 (dd, J = 7.2, 1.6 Hz, 1H), 8.10 (dd, J = 4.9, 1.6 Hz, 1H).

Reference Example 18

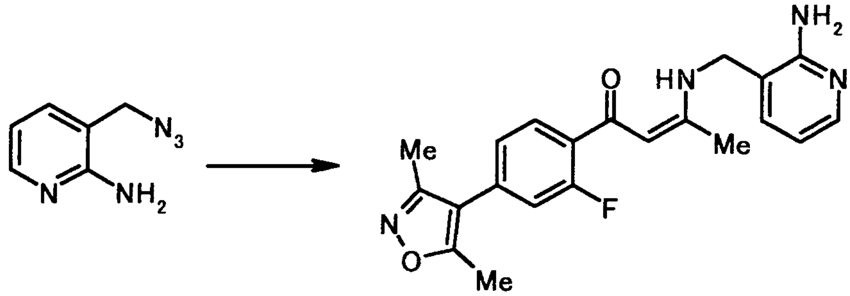

To a solution of 2.23 g of 2-amino-3-(azidomethyl)pyridine in 39 mL of THF was added 4.31 g of triphenylphosphine, and the mixture was stirred at room temperature for 1 hour and 45 minutes. 6 mL of water was added, and the mixture was further stirred at room temperature for 7 hours and 55 minutes. To the reaction mixture were added 1 mol/L hydrochloric acid and ethyl acetate. The aqueous layer was separated, and the organic layer was extracted with 1 mol/L hydrochloric acid. The aqueous layer and the extract were combined, and the solvent was distilled off under reduced pressure to obtain 2.50 g of 2-amino-3-(aminomethyl)pyridine hydrochloride as a pale yellow solid.
(E)-3-(dimethylamino)-1-(4-(3,5-dimethylisoxazol-4-yl)-2-fluorophenyl)-2-buten-1-one was obtained based on the method described in Example 1, from the compound obtained in Reference Example 1.
A suspension of 0.88 g of 2-amino-3-(aminomethyl)pyridine hydrochloride, 2.3 mL of triethylamine and 1.67 g of (E)-3-(dimethylamino)-1-(4-(3, 5-dimethylisoxazol-4-yl)-2-fluorophenyl)-2-buten-1-one in 2.8 mL of ethyl acetate and 2.8 mL of ethanol was heated under stirring at 80°C for 1 hour. 6 mL of ethyl acetate and 4 mL of ethanol were added, and the mixture was heated under stirring at 50 to 75°C for 4 hours and 40 minutes. 0.88 g of 2-amino-3-(aminomethyl) pyridine hydrochloride and 2.3 mL of triethylamine were added, and the mixture was heated under stirring at 70°C for 3 hours and 50 minutes. To the reaction mixture was added diisopropyl ether, and the solid was filtered. To the resulting solid were added ethyl acetate and water, and the organic layer was separated, and the aqueous layer was extracted with ethyl acetate. The organic layer and the extract were combined, and the solvent was distilled off under reduced pressure. The resulting residue was suspended in diisopropyl ether, and the solid was filtered to obtain 1.87 g of (Z)-3-((2-aminopyridin-3-yl)methylamino)-1-(4-(3, 5-dimethylisoxazol-4-yl)-2-fluorophenyl)-2-buten-1-one as a white solid.
$^1$H-NMR ($CDCl_3$) δ:2.12 (s, 3H), 2.30 (s, 3H), 2.44 (s, 3H), 4.39 (d, J = 5.8 Hz, 2H), 4.44-4.56 (br, 2H), 5.81 (d, J = 2.0 Hz, 1H), 6.74 (dd, J = 7.4, 5.0 Hz, 1H), 6.98 (dd, J = 11.7, 1.5 Hz, 1H), 7.10 (dd, J = 8.0, 1.5 Hz, 1H), 7.40-7.46 (m, 1H), 7.88 (dd, J = 8.0, 8.0 Hz, 1H), 8.09 (dd, J = 5.0, 1.6 Hz, 1H), 11.54-11.66 (m, 1H).

Reference Example 19

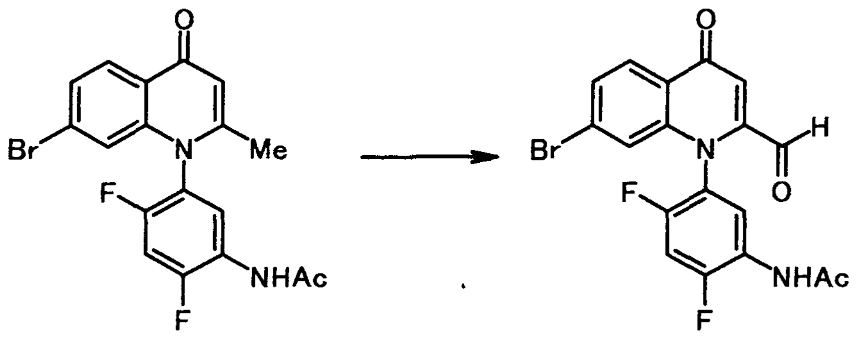

1-(5-acetylamino-2, 4-difluorophenyl)-7-bromo-2-methyl-1, 4-dihydro-4-oxoquinoline was obtained based on the method described in Reference Example 2, from N-(5-amino-2, 4-difluorophenyl)acetamide.

A suspension of 6.20 g of 1-(5-acetylamino-2, 4-difluorophenyl)-7-bromo-2-methyl-1, 4-dihydro-4-oxoquinoline and 2.03 g of selenium dioxide in 150 mL of 1, 4-dioxane was heated under reflux for 7 hours and 35 minutes. After cooling the reaction mixture, insoluble matters were filtered off. The insoluble matters were washed with methanol and ethyl acetate. The filtrate and the wash liquid were combined, and the solvent was distilled off under reduced pressure. To the resulting residue were added ethyl acetate and water. The organic layer was separated, and the aqueous layer was extracted with ethyl acetate. The organic layer and the extract were combined, and after washing with a saturated aqueous sodium chloride solution, the layer was dried over anhydrous magnesium sulfate, and the solvent was distilled off under reduced pressure. The resulting residue was purified by flash column chromatography [eluent; chloroform: methanol = 4:1] to obtain 4.53 g of 1-(5-acetylamino-2, 4-difluorophenyl)-7-promo-1, 4-dihydro-4-oxoquinoline-2-carbaldehyde as a brown solid.
$^1$H-NMR ($CDCl_3$) δ:2.25 (s, 3H), 6.87 (s, 1H), 7.04 (s, 1H), 7.18 (dd, J = 10.5, 8.8 Hz, 1H), 7.44-7.54 (br, 1H), 7.53 (dd, J = 8.6, 1.7 Hz, 1H), 8.29 (d, J = 8.6 Hz, 1H), 8.50 (dd, J = 8.0, 8.0 Hz, 1H), 9.60 (s, 1H).

Reference Example 20

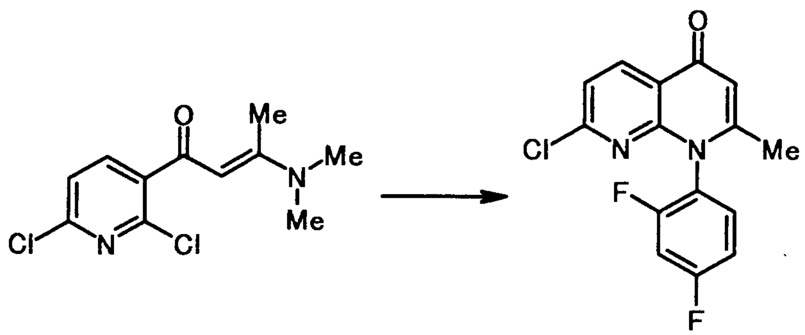

A solution of 1.5 g of (E)-1-(2, 6-dichloropyridin-3-yl)-3-dimethylamino-2-buten-1-one obtained in a similar manner as the method described in Reference Example 3 and 1.12 g of 2, 4-difluoroaniline in 7.5 mL of acetic acid was heated under stirring at 50 to 60°C for 3 hours and 30 minutes. The reaction mixture was added to a saturated aqueous sodium bicarbonate solution, and ethyl acetate was added, and the organic layer was separated. The aqueous layer was extracted twice with ethyl acetate. The organic layer and the extract were combined, and the layer was dried over anhydrous magnesium sulfate, and the solvent was distilled off under reduced pressure. To the solution of the resulting residue in 24 mL of acetonitrile was added 1.20 g of potassium carbonate, and the mixture was heated under reflux for 1 hour. Insoluble matters were filtered off, and the solvent was distilled off under reduced pressure. The resulting residue was suspended in diisopropyl ether, and the solid was filtered to obtain 0.89 g of 7-chloro-1-(2, 4-difluorophenyl)-2-methyl-1, 4-dihydro-4-oxo-l, 8-naphthyridine as a pale red solid.
$^1$H-NMR ($CDCl_3$) δ:2.14 (s, 3H), 6.36 (s, 1H), 7.05-7.14 (m, 2H), 7.25-7.32 (m, 2H), 8.60 (d, J = 8.3 Hz, 1H).

Reference Example 21

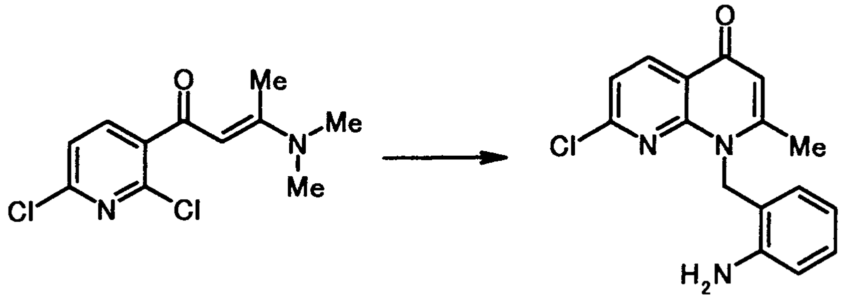

A suspension of 500 mg of (E)-1-(2, 6-dichloropyridin-3-yl)-3-dimethylamino-2-buten-1-one obtained in a similar manner as the method described in Reference Example 3 and 353 mg of 2-(aminomethyl)aniline in 1 mL of ethyl acetate and 1 mL of diisopropyl ether was heated under reflux for 15 minutes. After cooling the reaction mixture, ethyl acetate and diisopropyl ether were added. Insoluble matters were filtered off, and the solvent was distilled off under reduced pressure. To the resulting residue were added 10 mL of acetonitrile and 1.00 g of potassium carbonate, and the mixture was heated under reflux for 30 minutes. The solvent was distilled off under reduced pressure, and 5 mL of DMF was added, and the mixture was heated under stirring at 120°C for 1 hour. To the reaction mixture were added water and ethyl acetate. The organic layer was separated, and after washing with a saturated aqueous sodium chloride solution, the layer was dried over anhydrous magnesium sulfate, and the solvent was distilled off under reduced pressure. The resulting residue was suspended in a mixed solvent of ethyl acetate and diisopropyl ether, and the solid was filtered to obtain 100 mg of 1-(2-aminobenzyl)-7-chloro-2-methyl-1, 4-dihydro-4-oxo-1, 8-naphthyridine as a pale brown solid. $^1$H-NMR (DMSO-$d_6$) δ:2.31 (s, 3H), 5.21 (s, 2H), 5.45 (s, 2H), 6.09 (d, J = 6.8 Hz, 1H), 6.30 (s, 1H), 6.38-6.45 (m, 1H), 6.73 (dd, J=7.9, 1.1 Hz, 1H), 6.95-7.00 (m, 1H), 7.51 (d, J = 8.2 Hz, 1H), 8.54 (d, J = 8.2 Hz, 1H).

Reference Example 22

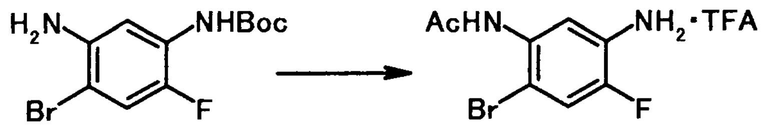

Tert-butyl 5-amino-4-bromo-2-fluorophenylcarbamate was obtained based on the method described in J. Med. Chem., Vol. 46, pages 1905 to 1917, 2003, from 4-bromo-2-fluorobenzoic acid.

To a solution of 16.9 g of tert-butyl 5-amino-4-bromo-2-fluorophenylcarbamate in 170 mL of ethyl acetate, under ice cooling, 15.4 mL of triethylamine and 3.43 mL of acetyl chloride were added, and the mixture was stirred at room temperature for 2 hours and 25 minutes. 0.62 mL of acetyl chloride was added, and the mixture was stirred for 2 hours and 50 minutes. To the reaction mixture were added water and ethyl acetate. The organic layer was separated, and after washing with a saturated aqueous sodium chloride solution, the layer was dried over anhydrous magnesium sulfate, and the solvent was distilled off under reduced pressure. The resulting residue was suspended in hexane, and the solid was filtered to obtain a white solid. To the suspension of the resulting white solid in 70 mL of chloroform, under ice cooling, 10.2 mL of TFA was added, and the mixture was stirred at room temperature for 6 hours and 50 minutes. 30 mL of TFA was further added, and the mixture was stirred for 1 hour. The solvent was distilled off under reduced pressure, and diisopropyl ether was added to the resulting residue, and the solid was filtered to obtain 15.4 g of N-(5-amino-2-bromo-4-fluorophenyl)acetamide TFA salt as a white solid.
$^1$H-NMR (DMSO-$d_6$) δ:2.02 (s, 3H), 5.55-6.70 (br, 2H), 7.00 (d, J = 9.3 Hz, 1H), 7.29 (d, J = 10.7 Hz, 1H), 9.22 (s, 1H)

Reference Example 23

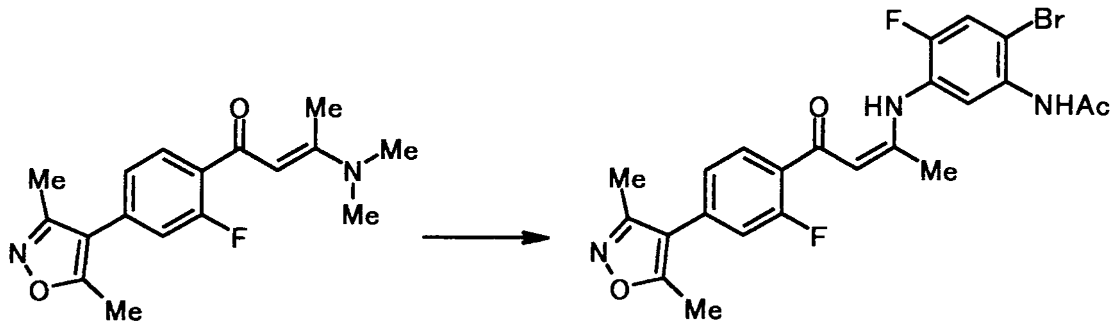

(E)-3-(dimethylamino)-1-(4-(3, 5-dimethylisoxazol-4-yl)-2-fluorophenyl)-2-buten-1-one was obtained based on the method described in Example 1, from the compound obtained in Reference Example 1.

A solution of 1.15 g of (E)-3-(dimethylamino)-1-(4-(3, 5-dimethylisoxazol-4-yl)-2-fluorophenyl)-2-buten-1-one and 1.58 g of N-(5-amino-2-bromo-4-fluorophenyl)acetamide TFA salt in 10 mL of acetic acid and 10 mL of ethanol was heated under stirring at 70 to 80°C for 1 hour and 30 minutes. After cooling the reaction mixture, diisopropyl ether was added, and the solid was filtered to obtain 0.47 g of (Z)-3-(5-acetylamino-4-bromo-2-fluorophenylamino)-1-(4-(3, 5-dimethylisoxazol-4-yl)-2-fluorophenyl)-2-buten-1-one as a yellow solid. $^1$H-NMR ($CDCl_3$) $\delta$:2.22 (s, 3H), 2.26 (s, 3H), 2.31 (s, 3H), 2.45 (s, 3H), 6.00 (d, J = 1.7 Hz, 1H), 7.01 (dd, J = 11.8, 1.7 Hz, 1H), 7.12 (dd, J = 8.0, 1.7 Hz, 1H), 7.40 (d, J = 9.0 Hz, 1H), 7.48-7.58 (br, 1H), 7.94 (dd, J = 8.0, 8.0 Hz, 1H), 8.44 (d, J = 8.0 Hz, 1H), 12.95 (s, 1H).

Reference Example 24

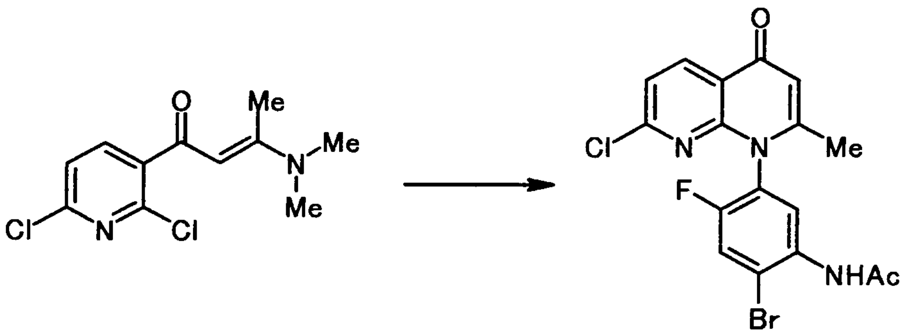

A suspension of 0.72 g of (E)-1-(2, 6-dichloropyridin-3-yl)-3-dimethylamino-2-buten-1-one obtained in a similar manner as the method described in Reference Example 3 and 1.00 g of N-(5-amino-2-bromo-4-fluorophenyl)acetamide TFA salt in 5 mL of acetic acid and 5 mL of ethanol was heated under stirring at 50 to 75°C for 3 hours. The solvent was distilled off under reduced pressure, and the resulting residue was suspended in a mixed solvent of ethyl acetate and diisopropyl ether, and the solid was filtered to obtain a yellow solid. To the solution of the resulting yellow solid in 15 mL of DMF was added 0.86 g of potassium carbonate, and the mixture was heated under stirring at 70°C for 1 hour. To the reaction mixture were added water and ethyl acetate. The organic layer was separated, and the aqueous layer was extracted with ethyl acetate. The organic layer and the extract were combined, and after washing with water and a saturated aqueous sodium chloride solution successively, the layer was dried over anhydrous magnesium sulfate, and the solvent was distilled off under reduced pressure. The resulting residue was purified by silica gel column chromatography [eluent; chloroform:acetone = 1:1], and suspended in a mixed solvent of ethyl acetate and diisopropyl ether, and the solid was filtered to obtain 388 mg of 1-(5-acetylamino-4-bromo-2-fluorophenyl)-7-chloro-2-methyl-1, 4-dihydro-4-oxo-1, 8-naphthyridine as a white solid.
$^1$H-NMR (DMSO-$d_6$) $\delta$:2.08 (s, 3H), 2.12 (s, 3H), 6.38 (s, 1H), 7.52 (d, J = 8.1 Hz, 1H), 7.87 (d, J = 7.8 Hz, 1H), 8.01 (d, J = 9.3 Hz, 1H), 8.51 (d, J = 8.3 Hz, 1H), 9.74 (brs, 1H).

Reference Example 25

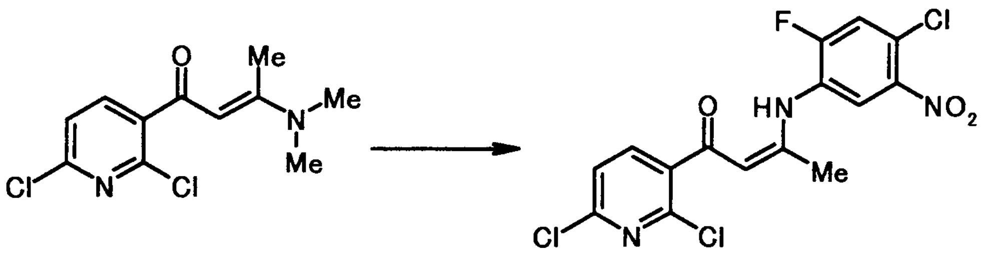

To a solution of 0.50 g of (E)-1-(2, 6-dichloropyridin-3-yi)-3-dimethylamino-2-buten-1-one obtained in a similar manner as the method described in Reference Example 3 in 2 mL of acetic acid was added 0.55 g of 4-chloro-2-fluoro-5-nitroaniline, and the mixture was heated under stirring at 60 to 70°C for 5 hours. After cooling the reaction mixture, ethyl acetate and a saturated aqueous sodium bicarbonate solution were added. The organic layer was separated, and after washing with a saturated aqueous sodium chloride solution, the layer was dried over anhydrous magnesium sulfate, and the solvent was distilled off under reduced pressure. The resulting solid was suspended in a mixed solvent of ethyl acetate and diisopropyl ether, and the solid was filtered to obtain 409 mg of (Z)-3-(4-chloro-2-fluoro-5-nitrophenylamino)-1-(2, 6-dichloropyridin-3-yl)-2-buten-1-one as a yellow solid. $^1$H-NMR (DMSO-$d_6$) δ:2.20 (s, 3H), 5.81 (s, 1H), 7.68 (d, J = 7.8 Hz, 1H), 8.04 (d, J = 10.0 Hz, 1H), 8.07 (d, J = 8.0 Hz, 1H), 8.36 (d, J = 7.8 Hz, 1H), 12.46 (s, 1H).

Reference Example 26

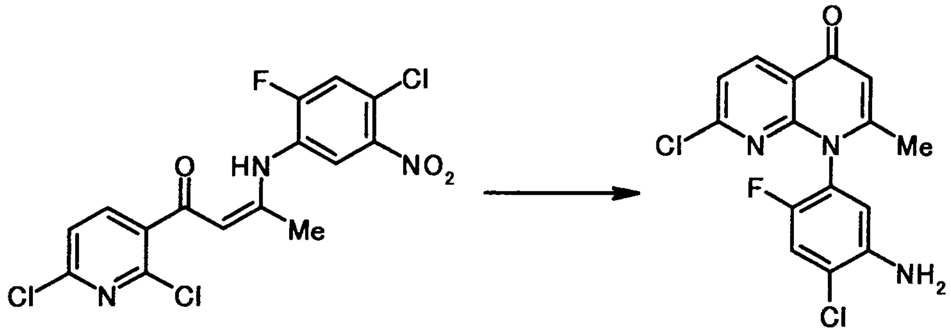

A suspension of 0.40 g of (Z)-3-(4-chloro-2-fluoro-5-nitrophenylamino)-1-(2, 6-dichloropyridin-3-yl)-2-buten-1-one obtained in a similar manner as the method described in Reference Example 25, 0.22 g of iron powder and 0.03 g of ammonium chloride in 6 mL of ethanol and 3 mL of water was heated under reflux for 20 minutes. After cooling the reaction mixture, insoluble matters were filtered off. Ethyl acetate was added to the filtrate, and the organic layer was separated, and after washing with a saturated aqueous sodium chloride solution, the layer was dried over anhydrous magnesium sulfate, and the solvent was distilled off under reduced pressure to obtain a yellow oil.

To the solution of the resulting yellow oil in 4 mL of DMSO was added 0.41 g of potassium carbonate, and the mixture was heated under stirring at 80°C for 1 hour and 30 minutes. After cooling the reaction mixture, ethyl acetate and water were added. The organic layer was separated, and after washing with water and a saturated aqueous sodium chloride solution successively, the layer was dried over anhydrous magnesium sulfate, and the solvent was distilled off under reduced pressure. The resulting residue was purified by silica gel column chromatography [eluent; chloroform: acetone = 4:1] to obtain 0.33 g of 1-(5-amino-4-chloro-2-fluorophenyl)-7-chloro-2-methyl-1, 4-dihydro-4-oxo-1, 8-naphthyridine as a yellow solid.
$^1$H-NMR (DMSO-$d_6$) δ:2.12 (s, 3H), 5.55 (s, 2H), 6.36 (d, J = 0.8 Hz, 1H), 6.87 (d, J = 7.3 Hz, 1H), 7.51 (d, J = 8.2 Hz, 1H), 7.54 (d, J = 9.3 Hz, 1H), 8.50 (d, J = 8.2 Hz, 1H).

Reference Example 27

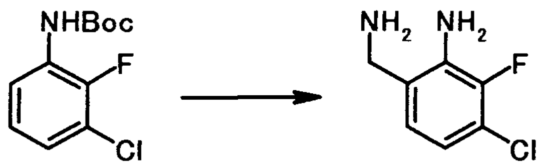

To a solution of 2.38 g of tert-butyl 3-chloro-2-fluorophenylcarbamate in 40 mL of THF was added dropwise 15.4 mL of a solution of n-butyllithium in n-hexane (1.57 mol/L) at -50°C. After increasing the temperature to 15°C over 20 minutes, the mixture was cooled to - 40°C, 5 mL ofDMF was added. After stirring at -54 to -40°C for 10 minutes, the temperature was increased to room temperature, and ethyl acetate and water were added. The organic layer was separated, and after washing with a saturated aqueous sodium chloride solution, the layer was dried over anhydrous magnesium sulfate, and the solvent was distilled off under reduced pressure to obtain yellow oil.

The suspension of the resulting yellow oil, 2.05 g of sodium carbonate and 0.67 g of hydroxylamine hydrochloride in 20 mL of methanol was stirred at room temperature for 20 minutes. The solvent was distilled off under reduced pressure, and ethyl acetate and water were added. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; hexane : ethyl acetate = 5:1] to obtain a yellow oil.

The suspension of the resulting yellow oil and 0.20 g of 5% palladium on carbon in 20 mL of methanol and 3 mL of concentrated hydrochloric acid was stirred under hydrogen atmosphere at room temperature for 5 hours and 30 minutes. Insoluble matters were filtered off, and the solvent was distilled off under reduced pressure, and ethyl acetate and a saturated aqueous sodium bicarbonate solution were added. The organic layer was separated, and after washing with a saturated aqueous sodium chloride solution, the layer was dried over anhydrous sodium sulfate, and the solvent was distilled off under reduced pressure. The resulting residue was purified by silica gel column chromatography [eluent; chloroform: methanol = 2:1] to obtain 0.21 g of 6-(aminomethyl)-3-chloro-2-fluoroaniline as a brown oil.

$^1$H-NMR ($CD_3OD$) $\delta$:3.77 (s, 2H), 6.67 (dd, J = 8.2, 7.0 Hz, 1H), 6.90 (dd, J = 8.2, 1.7 Hz, 1H).

Reference Example 28

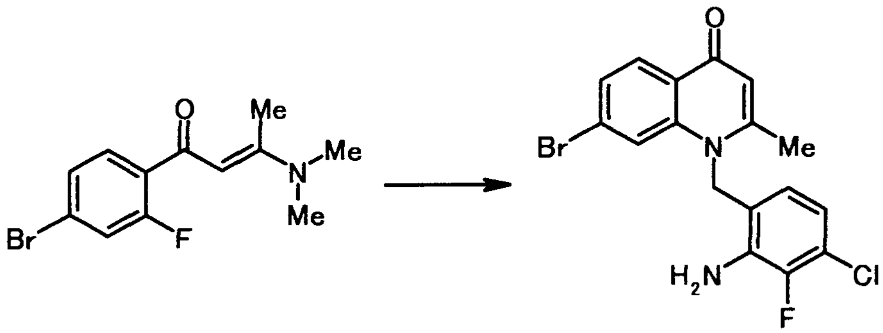

A solution of 0.20 g of (E)-1-(4-bromo-2-fluorophenyl)-3-dimethylamino-2-buten-1-one obtained in a similar manner as the method described in Reference Example 1, 0.21 g of 6-(aminomethyl)-3-chloro-2-fluoroaniline and 0.5 mL of triethylamine in 5 mL of ethyl acetate was heated under stirring at 40 to 50°C for 1 hour and 25 minutes. After cooling the reaction mixture, the solvent was distilled off under reduced pressure. To the resulting residue were added 5 mL of DMF and 0.20 g of potassium carbonate, and the mixture was heated under stirring at 90 to 105°C for 2 hours. After cooling the reaction mixture, ethyl acetate and water were added. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 2:1] to obtain 92 mg of 1-(2-amino-4-chloro-3-fluorobenzyl)-7-bromo-2-methyl-1, 4-dihydro-4-oxoquinoline as a brown solid.

Example 1

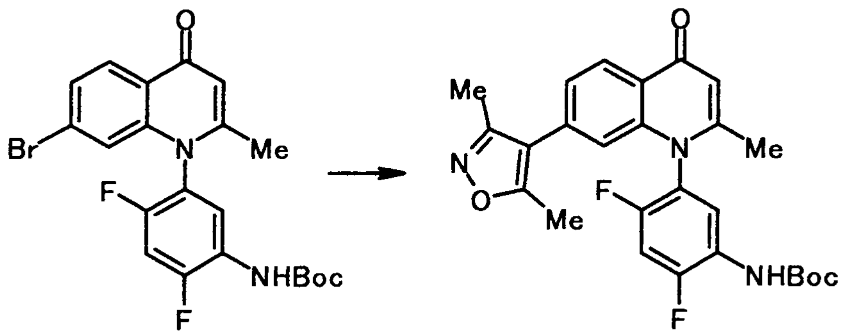

To a suspension of 3.80 g of 7-bromo-1-(5-tert-butoxycarbonylamino-2, 4-difluorophenyl)-2-methyl-1, 4-dihydro-4-oxoquinoline obtained in a similar manner as the method described in Reference Example 2, 2.37 g of 3, 5-dimethyl-4-(4, 4, 5, 5-tetramethyl-1, 3, 2-dioxaborolan-2-yl)isoxazole, 2.06 g of sodium bicarbonate and 0.32 g of triphenylphosphine in 40 mL of toluene, 32 mL of ethanol and 20 mL of water was added under nitrogen atmosphere 92 mg of palladium acetate, and the mixture was heated under reflux for 2 hours. To the reaction mixture was added ethyl acetate, and the organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform: acetone = 5:1] to obtain 3.73 g of 1-(5-tert-butoxycarbonylamino-2, 4-difluorophenyl)-7-(3, 5-dimethylisoxazol-4-yl)-2-methyl-1, 4-dihydro-4-oxoquinoline as a yellow white solid.

$^1$H-NMR (DMSO-$d_6$) δ:1.43 (s, 9H), 2.10 (s, 3H), 2.11 (s, 3H), 2.31 (s, 3H), 6.29 (s, 1H), 6.67 (brs, 1H), 7.43 (dd, J = 8.3, 1.2 Hz, 1H), 7.74 (dd, J = 10.1, 10.1 Hz, 1H), 7.96 (dd, J = 8.1, 8.1 Hz, 1H), 8.25 (d, J = 8.3 Hz, 1H), 9. 3 6 (brs, 1H).

<u>Example 2</u>

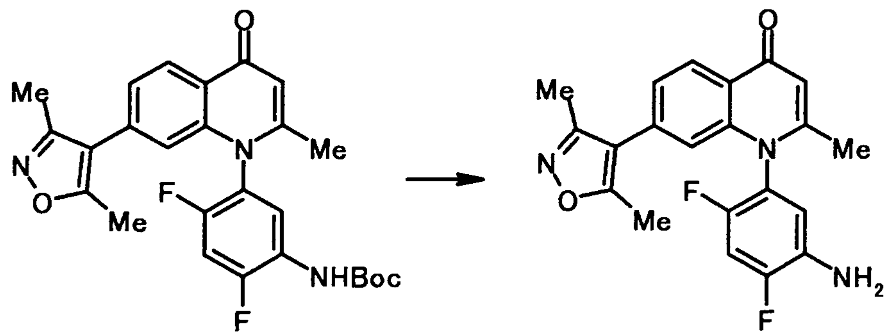

To a solution of 0.50 g of 1-(5-tert-butoxycarbonylamino-2, 4-difluorophenyl)-7-(3, 5-dimethylisoxazol-4-yl)-2-methyl-1, 4-dihydro-4-oxoquinoline in 5 mL of dioxane was added 5 mL of 3 mol/L hydrochloric acid, and the mixture was heated under reflux for 1 hour and 30 minutes. To the reaction mixture were added a saturated aqueous sodium bicarbonate solution and ethyl acetate. The organic layer was separated, and after washing with a saturated aqueous sodium chloride solution, the layer was dried over anhydrous magnesium sulfate, and the solvent was distilled off under reduced pressure. The resulting residue was suspended in a mixed solvent of ethyl acetate and diisopropyl ether, and the solid was filtered to obtain 0.36 g of 1-(5-amino-2, 4-difluorophenyl)-7-(3, 5-dimethylisoxazol-4-yl)-2-methyl-1, 4-dihydro-4-oxoquinoline as a pale yellow solid.

$^1$H-NMR (DMSO-$d_6$) δ:2.12 (s, 3H), 2.13 (s, 3H), 2.33 (s, 3H), 5.44 (brs, 2H), 6.27 (s, 1H), 6.70 (brs, 1H), 6.88 (dd, J = 8.4, 8.4 Hz, 1H), 7.43 (dd, J = 8.2, 1.2 Hz, 1H), 7.49 (dd, J = 10.5, 10.5 Hz, 1H), 8.24 (d, J = 8.2 Hz, 1H).

<u>Example 3</u>

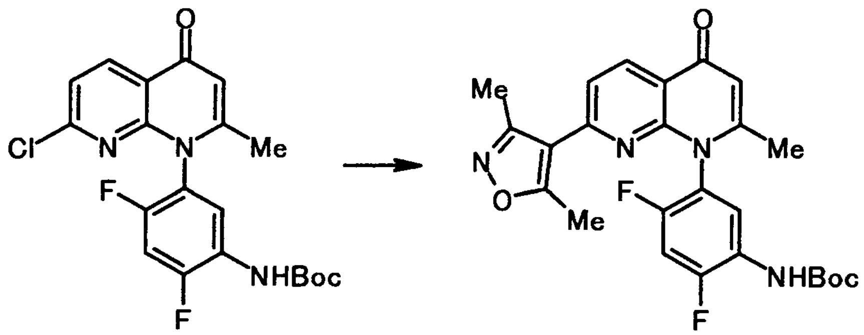

To a solution of 2.62 g of 1-(5-tert-butoxycarbonylamino-2, 4-difluorophenyl)-7-chloro-2-methyl-1, 4-dihydro-4-oxo-1, 8-naphthyridine obtained in a similar manner as the method described in Reference Example 4, 1.05 g of 3, 5-dimethylisoxazole-4-boronic acid, 4.3 mL of triethylamine and 0.25 g of S-Phos in 26 mL of dioxane was added under nitrogen atmosphere 70 mg of palladium acetate, and the mixture was heated under reflux for 2 hours. To the reaction mixture were added water and ethyl acetate. The organic layer was separated, and after washing with hydrochloric acid and a saturated aqueous sodium chloride solution successively, the layer was dried over anhydrous magnesium sulfate, and the solvent was distilled off under reduced pressure. The resulting residue was purified by silica gel column chromatography [eluent; hexane : ethyl acetate = 1:1 → 0:1], and suspended in a mixed solvent of ethyl acetate and diisopropyl ether, and the solid was filtered to obtain 2.70 g of 1-(5-tert-butoxycarbonylamino-2, 4-difluorophenyl)-7-(3, 5-dimethylisoxazol-4-yl)-2-methyl-1, 4-dihydro-4-oxo-1, 8-naphthyridine as a pale yellow solid.
$^1$H-NMR (DMSO-$d_6$) δ:1.42 (s, 9H), 2.08 (s, 3H), 2.15 (s, 3H), 2.38 (s, 3H), 6.34 (s, 1H), 7.65 (d, J = 7.9 Hz, 1H), 7.66 (dd, J = 10.4, 10.4 Hz, 1H), 7.86 (dd, J = 7.8, 7.8 Hz, 1H), 8.56 (d, J = 7.9 Hz, 1H), 9.26 (brs, 1H).

Example 4

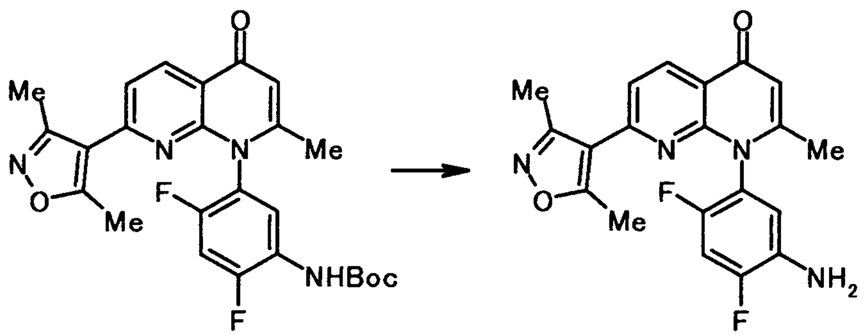

To a solution of 1.46 g of 1-(5-tert-butoxycarbonylamino-2, 4-difluorophenyl)-7-(3, 5-dimethylisoxazol-4-yl)-2-methyl-1, 4-dihydro-4-oxo-1, 8-naphthyridine in 29 mL of dioxane was added 29 mL of 3 mol/L hydrochloric acid, and the mixture was heated under reflux for 1 hour. To the reaction mixture were added an aqueous sodium hydroxide solution and ethyl acetate. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform: acetone = 5:1 → 1:2], and suspended in a mixed solvent of ethyl acetate and diisopropyl ether, and the solid was filtered to obtain 0.99 g of 1-(5-amino-2, 4-difluorophenyl)-7-(3, 5-dimethylisoxazol-4-yl)-2-methyl-1, 4-dihydro-4-oxo-1, 8-naphthyridine as a white solid.
$^1$H-NMR (DMSO-$d_6$) δ:2.11 (s, 3H), 2.15 (s, 3H), 2.41 (s, 3H), 5.30 (brs, 2H), 6.31 (s, 1H), 6.85 (dd, J = 9.0, 7.8 Hz, 1H), 7.39 (dd, J = 11.0, 9.8 Hz, 1H), 7.63 (d, J = 8.2 Hz, 1H), 8.55 (d, J = 8.2 Hz, 1H).

Example 5

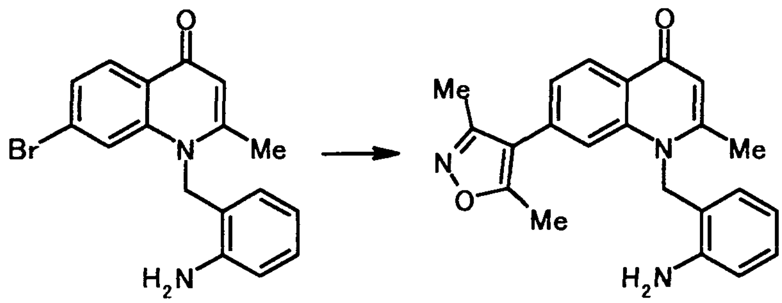

To a suspension of 2.14 g of 1-(2-aminobenzyl)-7-bromo-2-methyl-1, 4-dihydro-4-oxoquinoline obtained in a similar manner as the method described in Reference Example 5, 1.05 g of 3, 5-dimethylisoxazole-4-boronic acid, 4.3 mL of triethylamine and 0.51 g of S-Phos in 21 mL of dioxane was added under nitrogen atmosphere 0.14 g of palladium acetate, and the mixture was heated under reflux for 3 hours and 40 minutes. 0.53 g of 3, 5-dimethylisoxazole-4-boronic acid, 2.2 mL of triethylamine, 0.26 g of S-Phos and 0.07 g of palladium acetate were added, and the mixture was heated under reflux for further 1 hour. To the reaction mixture were added chloroform and methanol, and insoluble matters were filtered off, and the solvent was distilled off under reduced pressure. The resulting residue was purified by silica gel column chromatography [eluent; chloroform:acetone = 4:1 → 1:2]. To the solution of the resulting solid in 21 mL of dioxane was added 21 mL of 6 mol/L hydrochloric acid, and the mixture was heated under reflux for 4 hours. To the reaction mixture were added an aqueous sodium hydroxide solution and ethyl acetate. The organic layer was separated, and after washing with a saturated aqueous sodium chloride solution, the layer was dried over anhydrous magnesium sulfate, and the solvent was distilled off under reduced pressure, and the layer was suspended in ethyl acetate, and the solid was filtered to obtain 1.30 g of 1-(2-aminobenzyl)-7-(3, 5-dimethylisoxazol-4-yl)-2-methyl-1, 4-dihydro-4-oxoquinoline as a white solid. $^1$H-NMR (DMSO-$d_6$) δ:1.99 (s, 3H), 2.16 (s, 3H), 2.46 (s, 3H), 5.24 (brs, 2H), 5.27 (s, 2H), 6.22 (s, 1H), 6.24 (d, J = 7.4 Hz, 1H), 6.45 (dd, J = 7.4, 7.4 Hz, 1H), 6.78 (d, J = 7.6 Hz, 1H), 6.98 (dd, J = 7.4, 7.4 Hz, 1H), 7.16 (s, 1H), 7.36 (d, J = 8.2 Hz, 1H), 8.23 (d, J = 8.2 Hz, 1H).

Example 5-2

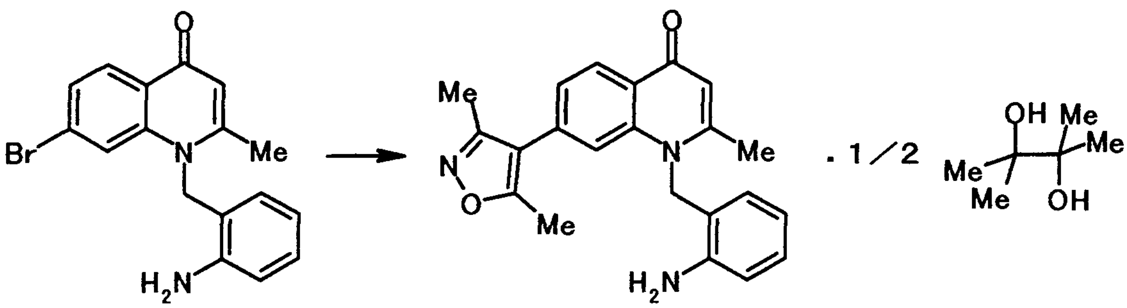

A suspension of 681 mg of 1-(2-aminobenzyl)-7-bromo-2-methyl- 1, 4-dihydro-4-oxoquinoline obtained in a similar manner as the method described in Reference Example 5, 575 mg of 3, 5-dimethyl-4-(4, 4, 5, 5-tetramethyl-1, 3, 2-dioxaborolan-2-yl)isoxazole, 500 mg of sodium bicarbonate, 260 mg of triphenylphosphine and 22 mg of palladium acetate in 6.8 mL of toluene, 5.4 mL of ethanol and 3.4 mL of water was heated under reflux under nitrogen atmosphere for 2 hours and 45 minutes. To the reaction mixture was added ethyl acetate, and the solid was filtered, followed by washing with ethyl acetate and water successively. The filtrate and the wash liquid were combined, and the organic layer was separated, and after washing with a saturated aqueous sodium chloride solution, the layer was dried over anhydrous sodium sulfate, and the solvent was distilled off under reduced pressure. The resulting residue and the filtered solid were suspended in chloroform and methanol. The solid was filtered, and suspended in ethyl acetate, and heated under reflux for 1 hour. The solid was filtered to obtain 512 mg of 1-(2-aminobenzyl)-7-(3, 5-dimethylisoxazol-4-yl)-2-methyl-1, 4-dihydro-4-oxoquinoline· 1/2 pinacol adduct as a white solid.

$^1$H-NMR (DMSO-$d_6$) δ:1.07 (s, 6H), 1.99 (s, 3H), 2.17 (s, 3H), 2.47 (s, 3H), 3.94 (s, 1H), 5.25 (s, 2H), 5.27 (s, 2H), 6.23 (s, 1H), 6.25 (d, J = 7.4 Hz, 1H), 6.45 (dd, J = 7.6, 7.3 Hz, 1H), 6.78 (d, J = 7.6 Hz, 1H), 6.98 (dd, J = 7.4, 7.3 Hz, 1H), 7.16 (brs, 1H), 7.36 (d, J = 8.2, 1.1 Hz, 1H), 8.23 (d, J = 8.2 Hz, 1H).

Example 6

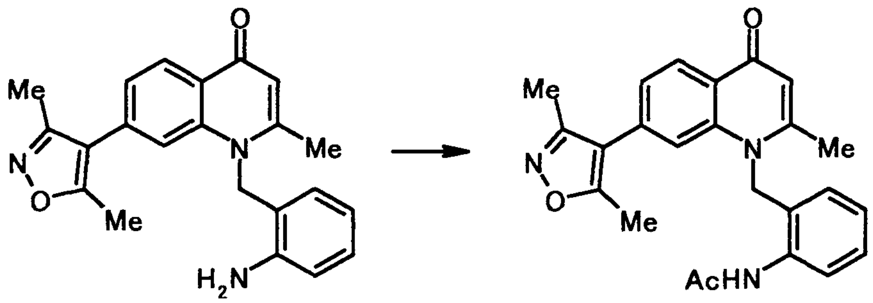

To a solution of 512 mg of 1-(2-aminobenzyl)-7-(3, 5-dimethylisoxazol-4-yl)-2-methyl-1, 4-dihydro-4-oxoquinoline in 14 mL of chloroform and 14 mL of methanol was added 0.20 mL of acetic anhydride under ice cooling, and the mixture was stirred at room temperature for 3 hours and 35 minutes. The solvent was distilled off under reduced pressure, and the resulting residue was suspended in a mixed solvent of ethyl acetate and diisopropyl ether, and the solid was filtered to obtain 532 mg of 1-(2-acetylaminobenzyl)-7-(3, 5-dimethylisoxazol-4-yl)-2-methyl-1, 4-dihydro-4-oxoquinoline as a white solid.
$^1$H-NMR (DMSO-$d_6$) $\delta$:1.95 (s, 3H), 2.11 (s, 6H), 2.43 (s, 3H), 5.40 (s, 2H), 6.24 (s, 1H), 6.45 (d, J = 7.8 Hz, 1H), 7.08-7.14 (m, 1H), 7.25 (brs, 1H), 7.28-7.33 (m, 2H), 7.36 (dd, J = 8.2, 0.6 Hz, 1H), 8.24 (d, J = 8.2 Hz, 1H), 9.87 (s, 1H).

Example 7

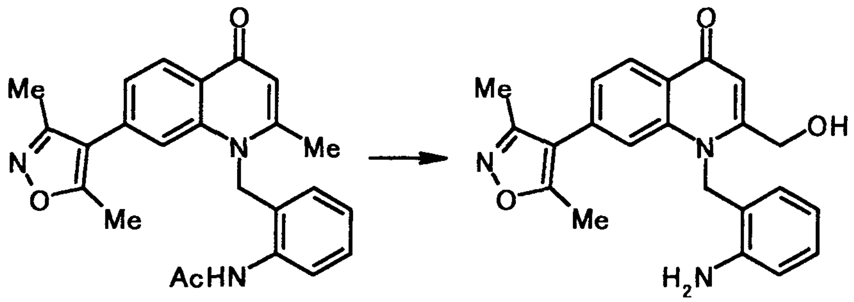

To a solution of 0.83 g of 1-(2-acetylaminobenzyl)-7-(3, 5-dimethylisoxazol-4-yl)-2-methyl-1, 4-dihydro-4-oxoquinoline in 80 mL of dioxane was added 0.32 g of selenium dioxide, and the mixture was heated under reflux for 8 hours. Insoluble matters were filtered off, and the solvent was distilled off under reduced pressure. To the solution of the resulting residue in 30 mL of methanol and 10 mL of chloroform was added 2.19 g of sodium triacetoxyborohydride under ice cooling, and the mixture was stirred at room temperature for 2 hours. 2.19 g of sodium triacetoxyborohydride was added, and the mixture was stirred for further 3 hours. The solvent was distilled off under reduced pressure, and ethyl acetate and water were added. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:methanol = 10:1 $\rightarrow$ 5: 1]. The solution of 0.40 g of the resulting solid in 12 mL of dioxane and 8 mL of 3 mol/L hydrochloric acid was heated under reflux for 3 hours. To the reaction mixture were added an aqueous sodium hydroxide solution and ethyl acetate. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:methanol = 20:1 $\rightarrow$ 10:1], and suspended in a mixed solvent of ethyl acetate and diisopropyl ether, and the solid was filtered to obtain 102 mg of 1-(2-aminobenzyl)-7-(3, 5-dimethylisoxazol-4-yl)-2-hydroxymethyl-1, 4-dihydro-4-oxoquinoline as a pale yellow solid.
$^1$H-NMR (DMSO-$d_6$) $\delta$:1.99 (s, 3H), 2.16 (s, 3H), 4.53 (s, 2H), 5.25 (s, 2H), 5.28 (s, 2H), 6.25 (d, J = 8.1 Hz, 1H), 6.39 (s, 1H), 6.43 (dd, J = 8.0, 7.8 Hz, 1H), 6.76 (d, J = 7.8 Hz, 1H), 6.97 (dd, J = 8.1, 8.0 Hz, 1H), 7.16 (s, 1H), 7.37 (d, J = 8.2 Hz, 1H), 8.24 (d, J = 8.2 Hz, 1H).

Example 8

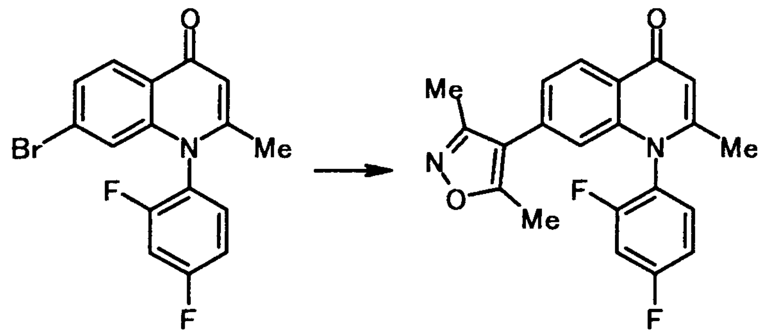

A suspension of 2.77 g of 7-bromo-1-(2, 4-difluorophenyl)-2-methyl-1, 4-dihydro-4-oxoquinoline obtained in a similar manner as the method described in Reference Example 6, 2.29 g of 3, 5-dimethyl-4-(4, 4, 5, 5-tetramethyl-1, 3, 2-dioxaborolan-2-yl)isoxazole, 1.99 g of sodium bicarbonate, 0.31 g of triphenylphosphine and 89 mg of palladium acetate in 28 mL of toluene, 20 mL of ethanol and 14 mL of water was heated under reflux under nitrogen atmosphere for 3 hours. To the reaction mixture was added ethyl acetate, and the organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 10:1 → 7:1] to obtain 2.47 g of 1-(2, 4-difluorophenyl)-7-(3, 5-dimethylisoxazol-4-yl)-2-methyl-1, 4-dihydro-4-oxoquinoline as a yellow white solid. $^1$H-NMR (DMSO-$d_6$) δ:2.08 (s, 3H), 2.11 (s, 3H), 2.31 (s, 3H), 6.30 (d, J = 0.5 Hz, 1H), 6.63 (s, 1H), 7.40-7.45 (m, 1H), 7.44 (dd, J = 8.2, 1.6 Hz, 1H), 7.72 (ddd, J = 10.2, 8.9, 2.8 Hz, 1H), 7.84 (ddd, J = 9.0, 8.9, 5.9 Hz, 1H), 8.25 (d, J = 8.2 Hz, 1H).

Example 9

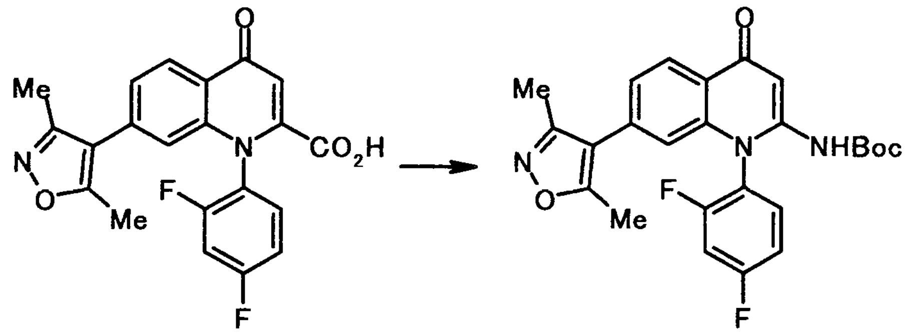

To a solution of 1.54 g of 1-(2, 4-difluorophenyl)-7-(3, 5-dimethylisoxazol-4-yl)-1, 4-dihydro-4-oxoquinoline-2-carboxylic acid obtained in a similar manner as the method described in Reference Example 7 in 30 mL of 2-methyl-2-propanol were added under nitrogen atmosphere 1.67 mL of DPPA and 1.08 mL of triethylamine, and the mixture was heated under reflux for 5 hours. To the reaction mixture were added an aqueous sodium hydroxide solution and ethyl acetate. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; hexane : ethyl acetate = 1:1 → 1:2], and suspended in a mixed solvent of ethyl acetate and diisopropyl ether, and the solid was filtered to obtain 1.02 g of 2-tert-butoxycarbonylamino-1-(2, 4-difluorophenyl)-7-(3, 5-dimethylisoxazol-4-yl)-1, 4-dihydro-4-oxoquinoline as a yellow solid.

$^1$H-NMR (DMSO-$d_6$) δ:1.29 (s, 9H), 2.12 (s, 3H), 2.33 (s, 3H), 6.20 (s, 1H), 6.72 (brs, 1H), 7.33-7.39 (m, 1H), 7.47 (dd, J = 8.3, 1.5 Hz, 1H), 7.60-7.66 (m, 1H), 7.69 (ddd, J = 8.8, 8.8, 6.0 Hz, 1H), 8.25 (d, J = 8.3 Hz, 1H), 9.51 (s, 1H).

Example 10

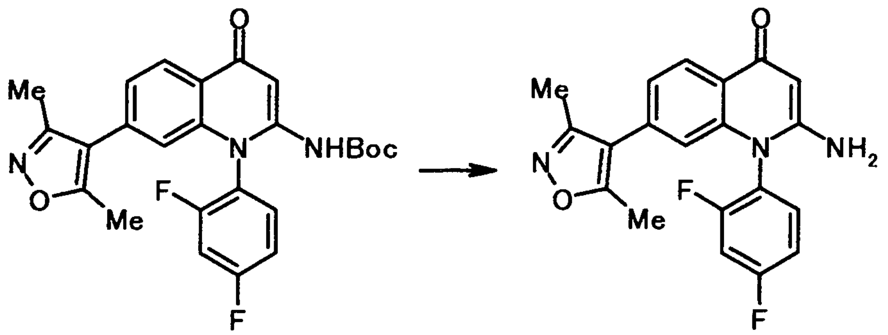

To a solution of 1.02 g of 2-tert-butoxycarbonylamino-1-(2, 4-difluorophenyl)-7-(3, 5-dimethylisoxazol-4-yl)-1, 4-dihydro-4-oxoquinoline in 5 mL of chloroform was added 5 mL of TFA, and the mixture was allowed to stand still for 13 hours and 30 minutes. To the reaction mixture were added an aqueous sodium hydroxide solution and ethyl acetate. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform : methanol : ammonia water = 100:5:1], and suspended in a mixed solvent of ethyl acetate and diisopropyl ether, and the solid was filtered to obtain 0.63 g of 2-amino-1-(2, 4-difluorophenyl)-7-(3, 5-dimethylisoxazol-4-yl)-1, 4-dihydro-4-oxoquinoline as a yellow white solid.
$^1$H-NMR (DMSO-$d_6$) δ:2.08 (s, 3H), 2.28 (s, 3H), 5.50 (s, 1H), 6.36 (s, 1H), 6.44 (brs, 2H), 7.27 (d, J = 8.1 Hz, 1H), 7.36-7.43 (m, 1H), 7.65-7.78 (m, 2H), 8.13 (d, J = 8.1 Hz, 1H).

Example 11

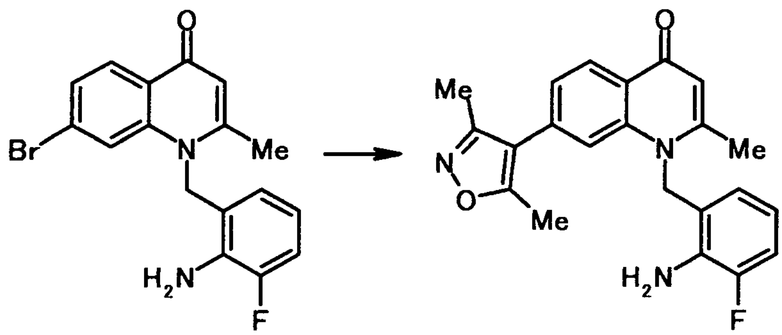

A suspension of 1.00 g of 1-(2-amino-3-fluorobenzyl)-7-bromo-2-methyl-1, 4-dihydro-4-oxoquinoline obtained in a similar manner as the method described in Reference Example 8, 0.80 g of 3, 5-dimethyl-4-(4, 4, 5, 5-tetramethyl-1, 3, 2-dioxaborolan-2-yl)isoxazole, 0.70 g of sodium bicarbonate, 0.29 g of triphenylphosphine and 62 mg of palladium acetate in 10 mL of toluene, 8 mL of ethanol and 5 mL of water was heated under reflux under nitrogen atmosphere for 1 hour. To the reaction mixture were added ethyl acetate and water, and the organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 2:1 → 3:2] to obtain 0.85 g of 1-(2-amino-3-fluorobenzyl)-7-(3, 5-dimethylisoxazol-4-yl)-2-methyl-1, 4-dihydro-4-oxoquinoline as a brown solid.
$^1$H-NMR (DMSO-$d_6$) δ:2.01 (s, 3H), 2.18 (s, 3H), 2.46 (s, 3H), 5.30 (s, 2H), 5.35 (s, 2H), 6.11 (d, J = 7.8 Hz, 1H), 6.23 (s, 1H), 6.46 (ddd, J = 7.9, 7.9, 5.2 Hz, 1H), 6.93-7.00 (m, 1H), 7.18 (d, J = 1.1 Hz, 1H), 7.37 (dd, J = 8.3, 1.1 Hz, 1H), 8.23 (d, J = 8.3 Hz, 1H).

Example 12

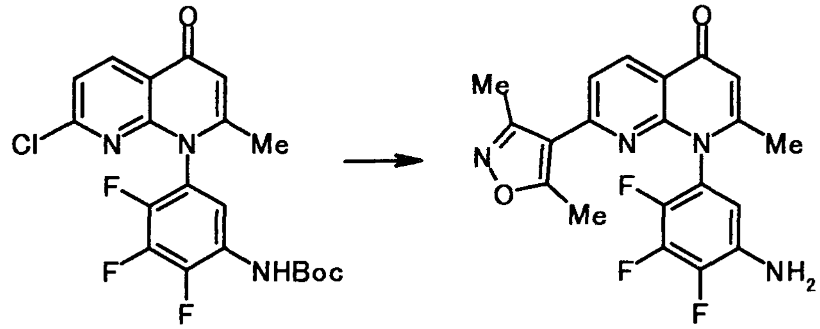

A suspension of 10.1 g of 1-(5-tert-butoxycarbonylamino-2, 3, 4-trifluorophenyl)-7-chloro-2-methyl-1, 4-dihydro-4-oxo-1, 8-naphthyridine obtained in a similar manner as the method described in Reference Example 9, 3.88 g of 3, 5-dimethylisoxazole-4-boronic acid, 14.6 g of potassium phosphate, 3.01 g of triphenylphosphine and 258 mg of palladium acetate in 100 mL of toluene, 80 mL of ethanol and 50 mL of water was heated under reflux under nitrogen atmosphere for 1 hour and 15 minutes. After cooling the reaction mixture, ethyl acetate and water were added. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 6:1] to obtain a brown oil.

To the resulting brown oil were added 100 mL of 1, 4-dioxane and 100 mL of 6 mol/L hydrochloric acid, and the mixture was heated under stirring at 85 to 95°C for 1 hour. After cooling the reaction mixture, a 20%(w/w) aqueous sodium hydroxide solution and ethyl acetate were added. The organic layer was separated, and after washing with a saturated aqueous sodium chloride solution, the layer was dried over anhydrous sodium sulfate, and the solvent was distilled off under reduced pressure. The resulting residue was purified by silica gel column chromatography [eluent; chloroform:acetone = 1:1], and suspended in a mixed solvent of ethyl acetate and diisopropyl ether, and the solid was filtered to obtain 8.50 g of 1-(5-amino-2, 3, 4-trifluorophenyl)-7-(3, 5-dimethylisoxazol-4-yl)-2-methyl-1, 4-dihydro-4-oxo-1, 8-naphthyridine as a pale brown solid.

$^1$H-NMR (DMSO-$d_6$) δ:2.13 (s, 3H), 2.19 (s, 3H), 2.43 (s, 3H), 5.70 (brs, 2H), 6.33 (s, 1H), 6.69-6.76 (m, 1H), 7.65 (d, J = 8.3 Hz, 1H), 8.55 (d, J = 8.3 Hz, 1H).

Example 13

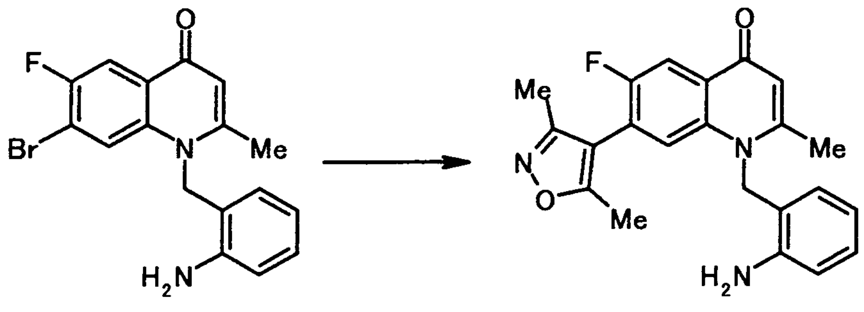

A suspension of 0.20 g of 1-(2-aminobenzyl)-7-bromo-6-fluoro-2-methyl-1, 4-dihydro-4-oxoquinoline obtained in a similar manner as the method described in Reference Example 11, 0.10 g of 3, 5-dimethylisoxazole-4-boronic acid, 0.14 g of sodium bicarbonate, 73 mg of triphenylphosphine and 6 mg of palladium acetate in 2 mL of toluene, 1.6 mL of ethanol and 1 mL of water was heated under reflux under nitrogen atmosphere for 1 hour and 45 minutes. After cooling the reaction mixture, ethyl acetate and water were added. The organic layer was separated, and after washing with a saturated aqueous sodium chloride solution, the layer was dried over anhydrous sodium sulfate, and the solvent was distilled off under reduced pressure. The resulting residue was purified by silica gel column chromatography [eluent; chloroform:ethanol = 9:1] to obtain 0.09 g of 1-(2-aminobenzyl)-7-(3, 5-dimethylisoxazol-4-yl)-6-fluoro-2-methyl-1, 4-dihydro-4-oxoquinoline as a pale yellow solid.
$^1$H-NMR (DMSO-$d_6$) δ:1.91 (s, 3H), 2.10 (s, 3H), 2.47 (s, 3H), 5.23 (s, 2H), 5.28 (s, 2H), 6.20-6.26 (m, 2H), 6.43-6.49 (m, 1H), 6.78 (d, J = 8.0 Hz, 1H), 6.95-7.02 (m, 1H), 7.28 (d, J = 5.9 Hz, 1H), 7.93 (d, J = 10.0 Hz, 1H).

Example 14

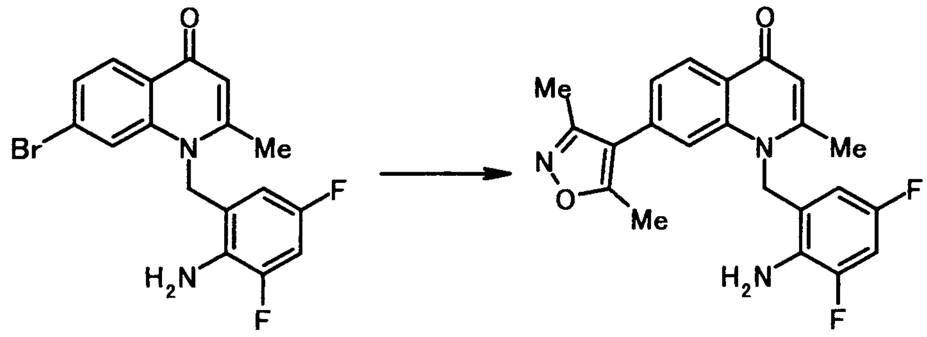

A suspension of 253 mg of 1-(2-amino-3, 5-difluorobenzyl)-7-bromo-2-methyl-1, 4-dihydro-4-oxoquinoline obtained in a similar manner as the method described in Reference Example 14, 113 mg of 3, 5-dimethylisoxazole-4-boronic acid, 168 mg of sodium bicarbonate, 87 mg of triphenylphosphine and 7 mg of palladium acetate in 2.5 mL of toluene, 2 mL of ethanol and 1.25 mL of water was heated under reflux under nitrogen atmosphere for 2 hours. After cooling the reaction mixture, ethyl acetate and water were added. The organic layer was separated, and after washing with a saturated aqueous sodium chloride solution, the layer was dried over anhydrous sodium sulfate, and the solvent was distilled off under reduced pressure. The resulting residue was purified by silica gel column chromatography [eluent; chloroform:acetone = 2:1], and suspended in diisopropyl ether, and the solid was filtered to obtain 250 mg of 1-(2-amino-3, 5-difluorobenzyl)-7-(3, 5-dimethylisoxazol-4-yl)-2-methyl-1, 4-dihydro-4-oxoquinoline as a white solid.
$^1$H-NMR (DMSO-$d_6$) δ:2.03 (s, 3H), 2.21 (s, 3H), 2.46 (s, 3H), 5.20 (brs, 2H), 5.37 (s, 2H), 5.92 (d, J = 9. 3 Hz, 1H), 6.23 (s, 1H), 7. 02-7.11 (m, 1H), 7.18 (d, J = 1.1 Hz, 1H), 7.40 (dd, J = 8.2, 1.1 Hz, 1H), 8.24 (d, J = 8.2 Hz, 1H).

Example 15

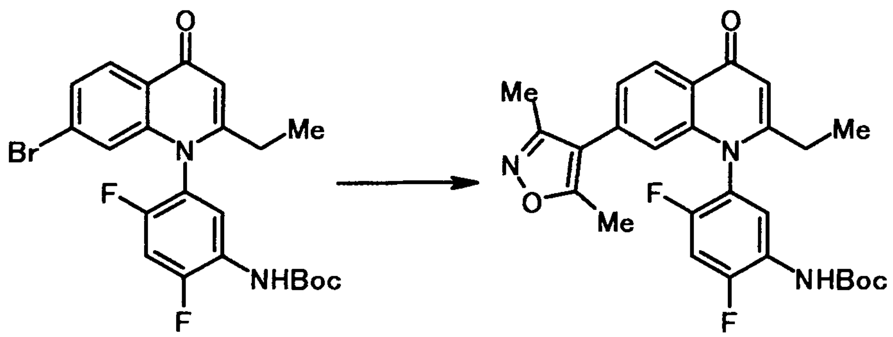

A suspension of 300 mg of 7-bromo-1-(5-tert-butoxycarbonylamino-2, 4-difluorophenyl)-2-ethyl-1, 4-dihydro-4-oxoquinoline obtained in a similar manner as the method described in Reference Example 16, 168 mg of 3, 5-dimethyl-4-(4, 4, 5, 5-tetramethyl-1, 3, 2-dioxaborolan-2-yl)isoxazole, 158 mg of sodium bicarbonate, 82 mg of triphenylphosphine and 7 mg of palladium acetate in 3 mL of toluene, 2.4 mL of ethanol and 1.5 mL of water was heated under reflux under nitrogen atmosphere for 1 hour and 20 minutes. 42 mg of 3, 5-dimethyl-4-(4, 4, 5, 5-tetramethyl-1, 3, 2-dioxaborolan-2-yl)isoxazole was added, and the mixture was heated under reflux for further 40 minutes. After cooling the reaction mixture, chloroform and water were added. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:methanol = 20:1] to obtain 270 mg of 1-(5-tert-butoxycarbonylamino-2, 4-difluorophenyl)-7-(3, 5-dimethylisoxazol-4-yl)-2-ethyl-1,

4-dihydro-4-oxoquinoline as a white solid.
$^1$H-NMR ($CDCl_3$) δ:1.21 (t, J = 7.4 Hz, 3H), 1.49 (s, 9H), 2.16 (s, 3H), 2.32 (s, 3H), 2.30-2.45 (m, 2H), 6.39 (s, 1H), 6.52 (s, 1H), 6.81 (brs, 1H), 7.14-7.21 (m, 1H), 7.24 (dd, J = 8.3, 1.5 Hz, 1H), 8.22-8.33 (m, 1H), 8.48 (d, J = 8.3 Hz, 1H).

Example 16

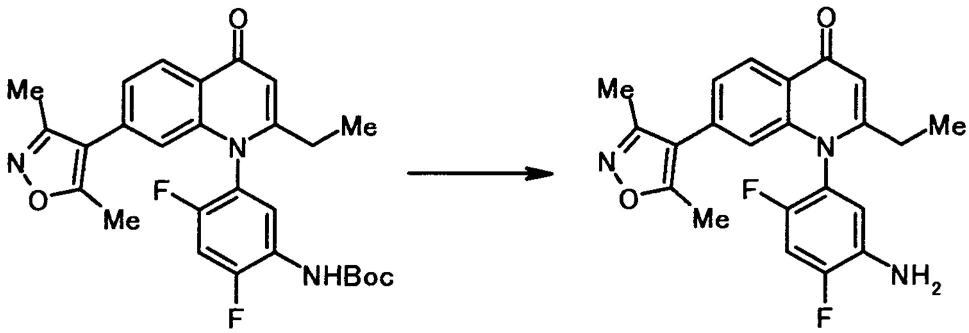

To a solution of 270 mg of 1-(5-tert-butoxycarbonylamino-2, 4-difluorophenyl)-7-(3, 5-dimethylisoxazol-4-yl)-2-ethyl-1, 4-dihydro-4-oxoquinoline in 3 mL of 1, 4-dioxane and 1.5 mL of water was added 1.5 mL of 6 mol/L hydrochloric acid, and the mixture was heated under stirring at 60°C for 1 hour. After cooling the reaction mixture, chloroform, water and a saturated aqueous sodium bicarbonate solution were added. The organic layer was separated, and the aqueous layer was extracted with chloroform and ethyl acetate. The organic layer and the extract were combined, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:methanol = 20:1] to obtain 199 mg of 1-(5-amino-2, 4-difluorophenyl)-7-(3, 5-dimethylisoxazol-4-yl)-2-ethyl-1, 4-dihydro-4-oxoquinoline as a white solid. $^1$H-NMR (DMSO-$d_6$) δ:1.12 (t, J = 7.4 Hz, 3H), 2.12 (s, 3H), 2.33 (s, 3H), 2.34-2.42 (m, 2H), 5.45 (brs, 2H), 6.22 (s, 1H), 6.67 (s, 1H), 6.89 (dd, J = 8.5, 8.5 Hz, 1H), 7.43 (dd, J = 8.3, 1.5 Hz, 1H), 7.50 (dd, J = 10.5, 10.5 Hz, 1H), 8.24 (d, J = 8.3 Hz, 1H).

Example 17

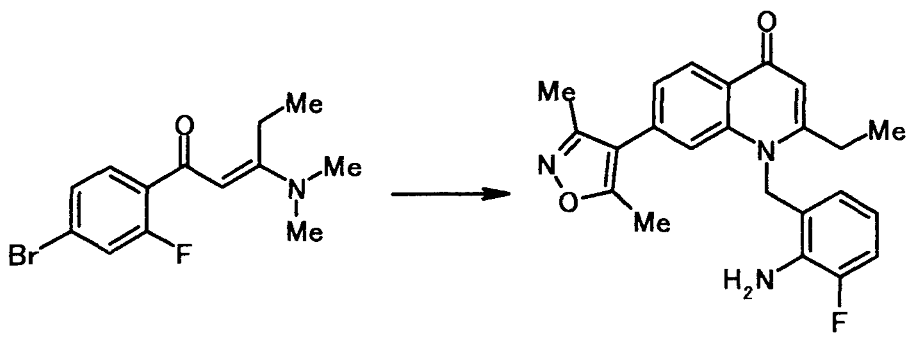

A suspension of 0.58 g of (E)-1-(4-bromo-2-fluorophenyl)-3-dimethylamino-2-penten-1-one obtained in a similar manner as the method described in Reference Example 15, 0.60 g of 3, 5-dimethyl-4-(4, 4, 5, 5-tetramethyl-1, 3, 2-dioxaborolan-2-yl)isoxazole, 0.49 g of sodium bicarbonate, 0.25 g of triphenylphosphine and 43 mg of palladium acetate in 5.8 mL of toluene, 4.6 mL of ethanol and 2.9 mL of water was heated under reflux under nitrogen atmosphere for 3 hours and 30 minutes. To the reaction mixture was added ethyl acetate. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 20:1] to obtain 0.15 g of a yellow white solid.

A solution of 75 mg of the resulting yellow white solid and 70 mg of 6-(aminomethyl)-2-fluoroaniline in 1 mL of ethyl acetate and 2 mL of ethanol was heated under reflux for 12 hours and 20 minutes. The solvent was distilled off

under reduced pressure, and 0.5 mL of ethyl acetate and 0.5 mL oftriethylamine were added, and the mixture was heated under stirring at 60°C for 4 hours. The solvent was distilled off under reduced pressure, and the resulting residue was purified by silica gel column chromatography [eluent; hexane : ethyl acetate = 3:1] to obtain 80 mg of a brown oil.

A suspension of 80 mg of the resulting brown oil and 100 mg of potassium carbonate in 1 mL of DMSO was heated under stirring at 100°C for 1 hour. To the reaction mixture were added ethyl acetate and water. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform: acetone = 1:1] to obtain 10 mg of 1-(2-amino-3-fluorobenzyl)-7-(3, 5 -dimethylisoxazol-4-yl)-2- ethyl-1, 4-dihydro-4-oxoquinoline as a pale brown solid.

$^1$H-NMR (DMSO-$d_6$) δ:1.26 (t, J = 7.3 Hz, 3H), 1.99 (s, 3H), 2.16 (s, 3H), 2.75 (q, J = 7.3 Hz, 2H), 5.32 (s, 2H), 5.35 (s, 2H), 6.09 (d, J = 7.8 Hz, 1H), 6.20 (s, 1H), 6.41-6.49 (m, 1H), 6.92-7.00 (m, 1H), 7.15 (s, 1H), 7.37 (dd, J = 8.3, 1.2 Hz, 1H),8.23(d,J=8.3Hz, 1H).

Example 18

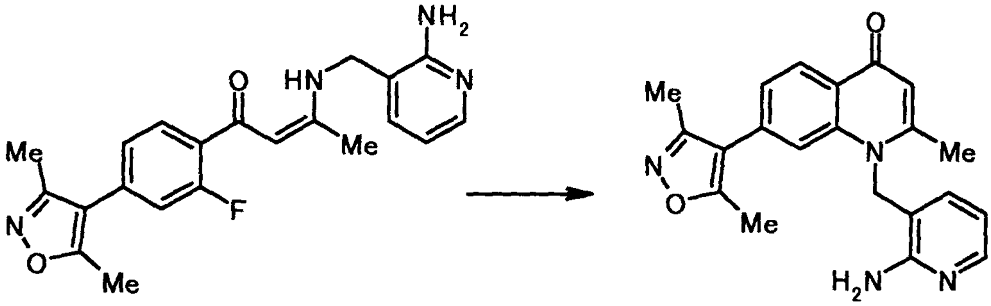

A suspension of 1.37 g of (Z)-3-((2-aminopyridin-3-yl)methylamino)-1-(4-(3, 5-dimethylisoxaaol-4-yl)-2-fluorophenyl)-2-buten-1-one obtained in a similar manner as the method described in Reference Example 18 and 0.75 g of potassium carbonate in 10 mL of DMSO was heated under stirring at 80°C for 10 hours and 10 minutes. To the reaction mixture were added ethyl acetate and water. The organic layer was separated, and the aqueous layer was extracted with ethyl acetate 6 times. The organic layer and the extract were combined, and after washing with a saturated aqueous sodium chloride solution, the layer was dried over anhydrous magnesium sulfate, and the solvent was distilled off under reduced pressure. The resulting residue was purified by silica gel column chromatography [eluent; acetone → methanol], and suspended in a mixed solvent of ethyl acetate and diisopropyl ether, and the solid was filtered to obtain 0.63 g of 1-((2-aminopyridin-3-yl)methyl)-7-(3, 5-dimethylisoxazol-4-yl)-2-methyl-1, 4-dihydro-4-oxoquinoline as a pale brown solid.

$^1$H-NMR (DMSO-$d_6$) δ:2.01 (s, 3H), 2.19 (s, 3H), 2.44 (s, 3H), 5.26 (brs, 2H), 6.09 (brs, 2H), 6.23 (s, 1H), 6.45 (dd, 1=7.4,5.1 Hz, 1H), 6.52-6.58 (m, 1H), 7.17 (s, 1H), 7.38 (d, J = 8.3 Hz, 1H), 7.89 (d, J = 5.1 Hz, 1H), 8.24 (d, J = 8.3 Hz, 1H).

Example 19

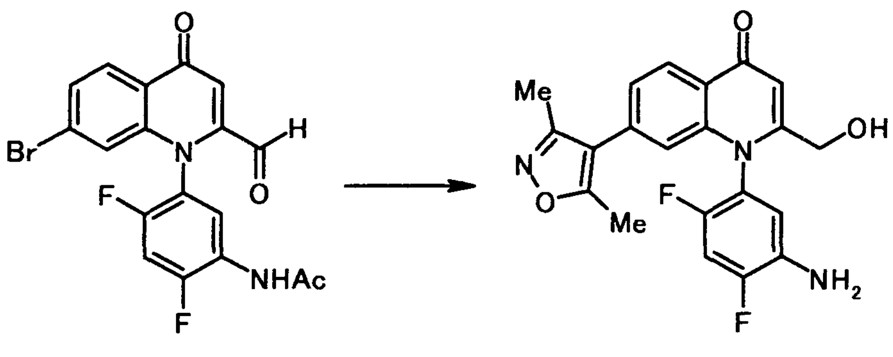

To a suspension of 500 mg of 1-(5-acetylamino-2, 4-difluorophenyl)-7-bromo-1, 4-dihydro-4-oxoquinoline-2-carbaldehyde obtained in a similar manner as the method described in Reference Example 19 in 20 mL of methanol was added 1.26 g of sodium triacetoxyborohydride, and the mixture was stirred at room temperature for 1 hour and 10 minutes. 1.26 g of sodium triacetoxyborohydride was added, and the mixture was stirred for 1 hour and 5 minutes. 1.26 g of sodium triacetoxyborohydride was added, and the mixture was stirred for 1 hour and 45 minutes. The solvent was distilled off under reduced pressure, and to the resulting residue were added ethyl acetate, water and a saturated aqueous sodium chloride solution. Insoluble matters were filtered off, and the organic layer of the filtrate was separated. The filter residue was dissolved in methanol, and the solution was combined with the organic layer, and dried over anhydrous magnesium sulfate, and the solvent was distilled off under reduced pressure. The resulting residue was suspended in a mixed solvent of ethyl acetate and hexane, and the solid was filtered to obtain 683 mg of a white solid.

A suspension of 600 mg of the resulting white solid, 474 mg of 3, 5-dimethyl-4-(4, 4, 5, 5-tetramethyl-1, 3, 2-dioxaboran-2-yl)isoxazole, 357 mg of sodium bicarbonate, 116 mg of S-Phos and 32 mg of palladium acetate in 12 mL of toluene, 9.6 mL of ethanol and 6 mL of water was heated under reflux under nitrogen atmosphere for 5 hours. After cooling the reaction mixture, ethyl acetate and water were added. The organic layer was separated, and after washing with a saturated aqueous sodium chloride solution, the layer was dried over anhydrous magnesium sulfate, and the solvent was distilled off under reduced pressure. The resulting residue was purified by flash column chromatography [eluent; chloroform: methanol = 4:1] to obtain a yellow oil.

To the resulting yellow oil were added 10.3 mL of 1, 4-dioxane and 5.2 mL of 1 mol/L hydrochloric acid, and the mixture was heated under reflux for 3 hours and 15 minutes. After cooling the reaction mixture, ethyl acetate and a 1 mol/L aqueous sodium hydroxide solution were added. The organic layer was separated, and the aqueous layer was extracted with ethyl acetate. The organic layer and the extract were combined, and after washing with a saturated aqueous sodium chloride solution, the layer was dried over anhydrous magnesium sulfate, and the solvent was distilled off under reduced pressure. The resulting solid was suspended in a mixed solvent of diisopropyl ether and 2-propanol, and the solid was filtered to obtain 160 mg of 1-(5-amino-2, 4-difluorophenyl)-7-(3, 5-dimethylisoxazol-4-yl)-2-(hydroxymethyl)-1, 4-dihydro-4-oxoquinoline as a pale green solid.

$^1$H-NMR (DMSO-$d_6$) $\delta$:2.13 (s, 3H), 2.33 (s, 3H), 4.01-4.24 (m, 2H), 5.44 (s, 2H), 5.73 (t, J = 5.9 Hz, 1H), 6.43 (s, 1H), 6.69 (s, 1H), 6.89 (dd, J = 8.4, 8.4 Hz, 1H), 7.40-7.52 (m, 2H), 8.25 (d, J = 8.3 Hz, 1H).

Example 20

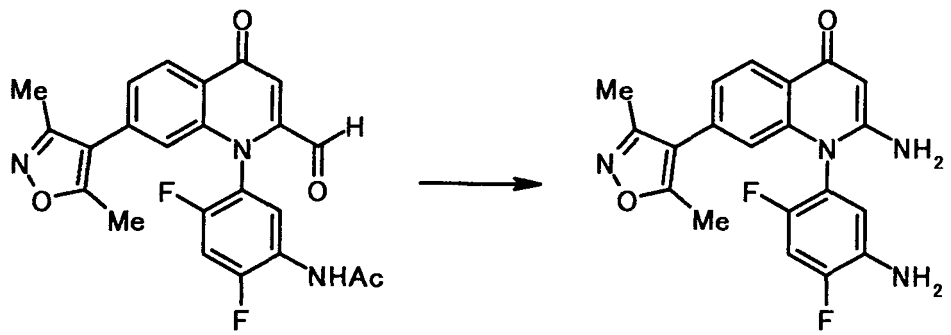

1-(5-acetylamino-2, 4-difluorophenyl)-7-(3, 5-dimethylisoxazol-4-yl)-1, 4-dihydro-4-oxoquinoline-2-carbaldehyde was obtained based on the method described in Reference Example 19, from 1-(5-acetylamino-2, 4-difluorophenyl)-7-(3, 5-dimethylisoxazol-4-yl)-2-methyl-1, 4-dihydro-4-oxoquinoline. 1-(5-acetylamino-2, 4-difluorophenyl)-7-(3, 5-dimethylisoxazol-4-yl)-2-methyl-1, 4-dihydro-4-oxoquinoline was obtained based on the method described in Example 6, from the compound obtained in Example 2.

To a solution of 0.60 g of 1-(5-acetylamino-2, 4-difluorophenyl)-7-(3, 5-dimethylisoxazol-4-yl)-1, 4-dihydro-4-oxoquinoline-2-carbaldehyde and 2.9 mL of 2-methyl-2-butene in 10 mL of 2-methyl-2-propanol and 10 mL of THF was added a solution of 3.42 g of sodium dihydrogen phosphate dihydrate and 0.32 g of sodium chlorite in 8.4 mL of water, and the mixture was stirred at room temperature for 2 hours and 50 minutes. To the reaction mixture were added a 0.5 mol/L aqueous sodium hydroxide solution and ethyl acetate. The aqueous layer was separated, and the organic layer was extracted with a 1 mol/L aqueous sodium hydroxide solution 3 times. The aqueous layer and the extract were combined, and the mixture was adjusted to pH 3.5 with 6 mol/L hydrochloric acid. The mixture was extracted with ethyl acetate twice, and after the organic layer was washed with a saturated aqueous sodium chloride solution, the layer was dried over anhydrous magnesium sulfate, and the solvent was distilled off under reduced pressure. The resulting solid was suspended in a mixed solvent of ethyl acetate and diisopropyl ether, and the solid was filtered to obtain 0.25 g of

a yellow white solid.

To a suspension of 180 mg of the resulting yellow white solid in 4 mL of 2-methyl-2-propanol were added 0.11 mL of triethylamine and 0.17 mL of DPPA, and the mixture was heated under reflux for 3 hours and 20 minutes. To the reaction mixture were added ethyl acetate and a saturated aqueous sodium bicarbonate solution. The organic layer was separated, and after washing with 1 mol/L hydrochloric acid and a saturated aqueous sodium chloride solution successively, the layer was dried over anhydrous magnesium sulfate, and the solvent was distilled off under reduced pressure. The resulting residue was purified by silica gel column chromatography [eluent; chloroform:acetone = 2:1] to obtain a yellow oil.

To the resulting yellow oil were added 2 mL of 1, 4-dioxane and 2 mL of 3 mol/L hydrochloric acid, and the mixture was heated under reflux for 5 hours. To the reaction mixture were added a 6 mol/L aqueous sodium hydroxide solution and ethyl acetate. The organic layer was separated, and the aqueous layer was extracted with ethyl acetate. The organic layer and the extract were combined, and the solvent was distilled off under reduced pressure. The resulting residue was purified by silica gel column chromatography [eluent; chloroform: methanol =5:1], and ethyl acetate and a saturated aqueous sodium bicarbonate solution were added. The organic layer was separated, and the solvent was distilled off under reduced pressure. The resulting solid was suspended in a mixed solvent of ethyl acetate and diisopropyl ether, and the solid was filtered to obtain 6 mg of 2-amino-1-(5-amino-2, 4-difluorophenyl)-7-(3, 5-dimethylisoxazol-4-yl)-1, 4-dihydro-4-oxoquinoline as a white solid. $^1$H-NMR (DMSO-$d_6$) δ:2.10 (s, 3H), 2.30 (s, 3H), 5.37-5.45 (br, 2H), 5.48 (s, 1H), 6.40-6.50 (m, 3H), 6.80 (dd, J = 8.7, 8.7 Hz, 1H), 7.26 (d, J = 8.0 Hz, 1H), 7.46 (dd, J = 10.5, 10.5 Hz, 1H), 8.11 (d, J = 8.0 Hz, 1H).

Example 21

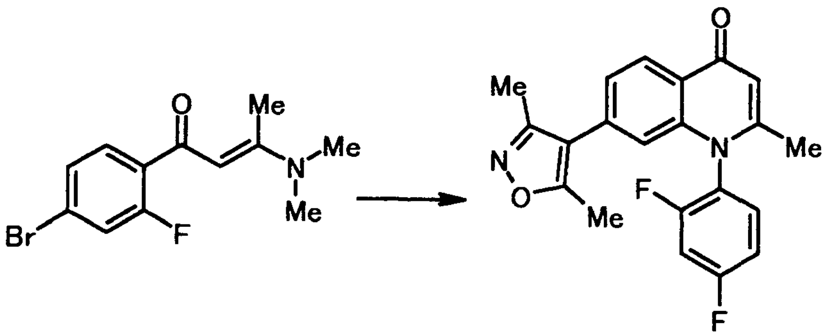

A solution of 2.00 g of (E)-1-(4-bromo-2-fluorophenyl)-3-dimethylamino-2-buten-1-one obtained in a similar manner as the method described in Reference Example 1 and 1.35 g of 2, 4-difluoroaniline in 10 mL of acetic acid was heated under stirring at 40°C for 1 hour. To the reaction mixture were added water and ethyl acetate. The organic layer was separated, and dried over anhydrous magnesium sulfate, and the solvent was distilled off under reduced pressure. The resulting residue was suspended in diisopropyl ether, and the solid was filtered.

To the resulting solid were added 20 mL of DMSO and 0.91 g of potassium carbonate, and the mixture was heated under stirring at 70 to 80°C for 1 hour. To the reaction mixture were added ethyl acetate and water. The organic layer was separated, and dried over anhydrous magnesium sulfate, and the solvent was distilled off under reduced pressure. The resulting residue was suspended in a mixed solvent of ethyl acetate and diisopropyl ether, and the solid was filtered.

The suspension of the resulting solid, 1.41 g of 3, 5-dimethyl-4-(4, 4, 5, 5-tetramethyl-1, 3, 2-dioxaborolan-2-yl)isoxazole, 1.22 g of sodium bicarbonate, 191 mg of triphenylphosphine and 54.5 mg of palladium acetate in 17 mL of toluene, 13.5 mL of ethanol and 8.5 mL of water was heated under reflux under nitrogen atmosphere for 2 hours. To the reaction mixture were added ethyl acetate and water. The organic layer was separated, and dried over anhydrous magnesium sulfate, and the solvent was distilled off under reduced pressure. The resulting residue was purified by silica gel column chromatography [eluent; chloroform:acetone = 10:1] to obtain 1.47 g of 1-(2, 4-difluorophenyl)-7-(3, 5-dimethylisoxazol-4-yl)-2-methyl-1, 4-dihydro-4-oxoquinoline as a pale yellow solid.

$^1$H-NMR (DMSO-$d_6$) δ:2.08 (s, 3H), 2.11 (s, 3H), 2.31 (s, 3H), 6.28-6.32 (m, 1H), 6.63 (s, 1H), 7.39-7.47 (m, 2H), 7.68-7.76 (m, 1H), 7.80-7.88 (m, 1H), 8.25 (d, J = 8.3 Hz, 1H).

Example 22

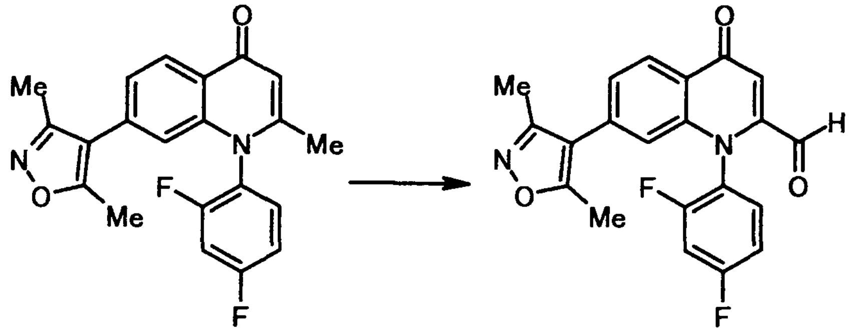

(1) To a suspension of 1.35 g of 1-(2, 4-difluorophenyl)-7-(3, 5-dimethylisoxazol-4-yl)-2-methyl-1, 4-dihydro-4-oxoquinoline in 40.5 mL of 1, 4-dioxane was added 0.45 g of selenium dioxide, and the mixture was heated under reflux for 4 hours and 30 minutes. To the reaction mixture were added ethyl acetate and a saturated aqueous sodium bicarbonate solution. The organic layer was separated, and dried over anhydrous magnesium sulfate, and the solvent was distilled off under reduced pressure. The resulting residue was purified by silica gel column chromatography [eluent; chloroform: methanol = 9:1] to obtain 926 mg of 1-(2, 4-difluorophenyl)-7-(3, 5-dimethylisoxazol-4-yl)-1, 4-dihydro-4-oxoquinoline-2-carbaldehyde as a white solid.
$^1$H-NMR ($CDCl_3$) δ:2.17 (s, 3H), 2.34 (s, 3H), 6.68 (s, 1H), 6.92 (s, 1H), 7.10-7.18 (m, 2H), 7.28-7.37 (m, 2H), 8.50 (d, J = 8.3 Hz, 1H), 9.65 (s, 1H).

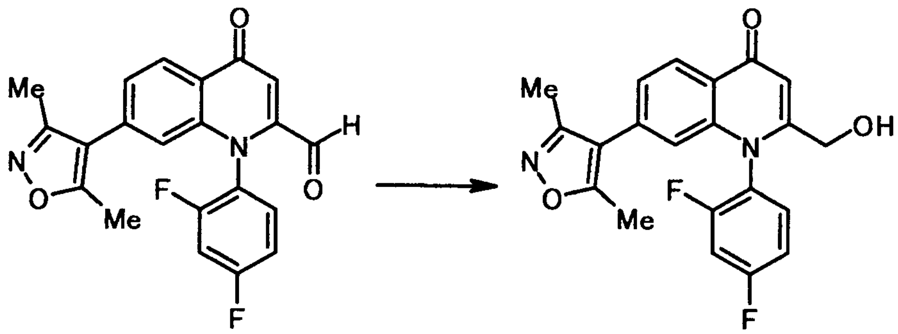

(2) To a suspension of 500 mg of 1-(2, 4-difluorophenyl)-7-(3, 5-dimethylisoxazol-4-yl)-1, 4-dihydro-4-oxoquinoline-2-carbaldehyde in 26 mL of methanol was added 1.39 g of sodium triacetoxyborohydride, and the mixture was stirred at room temperature for 1 hour and 25 minutes. 1.39 g of sodium triacetoxyborohydride was added, and the mixture was stirred at room temperature for 35 minutes. The solvent was distilled off under reduced pressure, and ethyl acetate and water were added. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform: acetone = 8:1] to obtain 400 mg of 1-(2, 4-difluorophenyl)-7-(3, 5-dimethylisoxazol-4-yl)-2-hydroxymethyl-1, 4-dihydro-4-oxoquinoline as a pale brown solid.
$^1$H-NMR (DMSO-$d_6$) δ:2.10 (s, 3H), 2.31 (s, 3H), 4.01, 4.14 (ABq, J = 15.7 Hz, 2H), 5.65-5.73 (br, 1H), 6.44 (s, 1H), 6.62 (s, 1H), 7.36-7.43 (m, 1H), 7.45 (dd, J = 8.2, 1.6 Hz, 1H), 7.65-7.73 (m, 1H), 7.78-7.87 (m, 1H), 8.27 (d, J = 8.2 Hz, 1H).

Example 23

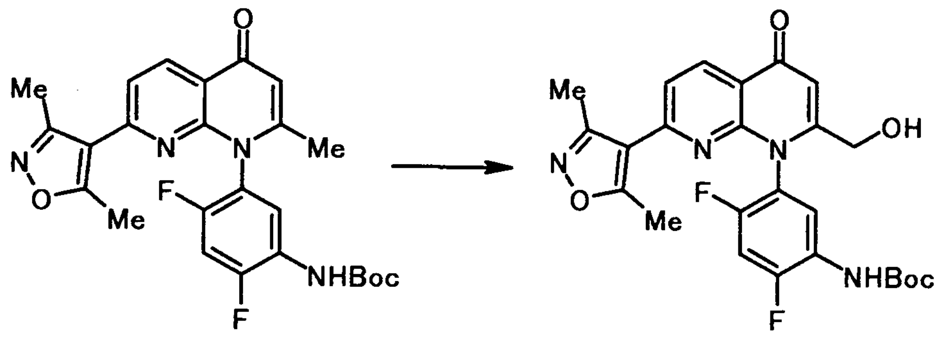

To a solution of 630 mg of 1-(5-tert-butoxycarbonylamino-2, 4-difluorophenyl)-7-(3, 5-dimethylisoxazol-4-yl)-2-methyl-1, 4-dihydro-4-oxo-l, 8-naphthyridine in 20 mL of 1, 4-dioxane was added 159 mg of selenium dioxide, and the mixture was heated under reflux for 6 hours and 30 minutes. The solvent was distilled off under reduced pressure, and ethyl acetate and a saturated aqueous sodium bicarbonate solution were added. Insoluble matters were filtered off, and ethyl acetate and a saturated aqueous sodium bicarbonate solution were added. The organic layer was separated, and dried over anhydrous magnesium sulfate, and the solvent was distilled off under reduced pressure. The resulting residue was suspended in diisopropyl ether, and the solid was filtered.

To the suspension of the resulting solid in 26 mL of methanol was added 1.13 g of sodium triacetoxyborohydride, and the mixture was stirred at room temperature for 45 minutes. 565 mg of sodium triacetoxyborohydride was added, and the mixture was stirred at room temperature for 1 hour and 45 minutes. 565 mg of sodium triacetoxyborohydride was added, and the mixture was stirred at room temperature for 1 hour. 565 mg of sodium triacetoxyborohydride was added, and the mixture was stirred at room temperature for 1 hour. The solvent was distilled off under reduced pressure, and ethyl acetate and a saturated aqueous sodium bicarbonate solution were added. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform: acetone = 1:1] to obtain 330 mg of 1-(5-tert-butoxycarbonylamino-2, 4-difluorophenyl)-7-(3, 5-dimethylisoxazol-4-yl)-2-hydroxymethyl-1, 4-dihydro-4-oxo-1, 8-naphthyridine as a brown oil. $^1$H-NMR (DMSO-$d_6$) δ:1.42 (s, 9H), 2.07 (s, 3H), 2.38 (s, 3H), 4.02-4.28 (m, 2H), 5.74-5.80 (m, 1H), 6.46 (s, 1H), 7.63 (dd, J = 10.1, 10.1 Hz, 1H), 7.66 (d, J = 8.3 Hz, 1H), 7.81-7.89 (m, 1H), 8.57 (d, J = 8.3 Hz, 1H), 9.18-9.31 (br, 1H).

Example 24

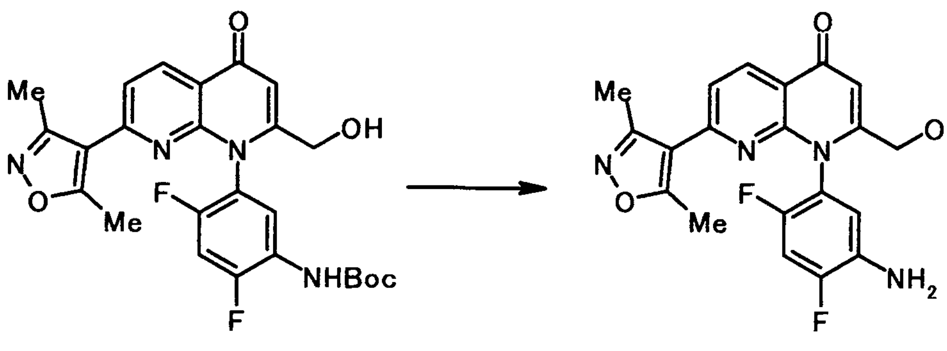

To a suspension of 324 mg of 1-(5-tert-butoxycarbonylamino-2, 4-difluorophenyl)-7-(3, 5-dimethylisoxazol-4-yl)-2-hydroxymethyl-1, 4-dihydro-4-oxo-1, 8-naphthyridine in 8 mL of chloroform was added 2 mL of TFA, and the mixture was stirred at room temperature for 1 hour. The reaction mixture was added to a saturated aqueous sodium bicarbonate solution, and to the mixture was added chloroform. The organic layer was separated, and after washing with a 1 mol/L aqueous sodium hydroxide solution, insoluble matters were filtered off. The filtrate was dried over anhydrous sodium sulfate, and the solvent was distilled off under reduced pressure. The resulting residue was suspended in diisopropyl ether, and the solid was filtered to obtain 85 mg of 1-(5-amino-2, 4-difluorophenyl)-7-(3, 5-dimethylisoxazol-4-yl)-2-hydroxymethyl-1, 4-dihydro-4-oxo-1, 8-naphthyridine as a pale brown solid.

$^1$H-NMR (DMSO-$d_6$) δ:2.11 (s, 3H), 2.41 (s, 3H), 4.07, 4.23 (ABq, J = 16.5 Hz, 2H), 5.30 (brs, 2H), 5.73-5.81 (br, 1H), 6.46 (s, 1H), 6.82-6.88 (m, 1H), 7.34-7.42 (m, 1H), 7.65 (d, J = 8.3 Hz, 1H), 8.56 (d, J = 8.3 Hz, 1H).

Example 25

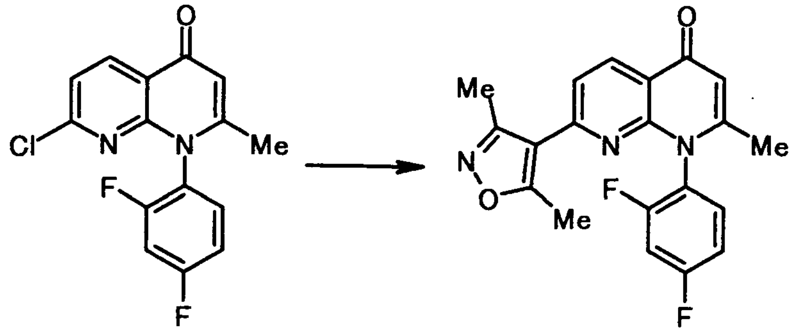

A suspension of 1.55 g of 7-chloro-1-(2, 4-difluorophenyl)-2-methyl-1, 4-dihydro-4-oxo-1, 8-naphthyridine obtained in a similar manner as the method described in Reference Example 20, 812 mg of 3, 5-dimethylisoxazole-4-boronic acid, 3.3 mL of triethylamine, 394 mg of S-Phos and 108 mg of palladium acetate in 15.5 mL of 1, 4-dioxane was heated under reflux under nitrogen atmosphere for 2 hours. The solvent was distilled off under reduced pressure, and ethyl acetate and water were added. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform: acetone = 5:1], and suspended in diisopropyl ether, and the solid was filtered to obtain 1.74 g of 1-(2, 4-difluorophenyl)-7-(3, 5-dimethylisoxazol-4-yl)-2-methyl-1, 4-dihydro-4-oxo-1, 8-naphthyridine as a yellow solid.
$^1$H-NMR ($CDCl_3$) δ:2.14 (s, 3H), 2.16 (s, 3H), 2.36 (s, 3H), 6.38 (s, 1H), 7.06-7.15 (m, 2H), 7.28-7.36 (m, 1H), 7.40 (d, J = 8.3 Hz, 1H), 8.72 (d, J = 8.3 Hz, 1H).

Example 26

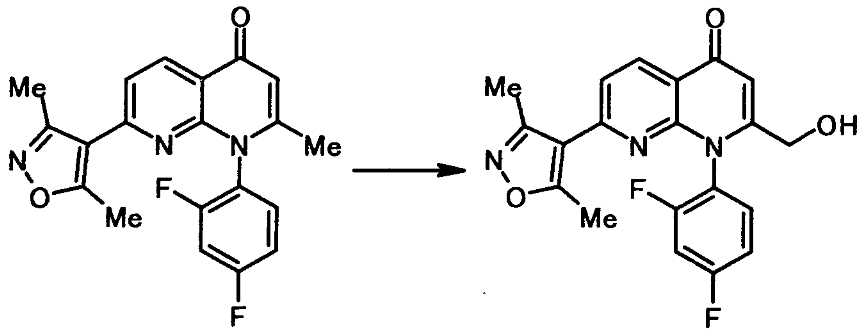

To the suspension of 700 mg of 1-(2, 4-difluorophenyl)-7-(3, 5-dimethylisoxazol-4-yl)-2-methyl-1, 4-dihydro-4-oxo-1, 8-naphthyridine in 20 mL of 1, 4-dioxane was added 233 mg of selenium dioxide, and the mixture was heated under reflux for 6 hours and 30 minutes. The solvent was distilled off under reduced pressure, and ethyl acetate and a saturated aqueous sodium bicarbonate solution were added. The organic layer was separated, and insoluble matters were filtered off. To the filtrate were added ethyl acetate and a saturated aqueous sodium bicarbonate solution. The organic layer was separated, and dried over anhydrous magnesium sulfate, and the solvent was distilled off under reduced pressure. The resulting residue was suspended in diisopropyl ether, and 700 mg of the solid was filtered.

To the suspension of 600 mg of the resulting solid in 30 mL of methanol was added 1.67 g of sodium triacetoxyborohydride, and the mixture was stirred at room temperature for 45 minutes. 834 mg of sodium triacetoxyborohydride was added, and the mixture was stirred at room temperature for 1 hour and 45 minutes. 834 mg of sodium triacetoxyborohydride was added, and the mixture was stirred at room temperature for 1 hour. 834 mg of sodium triacetoxyborohydride was added, and the mixture was stirred at room temperature for 1 hour. The solvent was distilled off under reduced pressure, and ethyl acetate and a saturated aqueous sodium bicarbonate solution were added. The organic layer was separated, and after washing with a saturated aqueous sodium chloride solution, the layer was dried over

anhydrous magnesium sulfate, and the solvent was distilled off under reduced pressure. The resulting residue was purified by silica gel column chromatography [eluent; chloroform:acetone = 1:1], and suspended in diisopropyl ether, and the solid was filtered to obtain 331 mg of 1-(2, 4-difluorophenyl)-7-(3, 5-dimethylisoxazol-4-yl)-2-hydroxymethyl-1, 4-dihydro-4-oxo-1, 8-naphthyridine as a pale brown solid.
$^1$H-NMR (DMSO-$d_6$) δ:2.04 (s, 3H), 2.37 (s, 3H), 4.04, 4.21 (ABq, J = 15.6 Hz, 2H), 5.70-5.80 (br, 1H), 6.48 (s, 1H), 7.30-7.38 (m, 1H), 7.57-7.65 (m, 1H), 7.66 (d, J = 8.3 Hz, 1H), 7.72-7.79 (m, 1H), 8.58 (d, J = 8.3 Hz, 1H).

Example 27

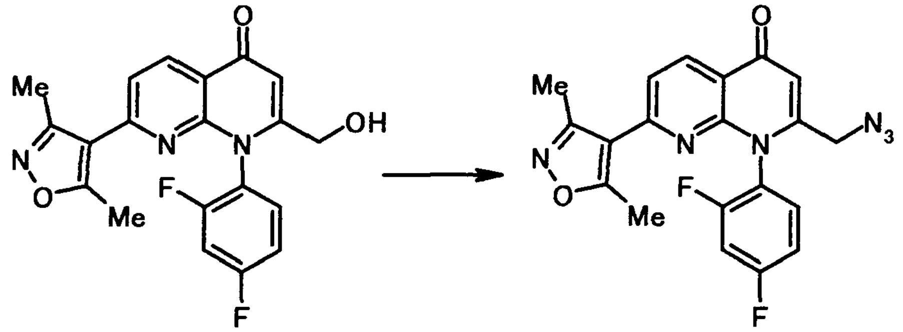

(1) To a suspension of 200 mg of 1-(2, 4-difluorophenyl)-7-(3, 5-dimethylisoxazol-4-yl)-2-hydroxymethyl-1, 4-dihydro-4-oxo-1, 8-naphthyridine and 94 μL of DBU in 3 mL of 1, 4-dioxane was added 135 μL of DPPA under ice cooling, and the mixture was allowed to stand still at room temperature overnight. After distilling off the solvent under reduced pressure, ethyl acetate and 1 mol/L hydrochloric acid were added. The organic layer was separated, and after washing with a saturated aqueous sodium chloride solution, the solvent was distilled off under reduced pressure. The resulting residue was purified by silica gel column chromatography [eluent; chloroform:acetone = 5:1] to obtain 53 mg of 2-azidomethyl-1-(2, 4-difluorophenyl)-7-(3, 5-dimethylisoxazol-4-yl)-1, 4-dihydro-4-oxo-1, 8-naphthyridine as a yellow oil.
$^1$H-NMR ($CDCl_3$) δ:2.14 (s, 3H), 2.36 (s, 3H), 4.05, 4.10 (ABq, J = 14.8 Hz, 2H), 6.55 (s, 1H), 7.07-7.18 (m, 2H), 7.36-7.45 (m, 1H), 7.44 (d, J = 8.2 Hz, 1H), 8.73 (d, J = 8.2 Hz, 1H).

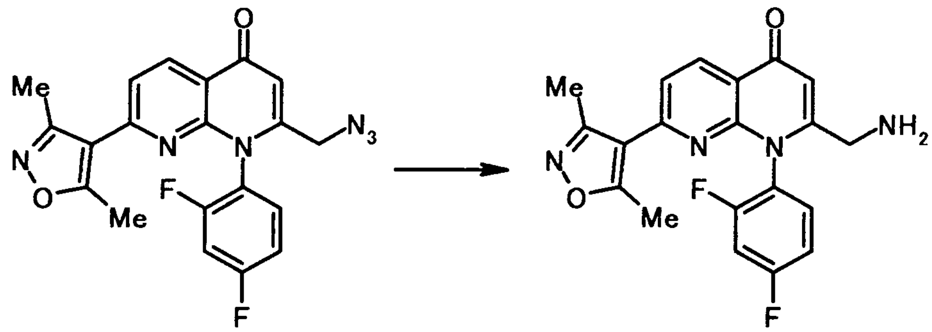

(2) A suspension of 22 mg of 2-azidomethyl-1-(2, 4-difluorophenyl)-7-(3, 5-dimethylisoxazol-4-yl)-1, 4-dihydro-4-oxo-1, 8-naphthyridine and 11 mg ofLindlar's catalyst (5% palladium) in 2 mL of methanol was stirred under hydrogen atmosphere at room temperature for 5 hours. Insoluble matters were filtered off, and the solvent was distilled off under reduced pressure. The resulting residue was purified by silica gel column chromatography [eluent; chloroform: methanol = 10:1], and suspended in diisopropyl ether, and the solid was filtered to obtain 6 mg of 2-(aminomethyl)-1-(2, 4-difluorophenyl)-7-(3, 5-dimethylisoxazol-4-yl)-1, 4-dihydro-4-oxo-1, 8-naphthyridine as a pale yellow solid.
$^1$H-NMR ($CDCl_3$) δ:2.14 (s, 3H), 2.36 (s, 3H), 3.48-3.67 (m, 2H), 6.64 (s, 1H), 7.06-7.15 (m, 2H), 7.31-7.39 (m, 1H), 7.41 (d, J = 8.3 Hz, 1H), 8.73 (d, J = 8.3 Hz, 1H).

Example 28

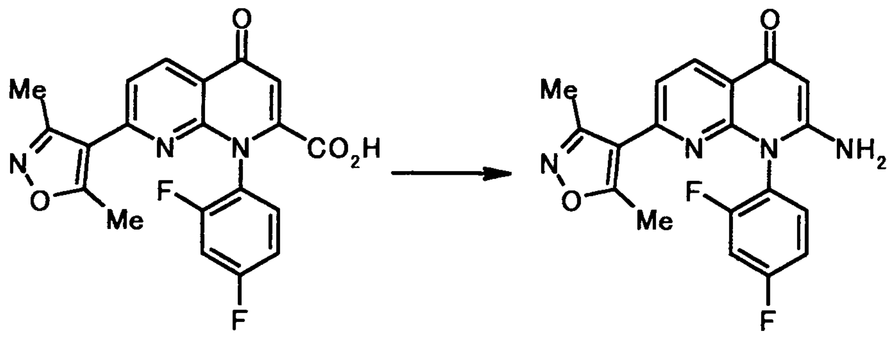

1-(2, 4-difluorophenyl)-7-(3, 5-dimethylisoxazol-4-yl)-1, 4-dihydro-4-oxo-1, 8-naphthyridine-2-carboxylic acid was obtained based on the method described in Reference Example 7, from the compound obtained in Example 25.
To a suspension of 110 mg of 1-(2, 4-difluorophenyl)-7-(3, 5-dimethylisoxazol-4-yl)-1, 4-dihydro-4-oxo-1, 8-naphthyridine-2-carboxylic acid in 2 mL of 2-methyl-2-propanol were added 77 μL of triethylamine and 119 μL of DPPA, and the mixture was heated under reflux for 6 hours. To the reaction mixture were added ethyl acetate and a saturated aqueous sodium bicarbonate solution. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 5:1] to obtain a brown oil.
To the resulting oil were added 2 mL of 1, 4-dioxane and 1 mL of water and 1 mL of 6 mol/L hydrochloric acid, and the mixture was heated under reflux for 1 hour. The solvent was distilled off under reduced pressure, and the resulting residue was suspended in a mixed solvent of chloroform, methanol and ethyl acetate, and the solid was filtered and recrystallized from a mixed solvent of chloroform, methanol and ethyl acetate to obtain 20 mg of 2-amino-1-(2, 4-difluorophenyl)-7-(3, 5-dimethylisoxazol-4-yl)-1, 4-dihydro-4-oxo-1, 8-naphthyridine as a pale yellow solid.
$^1$H-NMR (DMSO-$d_6$) δ:2.04 (s, 3H), 2.36 (s, 3H), 6.69 (s, 1H), 7.40-7.50 (m, 1H), 7.69-7.85 (m, 3H), 8.55 (d, J = 8.3 Hz, 1H).

Example 29

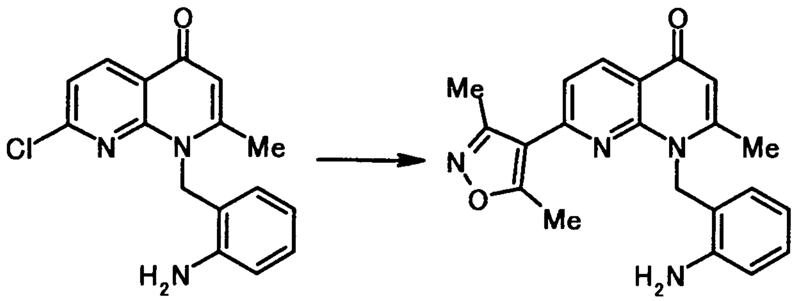

A suspension of 100 mg of 1-(2-aminobenzyl)-7-chloro-2-methyl-1, 4-dihydro-4-oxo-1, 8-naphthyridine obtained in a similar manner as the method described in Reference Example 21, 61 mg of 3, 5-dimethylisoxazole-4-boronic acid, 0.28 mL of triethylamine, 27 mg of S-Phos and 8 mg of palladium acetate in 3.3 mL of 1, 4-dioxane was heated under reflux under nitrogen atmosphere for 3 hours. To the reaction mixture were added ethyl acetate and water. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 1:1], and recrystallized from a mixed solvent of ethyl acetate and ethanol to obtain 60 mg of 1-(2-aminobenzyl)-7-(3, 5-dimethylisoxazol-4-yl)-2-methyl-1, 4-dihydro-4-oxo-1, 8-naphthyridine as a pale brown solid.
$^1$H-NMR (DMSO-$d_6$) δ:2.18 (s, 3H), 2.39 (s, 3H), 2.43 (s, 3H), 5.19 (s, 2H), 5.52 (s, 2H), 6.10 (d, J = 7.1 Hz, 1H), 6.28 (s, 1H), 6.35-6.44 (m, 1H), 6.72 (d, J = 7.6 Hz, 1H), 6.89-6.99 (m, 1H), 7.62 (d, J = 8.3 Hz, 1H), 8.58 (d, J = 8.3 Hz, 1H).

Example 30

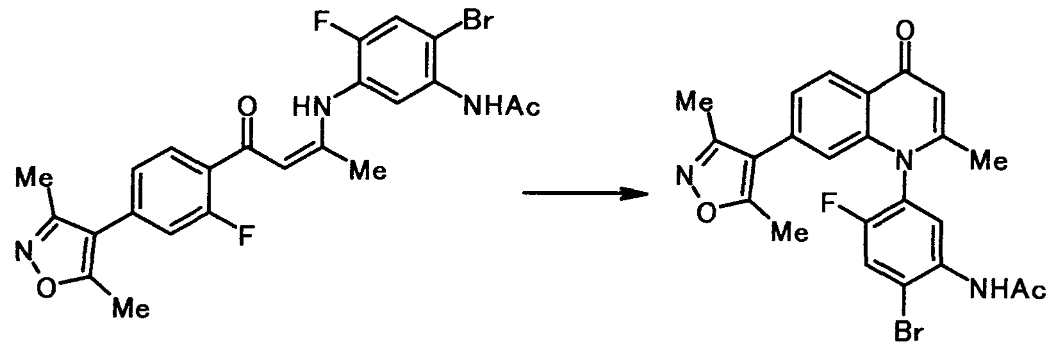

A suspension of 0.47 g of (Z)-3-(5-acetylamino-4-bromo-2-fluorophenylamino)-1-(4-(3, 5-dimethylisoxazol-4-yl)-2-fluorophenyl)-2-buten-1-one obtained in a similar manner as the method described in Reference Example 23 and 0.19 g of potassium carbonate in 8 mL of DMSO was heated under stirring at 75 to 80°C for 4 hours and 30 minutes. After cooling the reaction mixture, ethyl acetate and water were added. The organic layer was separated, and after washing with a saturated aqueous sodium chloride solution, the layer was dried over anhydrous magnesium sulfate, and the solvent was distilled off under reduced pressure. The resulting residue was purified by flash column chromatography [eluent; chloroform:methanol = 4:1] to obtain 0.48 g of 1-(5-acetylamino-4-bromo-2-fluorophenyl)-7-(3, 5-dimethylisoxazol-4-yl)-2-methyl-1, 4-dihydro-4-oxoquinoline as a brown solid.
$^1$H-NMR ($CDCl_3$) δ:2.14 (s, 6H), 2.27 (s, 3H), 2.31 (s, 3H), 6.36 (s, 1H), 6.54 (s, 1H), 7.24 (dd, J = 8.3, 1.5 Hz, 1H), 7.64 (d, J = 8.3 Hz, 1H), 7.67-7.73 (br, 1H), 8.48 (d, J = 8.3 Hz, 1H), 8.55 (d, J = 7.6 Hz, 1H).

Example 31

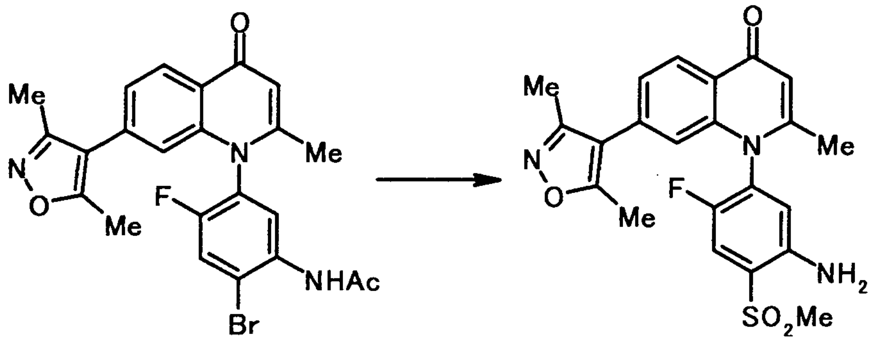

A suspension of 150 mg of 1-(5-acetylamino-4-bromo-2-fluorophenyl)-7-(3, 5-dimethylisoxazol-4-yl)-2-methyl-1, 4-dihydro-4-oxoquinoline, 45 mg of sodium methanesulfinate, 6 mg of copper (I) iodide, 7 mg of DL-proline and 3 mg of sodium hydroxide in 1 mL of DMSO was heated under stirring under nitrogen atmosphere at 110 to 120°C for 8 hours and 55 minutes. 45 mg of sodium methanesulfinate, 6 mg of copper (I) iodide, 7 mg of DL-proline and 3 mg of sodium hydroxide were added, and the mixture was heated under stirring at 110 to 120°C for 5 hours and 30 minutes. To the reaction mixture were added ethyl acetate, water and ammonia water. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:methanol = 10:1], and suspended in a mixed solvent of ethyl acetate and diisopropyl ether, and the solid was filtered to obtain 50 mg of a pale yellow solid.

To the suspension of 40 mg of the resulting pale yellow solid in 1.5 mL of 1, 4-dioxane was added 1.5 mL of 3 mol/L hydrochloric acid, and the mixture was heated under reflux for 40 minutes. To the reaction mixture were added a 6 mol/L aqueous sodium hydroxide solution and ethyl acetate. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform: acetone = 1:1], and suspended in a mixed solvent of ethyl acetate and diisopropyl ether, and the solid was filtered to obtain 20 mg of 1-(5-amino-2-fluoro-4-(methylsulfonyl)phenyl)-7-(3, 5-dimethylisoxazol-4-yl)-2-methyl-1, 4-dihydro-4-oxoquinoline as a white solid.
$^1$H-NMR (DMSO-$d_6$) δ:2.14 (s, 3H), 2.16 (s, 3H), 2.34 (s, 3H), 3.29 (s, 3H), 6.25 (brs, 2H), 6.30 (s, 1H), 6.77 (brs, 1H),

7.07 (d, J = 6.3 Hz, 1H), 7.46 (d, J = 8.3 Hz, 1H), 7.68 (d, J = 9.3 Hz, 1H), 8.24 (d, J = 8.3 Hz, 1H).

Example 32

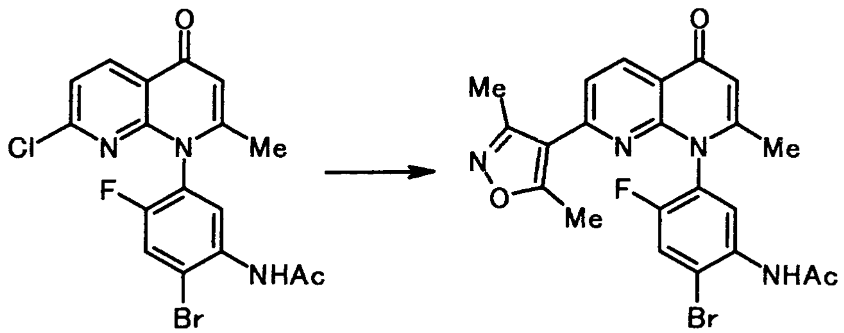

A suspension of 1.08 g of 1-(5-acetylamino-4-bromo-2-fluorophenyl)-7-chloro-2-methyl-1, 4-dihydro-4-oxo-1, 8-naphthyridine obtained in a similar manner as the method described in Reference Example 24, 0.50 g of 3, 5-dimethylisoxazole-4-boronic acid, 1.8 mL of triethylamine, 0.21 g of S-Phos and 57 mg of palladium acetate in 11 mL of 1, 4-dioxane was heated under reflux under nitrogen atmosphere for 4 hours. To the reaction mixture was added ethyl acetate, and insoluble matters were filtered off. To the filtrate were added water and 1 mol/L hydrochloric acid. The organic layer was separated, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 9:1], and suspended in a mixed solvent of ethyl acetate and diisopropyl ether, and the solid was filtered to obtain 285 mg of 1-(5-acetylamino-4-bromo-2-fluorophenyl)-7-(3, 5-dimethylisoxazol-4-yl)-2-methyl-1, 4-dihydro-4-oxo-1, 8-naphthyridine as a white solid.
$^1$H-NMR (DMSO-$d_6$) δ:2.06 (s, 6H), 2.16 (s, 3H), 2.37 (s, 3H), 6.34 (s, 1H), 7.65 (d, J = 8.3 Hz, 1H), 7.87 (d, J = 7.6 Hz, 1H), 8.00 (d, J = 9.0 Hz, 1H), 8.55 (d, J = 8.3 Hz, 1H), 9.72 (brs, 1H).

Example 33

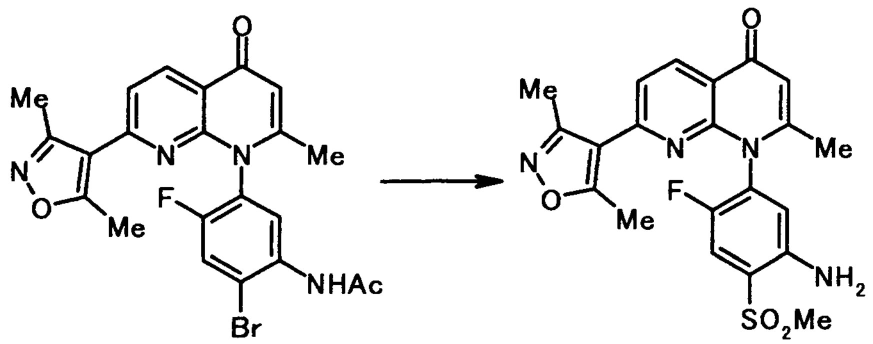

A suspension of 100 mg of 1-(5-acetylamino-4-bromo-2-fluorophenyl)-7-(3, 5-dimethylisoxazol-4-yl)-2-methyl-1, 4-dihydro-4-oxo-1, 8-naphthyridine, 49 mg of sodium methanesulfinate, 8 mg of copper (I) iodide, 7 mg of DL-proline and 2 mg of sodium hydroxide in 1 mL of DMSO was heated under stirring in a sealed tube at 100 to 120°C for 9 hours. To the reaction mixture were added ethyl acetate and ammonia water. The organic layer was separated, and the aqueous layer was extracted with ethyl acetate. The organic layer and the extract were combined, and after washing with a saturated aqueous sodium chloride solution, the layer was dried over anhydrous magnesium sulfate, and the solvent was distilled off under reduced pressure. The resulting residue was purified by silica gel column chromatography [eluent; chloroform:acetone = 2:1] to obtain a solid.

To the resulting solid were added 2 mL of 1, 4-dioxane and 2 mL of 3 mol/L hydrochloric acid, and the mixture was heated under reflux for 2 hours. To the reaction mixture were added an aqueous sodium hydroxide solution and ethyl acetate. The organic layer was separated, and after washing with a saturated aqueous sodium chloride solution,

the layer was dried over anhydrous magnesium sulfate, and the solvent was distilled off under reduced pressure. The resulting residue was purified by silica gel column chromatography [eluent; chloroform:acetone = 2:1], and suspended in a mixed solvent of ethyl acetate and diisopropyl ether, and the solid was filtered to obtain 16 mg of 1-(5-amino-2-fluoro-4-(methylsulfonyl)phenyl)-7-(3, 5-dimethylisoxazol-4-yl)-2-methyl-1, 4-dihydro-4-oxo-l, 8-naphthyridine as a white solid.

$^1$H-NMR (DMSO-$d_6$) δ:2.12 (s, 3H), 2.20 (s, 3H), 2.41 (s, 3H), 3.27 (s, 3H), 6.19 (brs, 2H), 6.35 (s, 1H), 7.06 (d, J = 6.1 Hz, 1H), 7.61 (d, J = 9.0 Hz, 1H), 7.67 (d, J = 8.2 Hz, 1H), 8.55 (d, J = 8.2 Hz, 1H).

Example 34

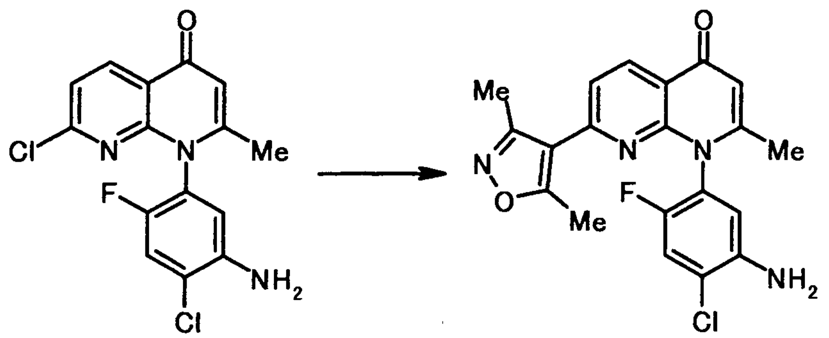

A suspension of 0.23 g of 1-(5-amino-4-chloro-2-fluorophenyl)-7-chloro-2-methyl-1, 4-dihydro-4-oxo-1, 8-naphthyridine obtained in a similar manner as the method described in Reference Example 26, 0.12 g of 3, 5-dimethylisoxazole-4-boronic acid, 0.5 mL of triethylamine, 58 mg of S-Phos and 16 mg of palladium acetate in 2.3 mL of 1, 4-dioxane was heated under reflux under nitrogen atmosphere for 2 hours. After cooling the reaction mixture, the solvent was distilled off under reduced pressure. Ethyl acetate was added, and insoluble matters were filtered off. The solvent was distilled off under reduced pressure, and the resulting residue was purified by silica gel column chromatography [eluent; chloroform:acetone =2:1], and suspended in a mixed solvent of ethyl acetate and diisopropyl ether, and the solid was filtered to obtain 121 mg of 1-(5-amino-4-chloro-2-fluorophenyl)-7-(3, 5-dimethylisoxazol-4-yl)-2-methyl-1, 4-dihydro-4-oxo-1, 8-naphthyridine as a yellow white solid. $^1$H-NMR (DMSO-$d_6$) δ:2.10 (s, 3H), 2.17 (s, 3H), 2.41 (s, 3H), 5.51 (brs, 2H), 6.32 (s, 1H), 6.88 (d, J = 7.3 Hz, 1H), 7.53 (dd, J = 9.3 Hz, 1H), 7.64 (d, J = 8.3 Hz, 1H), 8.55 (d, J = 8.3 Hz, 1H).

Example 35

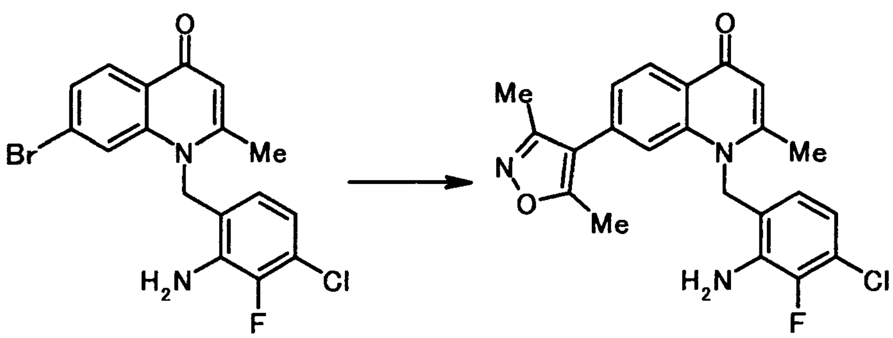

A suspension of 90 mg of 1-(2-amino-4-chloro-3-fluorobenzyl)-7-bromo-2-methyl-1, 4-dihydro-4-oxoquinoline obtained in a similar manner as the method described in Reference Example 28, 68 mg of 3, 5-dimethyl-4-(4, 4, 5, 5-tetramethyl-1, 3, 2-dioxaborolan-2-yl)isoxazole, 59 mg of sodium bicarbonate, 19 mg of triphenylphosphine and 8 mg of palladium acetate in 1 mL of toluene, 0.8 mL of ethanol and 0.5 mL of water was heated under reflux under nitrogen atmosphere for 1 hour. After cooling the reaction mixture, ethyl acetate was added. The organic layer was separated, and after washing with a saturated aqueous sodium chloride solution, the layer was dried over anhydrous magnesium sulfate, and the solvent was distilled off under reduced pressure. The resulting residue was purified by silica gel column

chromatography [eluent; chloroform:acetone = 1:1] to obtain 50 mg of 1-(2-amino-4-chloro-3-fluorobenzyl)-7-(3, 5-dimethylisoxazol-4-yl)-2-methyl-1, 4-dihydro-4-oxoquinoline as a pale brown solid. $^1$H-NMR (DMSO-$d_6$) $\delta$:2.04 (s, 3H), 2.21 (s, 3H), 2.44 (s, 3H), 5.33 (s, 2H), 5.66 (s, 2H), 6.09 (d, J = 8.3 Hz, 1H), 6.23 (s, 1H), 6.54-6.64 (m, 1H), 7.18 (s, 1H), 7.38 (d, J = 8.3 Hz, 1H), 8.24 (d, J = 8.1 Hz, 1H).

The heterocyclic compound having an isoxazolyl group or a salt thereof of the present invention has an excellent anti-HIV activity, and are useful as an anti-HIV agent.

## Claims

1. A heterocyclic compound represented by a general formula:

[Formula 1]

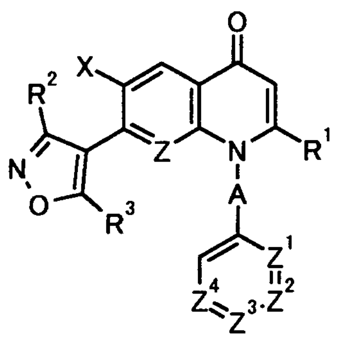

or a salt thereof, wherein $R^1$ represents an optionally protected amino group, or an optionally substituted $C_{1-6}$ alkyl group; $R^2$ and $R^3$ are the same or different and represent a $C_{1-2}$ alkyl group; X represents a hydrogen atom or a halogen atom; $Z^1$ represents C-$R^4$ (wherein $R^4$ represents a hydrogen atom, a halogen atom, an optionally protected amino group or a $C_{1-6}$ alkylsulfonyl group), or N; $Z^2$ represents C-$R^5$ (wherein $R^5$ represents a hydrogen atom, a halogen atom, an optionally protected amino group or a $C_{1-6}$ alkylsulfonyl group), or N; $Z^3$ represents C-$R^6$ (wherein $R^6$ represents a hydrogen atom, a halogen atom, an optionally protected amino group or a $C_{1-6}$ alkylsulfonyl group), or N; $Z^4$ represents C-$R^7$ (wherein $R^7$ represents a hydrogen atom, a halogen atom, an optionally protected amino group or a $C_{1-6}$ alkylsulfonyl group), or N; and Z represents CH or N; A represents a methylene group or a bond.

2. A heterocyclic compound represented by a general formula:

[Formula 2]

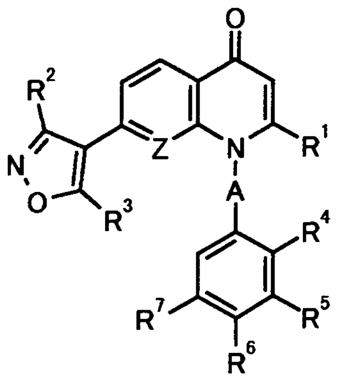

or a salt thereof, wherein $R^1$ represents an optionally protected amino group, or an optionally substituted $C_{1-6}$ alkyl group; $R^2$ and $R^3$ are the same or different and represent a $C_{1-2}$ alkyl group; $R^4$, $R^5$, $R^6$ and $R^7$ are the same or

different and represent a hydrogen atom, a halogen atom, an optionally protected amino group or a $C_{1-6}$ alkylsulfonyl group; Z represents CH or N; and A represents a methylene group or a bond.

3. The heterocyclic compound according to claim 2 or a salt thereof, wherein $R^4$, $R^5$, $R^6$ and $R^7$ are the same or different and are a hydrogen atom, a halogen atom or an optionally protected amino group.

4. The heterocyclic compound according to claim 3 or a salt thereof, wherein A is a bond.

5. The heterocyclic compound according to claim 3 or 4 or a salt thereof, wherein $R^2$ is a methyl group; and $R^3$ is a methyl group.

6. The heterocyclic compound according to any one of claims 3 to 5 or a salt thereof, wherein $R^1$ is a methyl group optionally substituted by a hydroxyl group, or an optionally protected amino group.

7. The heterocyclic compound according to any one of claims 3 to 5 or a salt thereof, wherein $R^1$ is a methyl group.

8. The heterocyclic compound according to any one of claims 3 to 7 or a salt thereof, wherein $R^4$ is a halogen atom or an optionally protected amino group; $R^5$ is a hydrogen atom or a halogen atom; $R^6$ is a hydrogen atom or a halogen atom; and $R^7$ is a hydrogen atom or an optionally protected amino group.

9. An anti-HIV agent comprising the heterocyclic compound according to any one of claims 1 to 8 or a salt thereof.

## INTERNATIONAL SEARCH REPORT

International application No.
PCT/JP2011/050927

A. CLASSIFICATION OF SUBJECT MATTER
*C07D413/04*(2006.01)i, *A61K31/4375*(2006.01)i, *A61K31/4709*(2006.01)i, *A61P31/18*(2006.01)i, *C07D471/04*(2006.01)i

According to International Patent Classification (IPC) or to both national classification and IPC

B. FIELDS SEARCHED

Minimum documentation searched (classification system followed by classification symbols)
C07D413/04, A61K31/4375, A61K31/4709, A61P31/18, C07D471/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 8-151368 A (Toyama Chemical Co., Ltd.), 11 June 1996 (11.06.1996), claims; examples 1 to 10 (Family: none) | 1-9 |
| A | DI, S.R. et al, Novel Quinolinonyl Diketo Acid Derivatives as HIV-1 Integrase Inhibitors: Design, Synthesis, and Biological Activities, Journal of Medicinal Chemistry, 2008, Vol.51, No.15, p.4744-4750, Compound 5i, 6i | 1-9 |
| A | WO 1990/13542 A1 (Daiichi Pharmaceutical Co., Ltd.), 15 November 1990 (15.11.1990), claims; examples 1 to 3 & EP 470252 A1 & DE 68923169 C | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 March, 2011 (28.03.11) | 05 April, 2011 (05.04.11) |
| Name and mailing address of the ISA/ Japanese Patent Office | Authorized officer |
| Facsimile No. | Telephone No. |

Form PCT/ISA/210 (second sheet) (July 2009)

INTERNATIONAL SEARCH REPORT

International application No.
PCT/JP2011/050927

C (Continuation). DOCUMENTS CONSIDERED TO BE RELEVANT

| Category* | Citation of document, with indication, where appropriate, of the relevant passages | Relevant to claim No. |
|---|---|---|
| A | WENTLAND, M.P. et al, Antiviral properties of 3-quinolinecarboxamides: a series of novel non-nucleoside antiherpetic agents, Drug Design and Discovery, 1997, Vol.15, No.1, p.25-38, Compound 54, 55 | 1-9 |
| A | WO 2001/081340 A2 (BRISTOL-MYERS SQUIBB CO.), 01 November 2001 (01.11.2001), claims; examples & JP 2003-531205 A & EP 1276739 A1 | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3713291 B
- JP 3754467 B


### Non-patent literature cited in the description


- *Antibiotics & Chemotherapy,* 2009, vol. 25, 26-33
- *Antibiotics & Chemotherapy,* 2004, vol. 20, 58-68
- *Antibiotics & Chemotherapy,* 2009, vol. 25, 40-53
- *Antibiotics & Chemotherapy,* 2009, vol. 25, 54-61
- **W. GREENE et al.** Protective Groups in Organic Synthesis. John Wiley & Sons, INC, 2007, 696-926
- *Tetrahedron,* 2002, vol. 58, 3323-3328
- *Journal of the American Chemical Society,* 1972, vol. 94, 6203-6205
- Strategic Applications of Named Reactions in Organic Synthesis. Elsevier, INC, 210-211266-267396-397
- **W. GREENE et al.** Protective Groups in Organic Synthesis. John Wiley & Sons, INC, 696-926
- *Collection of Czechoslovak Chemical Communications,* 2004, vol. 69, 822-832
- *Journal of Clinical Microbiology,* 2007, vol. 45, 477-487
- *J. Med. Chem.,* 2003, vol. 46, 1905-1917